# EUROPEAN PATENT SPECIFICATION

(11) **EP 3 844 243 B1**
(45) Date of publication and mention of the grant of the patent: **22.06.2022**
(21) Application number: 19756197.0
(22) Date of filing: 26.08.2019
(51) Int. Cl.: C09K 11/06, H01L 51/00

(54) **MATERIALS FOR ORGANIC ELECTROLUMINESCENT DEVICES**
MATERIALIEN FÜR ORGANISCHE ELEKTROLUMINESZENTE VORRICHTUNGEN
MATÉRIAUX POUR DISPOSITIFS ÉLECTROLUMINESCENTS ORGANIQUES

(30) Priority: 28.08.2018 EP 18191167
(43) Date of publication of application: 07.07.2021
(73) Proprietor: Merck Patent GmbH, 64293 Darmstadt (DE)
(72) Inventor: LINGE, Rouven, 64291 Darmstadt (DE); MEYER, Sebastian, 60594 Frankfurt am Main (DE); RODRIGUEZ, Lara-Isabel, 64285 Darmstadt (DE); LACKNER, Aaron, 68165 Mannheim (DE)
(86) International application number: PCT/EP2019/072670
(87) International publication number: WO 2020/043646

(56) References cited:
- CN-A- 107 573 306
- CN-A- 107 573 925
- CN-A- 108 341 795

## Description

The present invention relates to a compound of the formula (1), to the use of the compound in an electronic device, and to an electronic device comprising a compound of the formula (1). The present invention furthermore relates to a process for the preparation of a compound of the formula (1) and to a formulation comprising one or more compounds of the formula (1).

The development of functional compounds for use in electronic devices is currently the subject of intensive research. The aim is, in particular, the development of compounds with which improved properties of electronic devices in one or more relevant points can be achieved, such as, for example, power efficiency and lifetime of the device as well as colour coordinates of the emitted light.

In accordance with the present invention, the term electronic device is taken to mean, inter alia, organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs).

Of particular interest is the provision of compounds for use in the lastmentioned electronic devices called OLEDs. The general structure and the functional principle of OLEDs are known to the person skilled in the art and are described, for example, in US 4539507. Compounds similar to the compounds of the invention are disclosed in CN108341795.

Further improvements are still necessary with respect to the performance data of OLEDs, in particular with a view to broad commercial use, for example in display devices or as light sources. Of particular importance in this connection are the lifetime, the efficiency and the operating voltage of the OLEDs and as well as the colour values achieved. In particular, in case of blue-emitting OLEDs, there is potential for improvement with respect to the lifetime and the efficiency of the devices.

An important starting point for achieving the said improvements is the choice of the emitter compound and of the host compound employed in the electronic device.

Blue-fluorescent emitters known from the prior art are a multiplicity of compounds. Arylamines containing one or more condensed aryl are known from the prior art. Arylamines containing dibenzofuran groups (as disclosed in US 2017/0012214) or indenodibenzofuran groups (as disclosed in CN 10753308 or CN107573925) are also known from the prior art.

However, there is still a need for further fluorescent emitters, especially blue-fluorescent emitters, which may be employed in OLEDs and lead to OLEDs having very good properties in terms of lifetime, color emission and efficiency. More particularly, there is a need for blue-fluorescent emitters combining very high efficiencies, very good life time and suitable color coordinates as well as high color purity.

Furthermore, it is known that an OLED may comprise different layers, which may be applied either by vapour deposition in a vacuum chamber or by processing from a solution. The processes based on vapour deposition lead to good results, but such processes are complex and expensive. Therefore, there is also a need for OLED materials that can be easily and reliably processed from solution. In this case, the materials should have good solubility properties in the solution that comprises them. Additionally, the OLED materials that are processed from a solution should be able to orientate themselves in the deposited film to improve the overall efficiency of the OLED. The term orientation means here the horizontal molecular orientation of the compounds, as explained in Zhao et al., Horizontal molecular orientation in solution-processed organic light-emitting diodes, Appl. Phys. Lett. 106063301, 2015.

The present invention is thus based on the technical object of providing compounds which are suitable for use in electronic devices, such as OLEDs, more particularly as blue-fluorescent emitters or matrix materials and, which are suitable for vacuum processing or for solution processing.

In investigations on novel compounds for use in electronic devices, it has now been found, that compounds of formula (1) as defined below are eminently suitable for use in electronic devices. In particular, they achieve one or more, preferably all, of the above-mentioned technical objects.

The invention thus relates to compounds of formula (1), 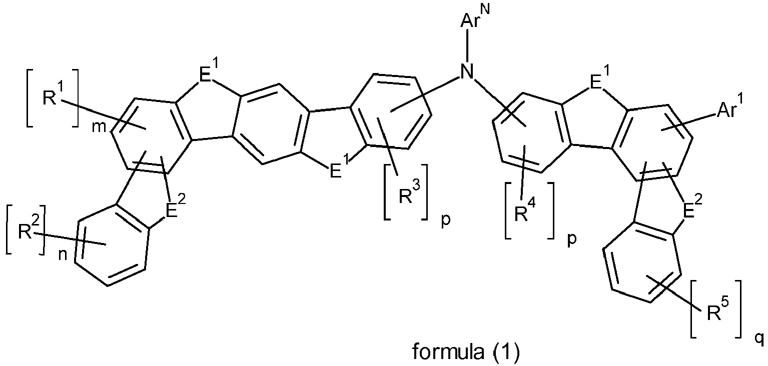 where the following applies to the symbols and indices used:
Ar¹, Ar^{N} are on each occurrence, identically or differently, selected from an aromatic or heteroaromatic ring system having 5 to 60 aromatic ring atoms, which may in each case be substituted by one or more radicals R;
E¹ is on each occurrence, identically or differently, selected from -BR⁰-, - C(R⁰)₂-, -C(R⁰)₂-C(R⁰)₂-, -C(R⁰)₂-O-, -C(R⁰)₂-S-, -R⁰C=CR⁰-, -R⁰C=N-, Si(R⁰)₂, -Si(R⁰)₂-Si(R⁰)₂-, -C(=O)-, -C(=NR⁰)-, -C(=C(R⁰)₂)-, -O-, -S-, - S(=O)-, -SO₂-, -N(R⁰)-, -P(R⁰)- and -P((=O)R⁰)-; or E¹ is a group of formula (E-1), 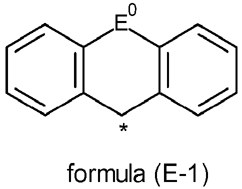
   where the symbol * in formula (E-1) indicates the corresponding group E¹ in formula (1); and
   E⁰ is identically or differently on each occurrence, selected from the group consisting of a single bond, -BR⁰-, -C(R⁰)₂-, -C(R⁰)₂-C(R⁰)₂-,-C(R⁰)₂-O-, -C(R⁰)₂-S-, -R⁰C=CR⁰-, -R⁰C=N-, Si(R⁰)₂, -Si(R⁰)₂-Si(R⁰)₂-,-C(=O)-, -C(=NR⁰)-, -C(=C(R⁰)2)-, -O-, -S-, -S(=O)-, -SO₂-, -N(R⁰)-,-P(R⁰)- and -P((=O)R⁰)-;
E² is, identically or differently, on each occurrence, selected from the group consisting of -O-, -S-, -S(=O)- and -SO₂-;
R⁰ to R⁵ stand on each occurrence, identically or differently, for H, D, F, CI, Br, I, CHO, CN, N(Ar)₂, C(=O)Ar, P(=O)(Ar)₂, S(=O)Ar, S(=O)₂Ar, NO₂, Si(R)₃, B(OR)₂, OSO₂R, a straight-chain alkyl, alkoxy or thioalkyl group having 1 to 40 C atoms or branched or cyclic alkyl, alkoxy or thioalkyl group having 3 to 40 C atoms, each of which may be substituted by one or more radicals R, where in each case one or more non-adjacent CH₂ groups may be replaced by RC=CR, C=C, Si(R)₂, Ge(R)₂, Sn(R)₂, C=O, C=S, C=Se, P(=O)(R), SO, SO₂, O, S or CONR and where one or more H atoms may be replaced by D, F, CI, Br, I, CN or NO₂, an aromatic or heteroaromatic ring system having 5 to 60 aromatic ring atoms, which may in each case be substituted by one or more radicals R, or an aryloxy group having 5 to 60 aromatic ring atoms, which may be substituted by one or more radicals R, where two adjacent substituents R⁰, and/or two adjacent substituents R¹, and/or two adjacent substituents R², and/or two adjacent substituents R³, and/or two adjacent substituents R⁴, and/or two adjacent substituents R⁵ may form a mono- or polycyclic, aliphatic ring system or aromatic ring system, which may be substituted by one or more radicals R;
m stands on each occurrence, identically or differently, for an integer selected from 0, 1 or 2;
n, q stand on each occurrence, identically or differently, for an integer selected from 0, 1, 2, 3 or 4;
p stands on each occurrence, identically or differently, for an integer selected from 0, 1, 2 or 3;
R stands on each occurrence, identically or differently, for H, D, F, CI, Br, I, CHO, CN, N(Ar)₂, C(=O)Ar, P(=O)(Ar)₂, S(=O)Ar, S(=O)₂Ar, NO₂, Si(R')₃, B(OR')₂, OSO₂R', a straight-chain alkyl, alkoxy or thioalkyl group having 1 to 40 C atoms or branched or cyclic alkyl, alkoxy or thioalkyl groups having 3 to 40 C atoms, each of which may be substituted by one or more radicals R', where in each case one or more non-adjacent CH₂ groups may be replaced by R'C=CR', C=C, Si(R')₂, Ge(R')₂, Sn(R')₂, C=O, C=S, C=Se, P(=O)(R'), SO, SO₂, O, S or CONR' and where one or more H atoms may be replaced by D, F, CI, Br, I, CN or NO₂, an aromatic or heteroaromatic ring system having 5 to 60 aromatic ring atoms, which may in each case be substituted by one or more radicals R', or an aryloxy group having 5 to 60 aromatic ring atoms, which may be substituted by one or more radicals R', where two adjacent substituents R may form a mono- or polycyclic, aliphatic ring system or aromatic ring system, which may be substituted by one or more radicals R';
Ar is on each occurrence, identically or differently, an aromatic or heteroaromatic ring system having 5 to 24 aromatic ring atoms, which may in each case also be substituted by one or more radicals R';
R' stands on each occurrence, identically or differently, for H, D, F, CI, Br, I, CN, a straight-chain alkyl, alkoxy or thioalkyl group having 1 to 20 C atoms or branched or cyclic alkyl, alkoxy or thioalkyl group having 3 to 20 C atoms, where in each case one or more non-adjacent CH₂ groups may be replaced by SO, SO₂, O, S and where one or more H atoms may be replaced by D, F, CI, Br or I, or an aromatic or heteroaromatic ring system having 5 to 24 C atoms.

Adjacent substituents in the sense of the present invention are substituents which are bonded to atoms which are linked directly to one another or which are bonded to the same atom.

Furthermore, the following definitions of chemical groups apply for the purposes of the present application:
An aryl group in the sense of this invention contains 6 to 60 aromatic ring atoms, preferably 6 to 40 aromatic ring atoms, more preferably 6 to 20 aromatic ring atoms; a heteroaryl group in the sense of this invention contains 5 to 60 aromatic ring atoms, preferably 5 to 40 aromatic ring atoms, more preferably 5 to 20 aromatic ring atoms, at least one of which is a heteroatom.

The heteroatoms are preferably selected from N, O and S. This represents the basic definition. If other preferences are indicated in the description of the present invention, for example with respect to the number of aromatic ring atoms or the heteroatoms present, these apply.

An aryl group or heteroaryl group here is taken to mean either a simple aromatic ring, i.e. benzene, or a simple heteroaromatic ring, for example pyridine, pyrimidine or thiophene, or a condensed (annellated) aromatic or heteroaromatic polycycle, for example naphthalene, phenanthrene, quinoline or carbazole. A condensed (annellated) aromatic or heteroaromatic polycycle in the sense of the present application consists of two or more simple aromatic or heteroaromatic rings condensed with one another.

An aryl or heteroaryl group, which may in each case be substituted by the above-mentioned radicals and which may be linked to the aromatic or heteroaromatic ring system via any desired positions, is taken to mean, in particular, groups derived from benzene, naphthalene, anthracene, phenanthrene, pyrene, dihydropyrene, chrysene, perylene, fluoranthene, benzanthracene, benzophenanthrene, tetracene, pentacene, benzopyrene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, pyrazine, phenazine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

An aryloxy group in accordance with the definition of the present invention is taken to mean an aryl group, as defined above, which is bonded via an oxygen atom. An analogous definition applies to heteroaryloxy groups.

An aromatic ring system in the sense of this invention contains 6 to 60 C atoms in the ring system, preferably 6 to 40 C atoms, more preferably 6 to 20 C atoms. A heteroaromatic ring system in the sense of this invention contains 5 to 60 aromatic ring atoms, preferably 5 to 40 aromatic ring atoms, more preferably 5 to 20 aromatic ring atoms, at least one of which is a heteroatom. The heteroatoms are preferably selected from N, O and/or S. An aromatic or heteroaromatic ring system in the sense of this invention is intended to be taken to mean a system which does not necessarily contain only aryl or heteroaryl groups, but instead in which, in addition, a plurality of aryl or heteroaryl groups may be connected by a non-aromatic unit (preferably less than 10% of the atoms other than H), such as, for example, an sp³-hybridised C, Si, N or O atom, an sp²-hybridised C or N atom or an sp-hybridised C atom. Thus, for example, systems such as 9,9'-spirobifluorene, 9,9'-diarylfluorene, triarylamine, diaryl ether, stilbene, etc., are also intended to be taken to be aromatic ring systems in the sense of this invention, as are systems in which two or more aryl groups are connected, for example, by a linear or cyclic alkyl, alkenyl or alkynyl group or by a silyl group. Furthermore, systems in which two or more aryl or heteroaryl groups are linked to one another via single bonds are also taken to be aromatic or heteroaromatic ring systems in the sense of this invention, such as, for example, systems such as biphenyl, terphenyl or diphenyltriazine.

An aromatic or heteroaromatic ring system having 5 - 60 aromatic ring atoms, which may in each case also be substituted by radicals as defined above and which may be linked to the aromatic or heteroaromatic group via any desired positions, is taken to mean, in particular, groups derived from benzene, naphthalene, anthracene, benzanthracene, phenanthrene, benzophenanthrene, pyrene, chrysene, perylene, fluoranthene, naphthacene, pentacene, benzopyrene, biphenyl, biphenylene, terphenyl, terphenylene, quaterphenyl, fluorene, spirobifluorene, dihydrophenanthrene, dihydropyrene, tetrahydropyrene, cis- or trans-indenofluorene, truxene, isotruxene, spirotruxene, spiroisotruxene, furan, benzofuran, isobenzofuran, dibenzofuran, thiophene, benzothiophene, isobenzothiophene, dibenzothiophene, pyrrole, indole, isoindole, carbazole, indolocarbazole, indenocarbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, 1,5-diazaanthracene, 2,7-diazapyrene, 2,3-diazapyrene, 1,6-diazapyrene, 1,8-diazapyrene, 4,5-diazapyrene, 4,5,9,10-tetraazaperylene, pyrazine, phenazine, phenoxazine, phenothiazine, fluorubin, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole, or combinations of these groups.

For the purposes of the present invention, a straight-chain alkyl group having 1 to 40 C atoms or a branched or cyclic alkyl group having 3 to 40 C atoms or an alkenyl or alkynyl group having 2 to 40 C atoms, in which, in addition, individual H atoms or CH₂ groups may be substituted by the groups mentioned above under the definition of the radicals, is preferably taken to mean the radicals methyl, ethyl, n-propyl, i-propyl, n-butyl, i-butyl, s-butyl, t-butyl, 2-methylbutyl, n-pentyl, s-pentyl, cyclopentyl, neopentyl, n-hexyl, cyclohexyl, neohexyl, n-heptyl, cycloheptyl, n-octyl, cyclooctyl, 2-ethylhexyl, trifluoromethyl, pentafluoroethyl, 2,2,2-trifluoroethyl, ethenyl, propenyl, butenyl, pentenyl, cyclopentenyl, hexenyl, cyclohexenyl, heptenyl, cycloheptenyl, octenyl, cyclooctenyl, ethynyl, propynyl, butynyl, pentynyl, hexynyl or octynyl. An alkoxy or thioalkyl group having 1 to 40 C atoms is preferably taken to mean methoxy, trifluoromethoxy, ethoxy, n-propoxy, i-propoxy, n-butoxy, i-butoxy, s-butoxy, t-butoxy, n-pentoxy, s-pentoxy, 2-methylbutoxy, n-hexoxy, cyclohexyloxy, n-heptoxy, cycloheptyloxy, n-octyloxy, cyclooctyloxy, 2-ethylhexyloxy, pentafluoroethoxy, 2,2,2-trifluoroethoxy, methylthio, ethylthio, n-propylthio, i-propylthio, n-butylthio, i-butylthio, s-butylthio, t-butylthio, n-pentylthio, s-pentylthio, n-hexylthio, cyclohexylthio, n-heptylthio, cycloheptylthio, n-octylthio, cyclooctylthio, 2-ethylhexylthio, trifluoromethylthio, pentafluoroethylthio, 2,2,2-trifluoroethylthio, ethenylthio, propenylthio, butenylthio, pentenylthio, cyclopentenylthio, hexenylthio, cyclohexenylthio, heptenylthio, cycloheptenylthio, octenylthio, cyclooctenylthio, ethynylthio, propynylthio, butynylthio, pentynylthio, hexynylthio, heptynylthio or octynylthio.

The formulation that two or more radicals may form a ring with one another is, for the purposes of the present application, intended to be taken to mean, inter alia, that the two radicals are linked to one another by a chemical bond. This is illustrated by the following schemes:

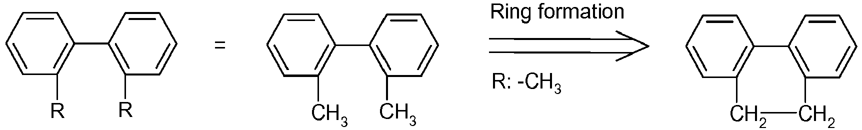

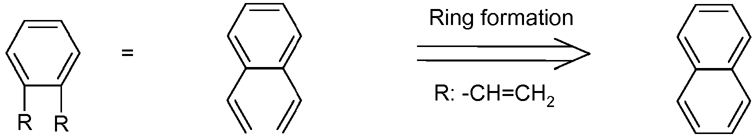

Furthermore, however, the above-mentioned formulation is also intended to be taken to mean that, in the case where one of the two radicals represents hydrogen, the second radical is bonded at the position to which the hydrogen atom was bonded, with formation of a ring. This is illustrated by the following scheme:

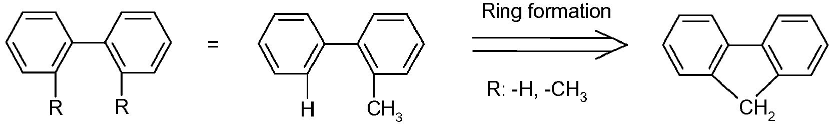

In accordance with a preferred embodiment, the group E¹ is on each occurrence, identically or differently, selected from -C(R⁰)₂-, -Si(R⁰)₂-, -O-, - S-, -N(R⁰)-; or E¹ is a group of formula (E-1), 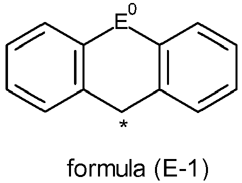 where the symbol * in formula (E-1) indicates the corresponding group E¹ in formula (1) and where E⁰ has the same meaning as above.

In accordance with a very preferred embodiment, the group E¹ stands for - C(R⁰)₂-.

In accordance with another very preferred embodiment, the group E¹ stands for a group of formula (E-1) where, E⁰ stands for a single bond or -C(R⁰)₂-.

Preferably, the group E² is on each occurrence, identically or differently, selected from -O- or -S-. Very preferably, the group E² stands for -O-.

Preferably, Ar^{N} stands for phenyl, biphenyl, fluorene, spirobifluorene, naphthalene, phenanthrene, dibenzofuran, dibenzothiophene, carbazole, pyridine, pyrimidine, pyrazine, pyridazine, triazine, benzopyridine, benzopyridazine, benzopyrimidine or quinazoline, or for a combination of two to six of these groups, each of which may be substituted by one or more radicals R.

Very preferably, Ar^{N} stands for phenyl, biphenyl, fluorene, naphthalene, phenanthrene, dibenzofuran, dibenzothiophene, carbazole, or for a combination of two to six of these groups, each of which may be substituted by one or more radicals R.

Particularly preferably, Ar^{N} stands for a group of one of the formulae (ArN-1) to (ArN-22) as depicted in the table below:

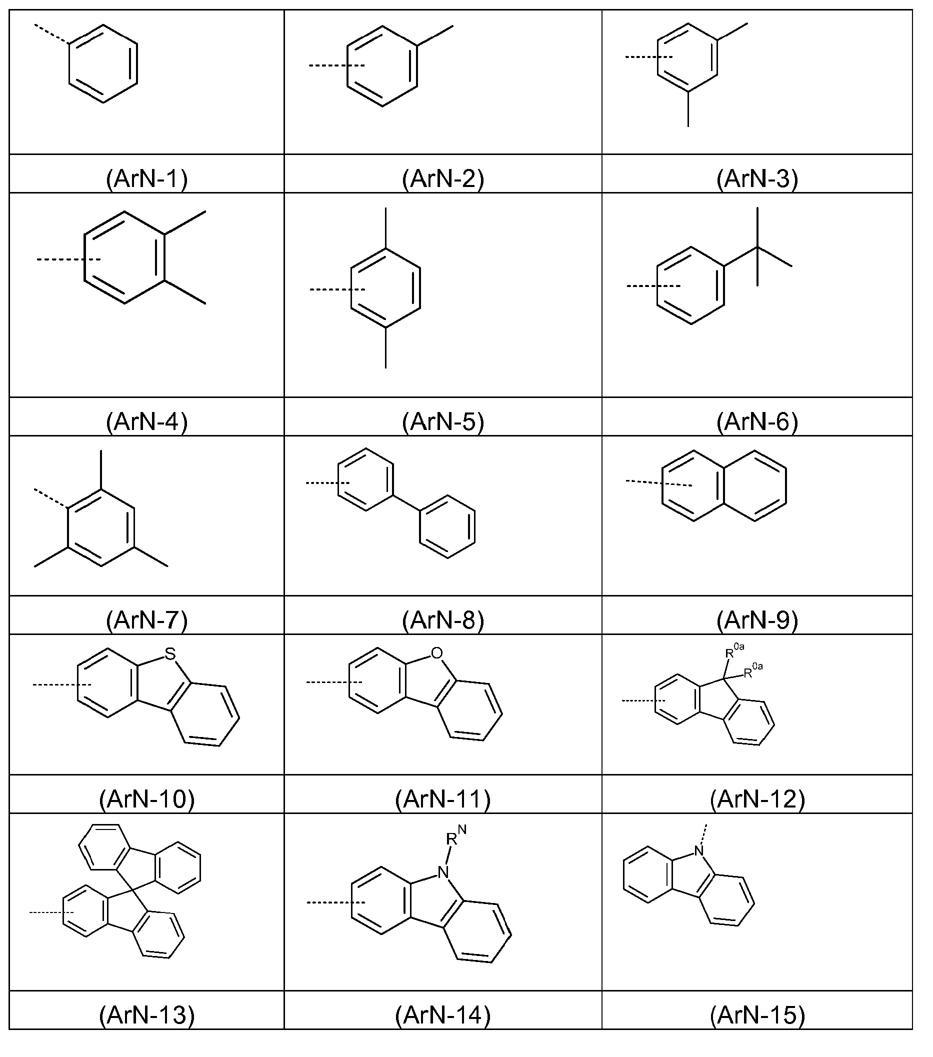

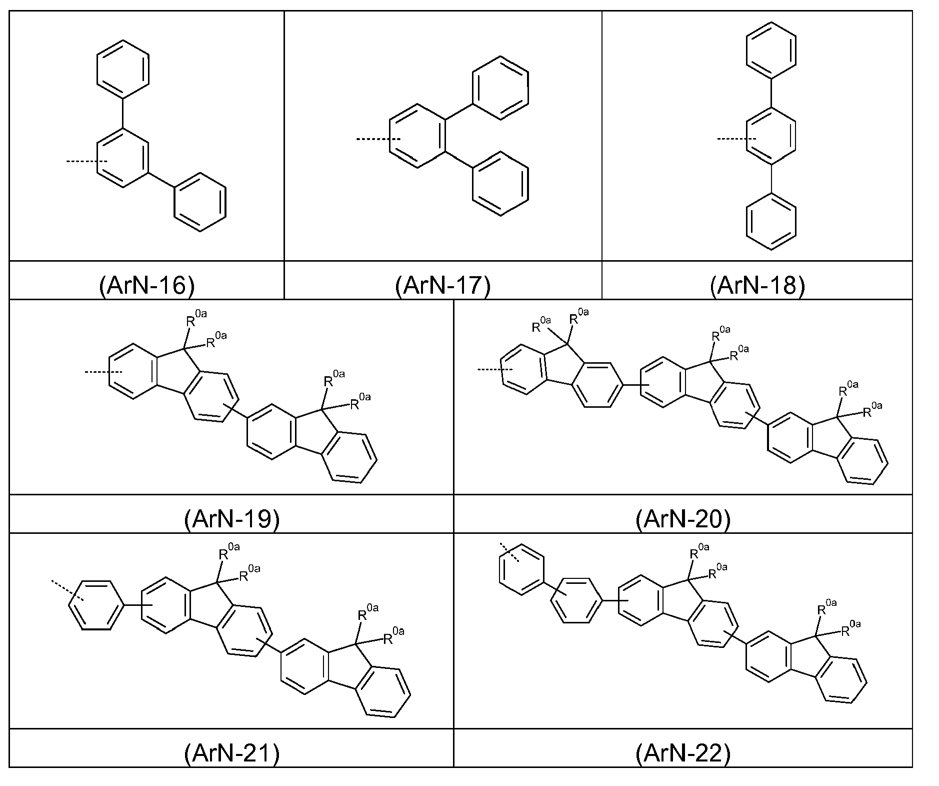

where in formulae (ArN-1) to (ArN-22):
- the dashed bond indicates the bonding to the nitrogen of the structure of formula (1);
- the group R^{N} in formula (ArN-14) stands on each occurrence, identically or differently, for H, D, a straight-chain alkyl group having 1 to 40, preferably 1 to 20, more preferably 1 to 10 C atoms or branched or cyclic alkyl group having 3 to 40, preferably 3 to 20, more preferably 3 to 10 C atoms, each of which may be substituted by one or more radicals R, where in each case one or more non-adjacent CH₂ groups may be replaced by RC=CR, C=C, C=O, C=S, SO, SO₂, O or S, and where one or more H atoms may be replaced by D, F or CN, an aromatic or heteroaromatic ring system having 5 to 60, preferably 5 to 40, more preferably 5 to 30, particularly preferably 5 to 18 aromatic ring atoms, which may in each case be substituted by one or more radicals R, where two adjacent substituents R^{N} may form a mono- or polycyclic, aliphatic ring system or aromatic ring system, which may be substituted by one or more radicals R, where R has the same meaning as above;
- the group R^{0a} in formulae (ArN-12) and (ArN-19) to (ArN-22) has the same definition as the group R⁰ as defined above; and
- the groups of formulae (ArN-1) to (ArN-22) may be substituted at each free position by a group R, which has the same meaning as above.

In accordance with a preferred embodiment, the group Ar¹ stands on each occurrence, identically or differently, for an aromatic or heteroaromatic ring system selected from phenyl, biphenyl, fluorene, spirobifluorene, naphthalene, phenanthrene, dibenzofuran, dibenzothiophene, carbazole, pyridine, pyrimidine, pyrazine, pyridazine, triazine, benzopyridine, benzopyridazine, benzopyrimidine, quinazoline, or for a combination of two to six of these groups, each of which may be substituted by one or more radicals R.

Very preferably, the group Ar¹ stands on each occurrence, identically or differently, for an aromatic or heteroaromatic ring system selected from phenyl, biphenyl, fluorene or naphthalene, each of which may be substituted by one or more radicals R, or for a combination of two to six groups selected from phenyl, biphenyl, fluorene or naphthalene, each of which may be substituted by one or more radicals R.

Examples of suitable groups Ar¹ are the groups of formulae (Ar1-1) to (Ar1-22) represented below:

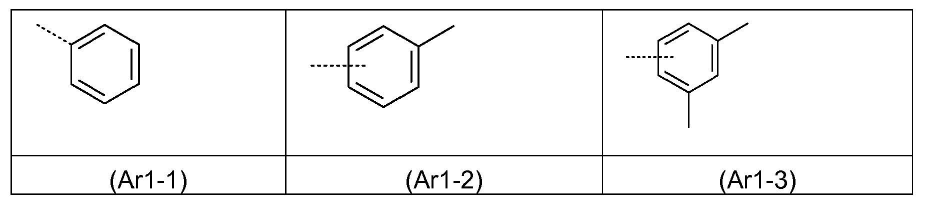

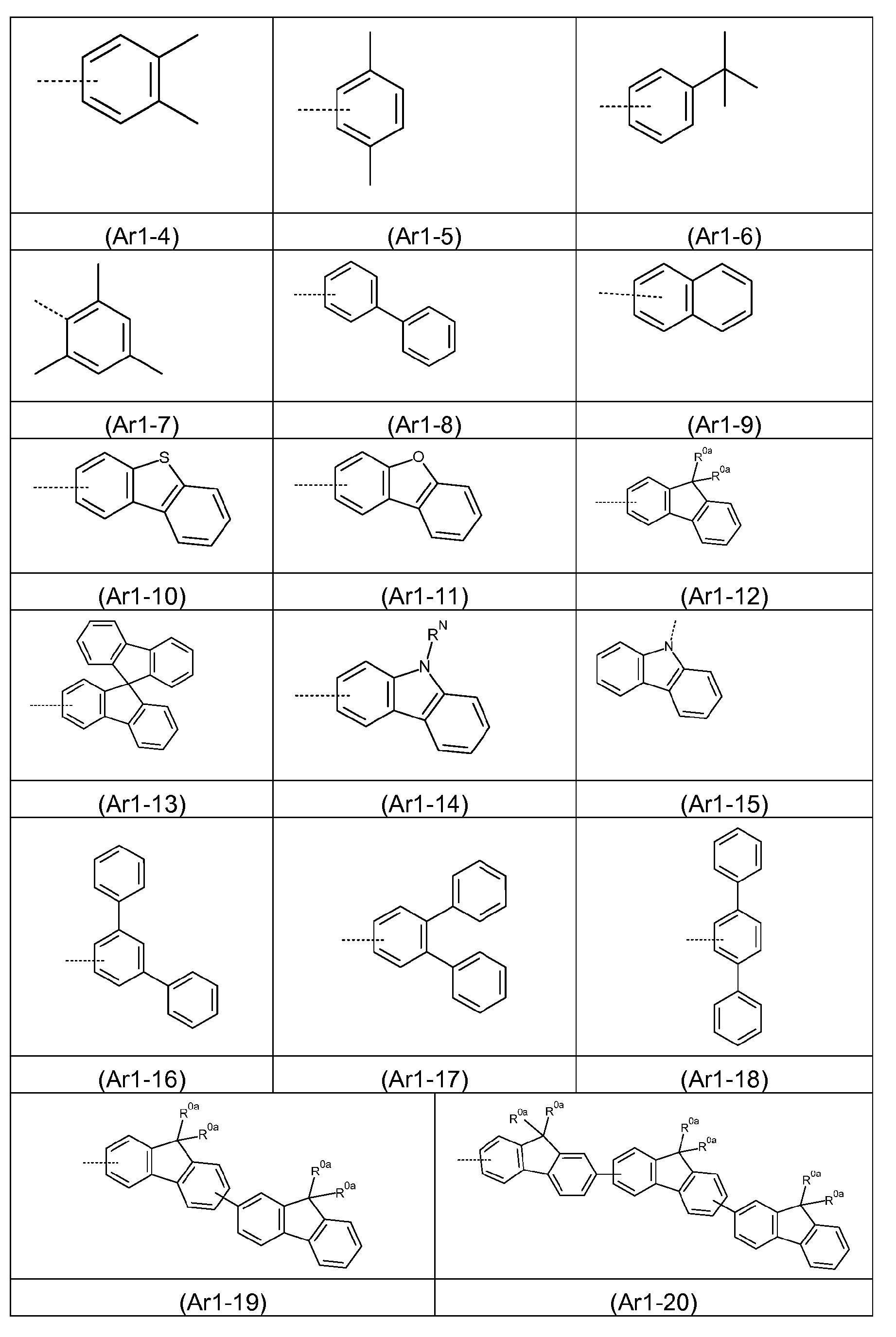

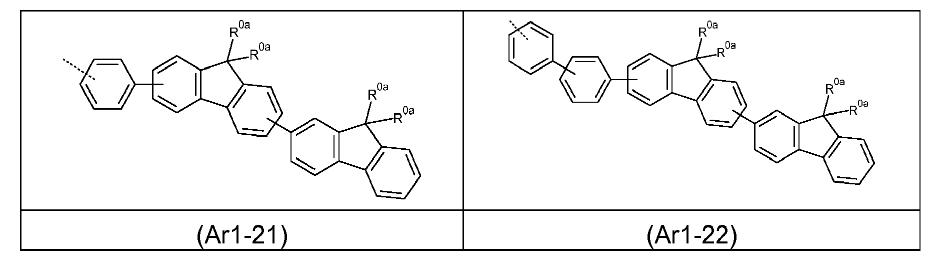

where in formulae (Ar1-1) to (Ar1-22):
- the dashed bond indicates the bonding to the structure of formula (1);
- the group R^{N} in formula (Ar1-14) has the same definition as above;
- the group R^{0a} has the same definition as above; and
- the groups of formulae (Ar1-1) to (Ar1-22) may be substituted at each free position by a group R, which has the same meaning as above.

Preferably, the compounds of formula (1) are selected from compounds of formula (2), 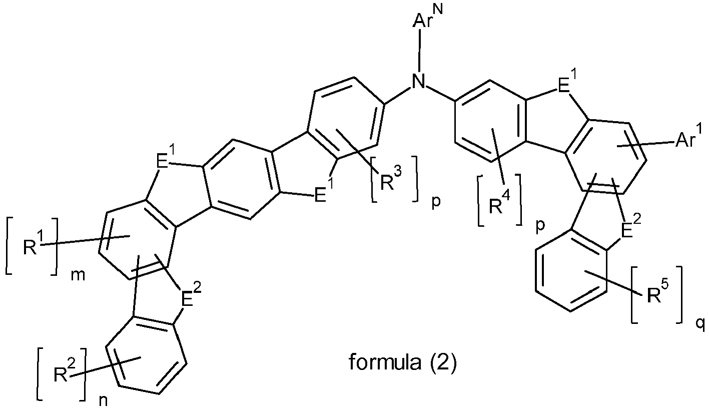 where the symbols Ar^{N}, E¹, E², Ar¹, R¹ to R⁵ and the indices m, n, p and q have the same meaning as above.

Very preferably, the compounds of formula (1) are selected from compounds of formula (3), 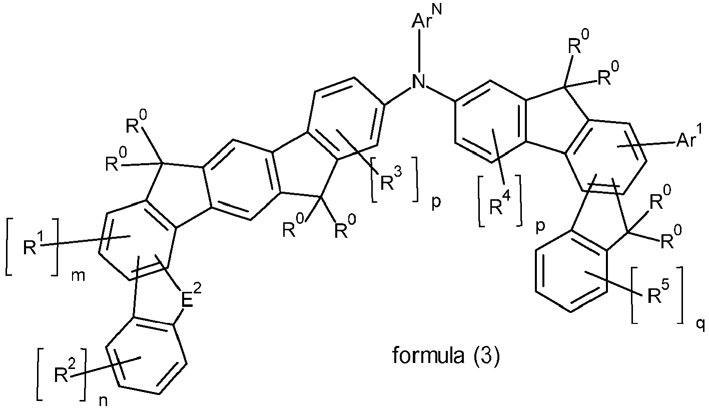 where the symbols Ar^{N}, E², Ar¹, R⁰, R¹ to R⁵ and the indices m, n, p and q have the same meaning as above.

Particularly preferably, the compounds of formula (1) are selected from compounds of formula (4), 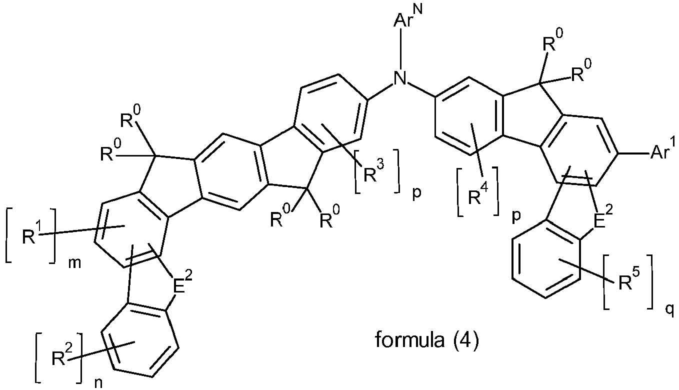 where the symbols Ar^{N}, E², Ar¹, R⁰, R¹ to R⁵ and the indices m, n, p and q have the same meaning as above.

Very particularly preferably, the compounds of formula (1) are selected from compounds of formulae (4-1) to (4-4), 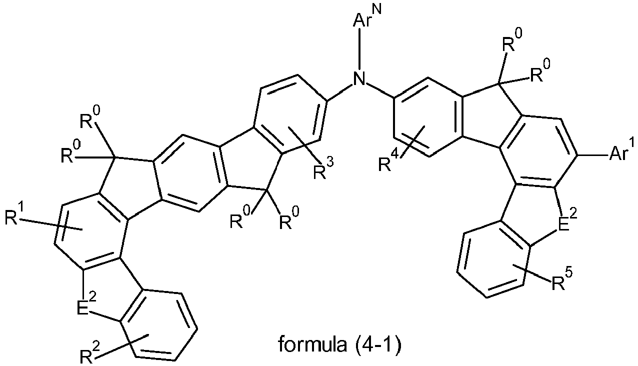 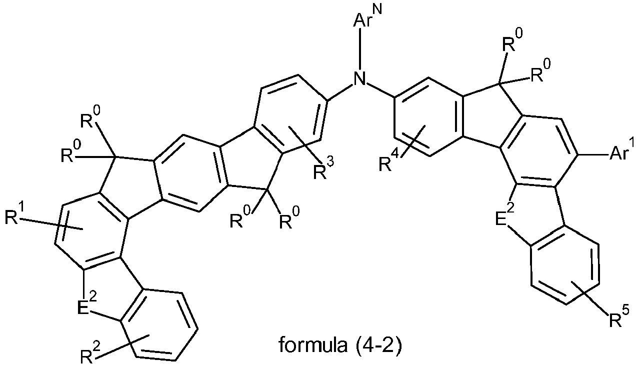 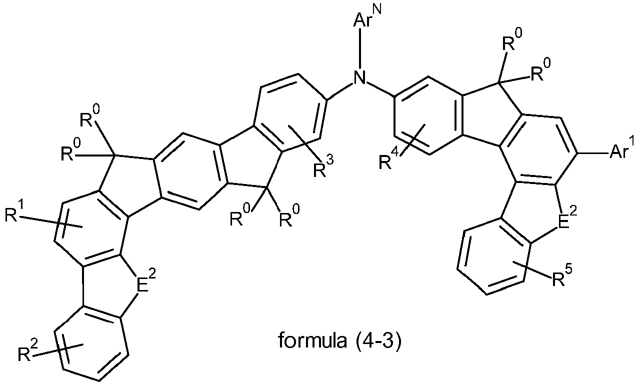 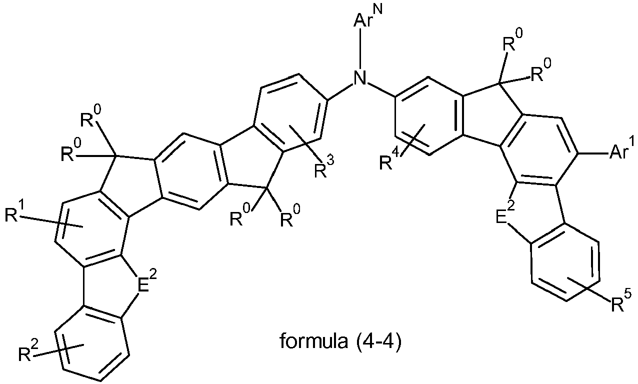 where the symbols Ar^{N}, E², Ar¹, R⁰, R¹ to R⁵ have the same meaning as above.

In accordance with a preferred embodiment, the compounds of formulae (4-1) to (4-4) are selected from compounds of formulae (4-1a) to (4-4a), 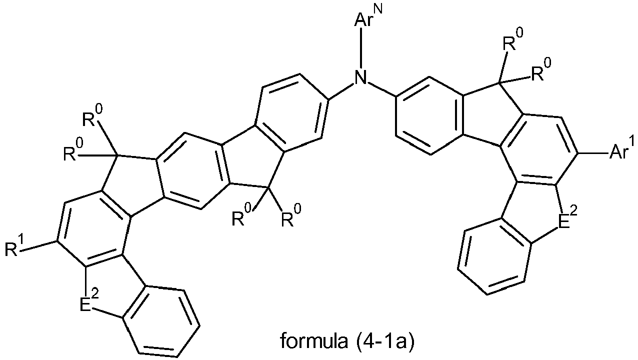 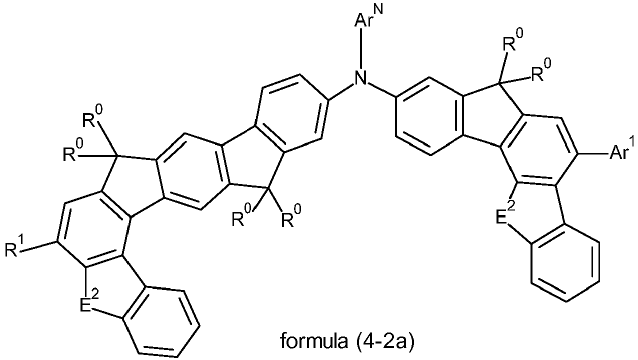 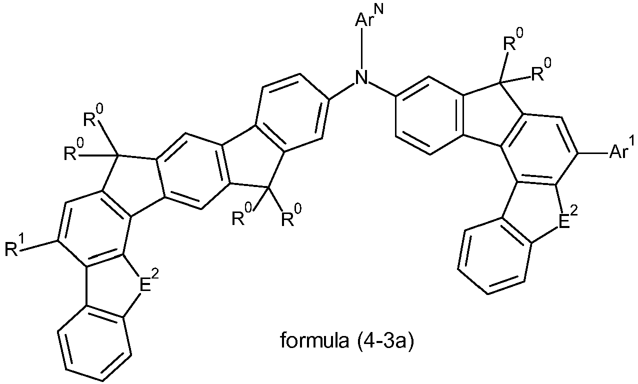 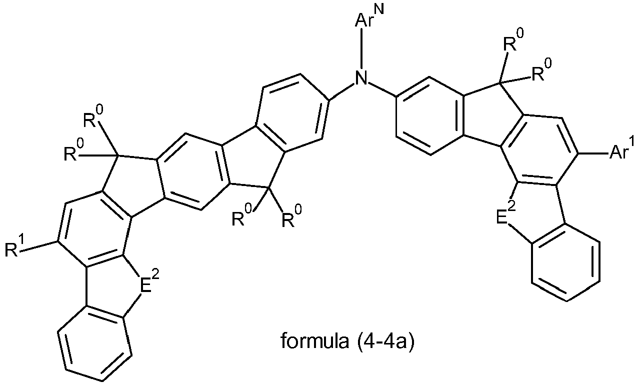 where the symbols Ar^{N}, E², Ar¹, R⁰ and R¹ have the same meaning as above.

In accordance with another preferred embodiment, the compounds of formulae (4-1) to (4-4) are selected from compounds of formulae (4-1b) to (4-4b), 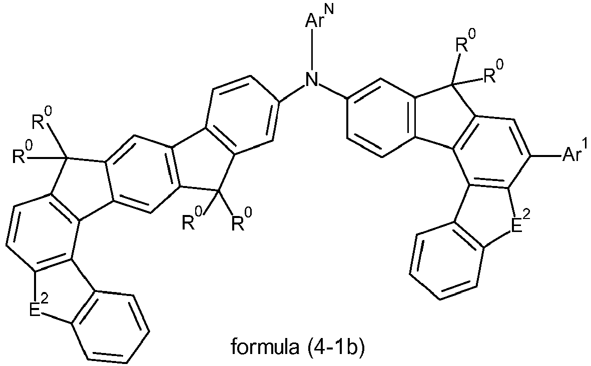 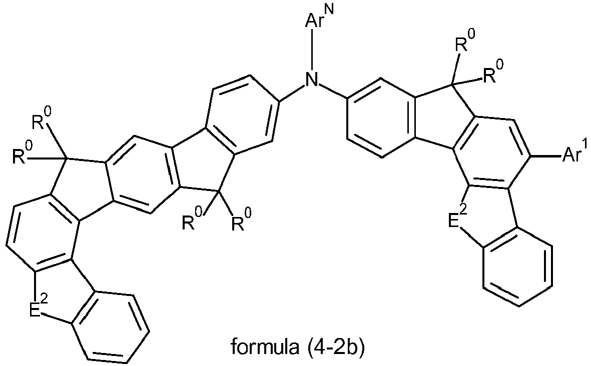 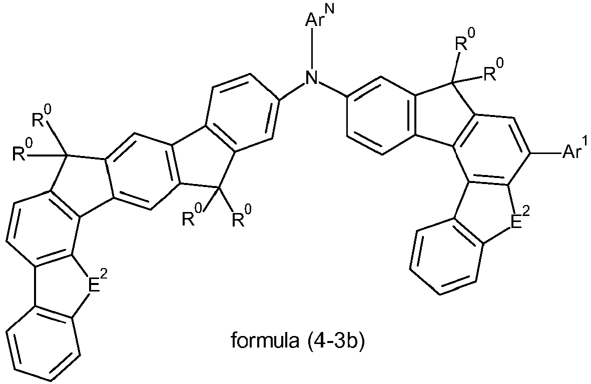 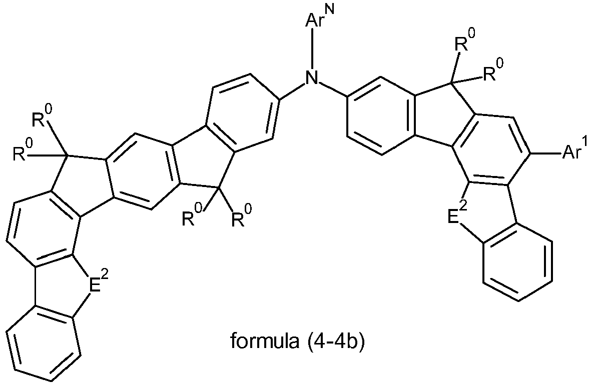 where the symbols Ar^{N}, E², Ar¹ and R⁰ have the same meaning as above.

Preferably, the group R⁰ and R^{0a} stand on each occurrence, identically or differently, for H, D, F, CN, a straight-chain alkyl group having 1 to 40, preferably 1 to 20, more preferably 1 to 10 C atoms or branched or cyclic alkyl group having 3 to 40, preferably 3 to 20, more preferably 3 to 10 C atoms, each of which may be substituted by one or more radicals R, where in each case one or more non-adjacent CH₂ groups may be replaced by RC=CR, C=C, C=O, C=S, SO, SO₂, O or S and where one or more H atoms may be replaced by D, F, CI, Br, I, CN or NO₂, an aromatic or heteroaromatic ring system having 5 to 60, preferably 5 to 40, more preferably 5 to 25, even more preferably 5 to 18 aromatic ring atoms, which may in each case be substituted by one or more radicals R; where two adjacent substituents R⁰ and/or R^{0a} may form a mono- or polycyclic, aliphatic ring system or aromatic ring system, which may be substituted by one or more radicals R.

Very preferably, the groups R⁰ and R^{0a} stand on each occurrence, identically or differently, for H, a straight-chain alkyl group having 1 to 10 C atoms or branched or cyclic alkyl group having 3 to 10 C atoms, each of which may be substituted by one or more radicals R, where one or more H atoms may be replaced by D, F, CI, Br, I, CN or NO₂, an aromatic or heteroaromatic ring system having 5 to 18 aromatic ring atoms, which may in each case be substituted by one or more radicals R, where two adjacent substituents R⁰ and/or R^{0a} may form a mono- or polycyclic, aliphatic ring system or aromatic ring system, which may be substituted by one or more radicals R.

Particularly preferably, the groups R⁰ and/or R^{0a} stand on each occurrence, identically or differently, for a straight-chain alkyl group having 1 to 10 C atoms, which may be substituted by one or more radicals R, or an aromatic or heteroaromatic ring system having 5 to 18 aromatic ring atoms, which may in each case be substituted by one or more radicals R, where two adjacent substituents R⁰ may form a mono- or polycyclic, aliphatic ring system or aromatic ring system, which may be substituted by one or more radicals R.

Very particularly preferably, R⁰ stands on each occurrence, identically or differently, for a methyl or a phenyl group, which may be substituted by one or more radicals R.

Very particularly preferably, the group R^{0a} stand on each occurrence, identically or differently, for a straight-chain alkyl group having 1 to 10 C atoms, which may be substituted by one or more radicals R.

In accordance with a preferred embodiment, the groups of formulae (ArN-12) and (Ar1-12) comprise 1 or 2 groups R^{0a}, which stand(s) for a straight-chain alkyl group having 3 to 10, preferably 5 to 10, more preferably 6 to 10 C atoms and the groups or formulae (ArN-19) to (ArN-22), (Ar1-19) to (Ar1-22) comprise 1, 2, 3 or 4 groups R^{0a}, which stand(s) for a straight-chain alkyl group having 3 to 10, preferably 5 to 10, more preferably 6 to 10 C atoms.

Preferably, R¹ to R⁵ stand on each occurrence, identically or differently, for H, D, F, CN, N(Ar)₂, Si(R)₃, a straight-chain alkyl, alkoxy or thioalkyl group having 1 to 40, preferably 1 to 20, more preferably 1 to 10 C atoms or branched or cyclic alkyl, alkoxy or thioalkyl group having 3 to 40, preferably 3 to 20, more preferably 3 to 10 C atoms, each of which may be substituted by one or more radicals R, where in each case one or more non-adjacent CH₂ groups may be replaced by RC=CR, C=C, C=O, C=S, SO, SO₂, O or S and where one or more H atoms may be replaced by D, F, CI, Br, I, CN or NO₂, an aromatic or heteroaromatic ring system having 5 to 60, preferably 5 to 40, more preferably 5 to 25, even more preferably 5 to 18 aromatic ring atoms, which may in each case be substituted by one or more radicals R, or an aryloxy group having 5 to 60, preferably 5 to 40, more preferably 5 to 25, even more preferably 5 to 18 aromatic ring atoms, which may be substituted by one or more radicals R, where two adjacent substituents R¹, and/or two adjacent substituents R², and/or two adjacent substituents R³, and/or two adjacent substituents R⁴, and/or two adjacent substituents R⁵ may form a mono- or polycyclic, aliphatic ring system or aromatic ring system, which may be substituted by one or more radicals R.

More preferably, R¹ stands on each occurrence, identically or differently, for H, D, F, CN, a straight-chain alkyl group having 1 to 40, preferably 1 to 20, more preferably 1 to 10 C atoms or branched or cyclic alkyl group having 3 to 40, preferably 3 to 20, more preferably 3 to 10 C atoms, each of which may be substituted by one or more radicals R, an aromatic or heteroaromatic ring system having 5 to 60, preferably 5 to 40, more preferably 5 to 25, even more preferably 5 to 18 aromatic ring atoms, which may in each case be substituted by one or more radicals R, which may be substituted by one or more radicals R.

In accordance with a preferred embodiment, R¹ is H.

In accordance with another preferred embodiment, the compounds of formula (1) comprise at least one group R¹ selected from an aromatic or heteroaromatic ring system having 5 to 60 aromatic ring atoms.

When the compounds of formula (1) comprise at least one group R¹ selected from an aromatic or heteroaromatic ring system having 5 to 60 aromatic ring atoms, then R¹ stands preferably for a group selected from phenyl, biphenyl, fluorene, spirobifluorene, naphthalene, phenanthrene, dibenzofuran, dibenzothiophene, carbazole, pyridine, pyrimidine, pyrazine, pyridazine, triazine, benzopyridine, benzopyridazine, benzopyrimidine, quinazoline, or a combination of two to six of these groups, each of which may be substituted by one or more radicals R.

When the compounds of formula (1) comprise at least one group R¹ selected from an aromatic or heteroaromatic ring system having 5 to 60 aromatic ring atoms, then R¹ stands very preferably for a group selected from phenyl, biphenyl, fluorene and naphthalene, each of which may be substituted by one or more radicals R, or for a combination of two to six groups selected from phenyl, biphenyl, fluorene or naphthalene, each of which may be substituted by one or more radicals R.

Particularly preferably, the compounds of formula (1) comprise at least one group R¹, which stands for a group selected from formulae (Ar1-1) to (Ar1-22) as represented above. Very particularly preferably, the compounds of formula (1) comprise at least one group R¹, which stands for a group selected from formulae (Ar1-19) to (Ar1-22) as represented above.

In accordance with a preferred embodiment, the groups R² to R⁵ stand on each occurrence, identically or differently, for H, D, F, CN, a straight-chain alkyl group having 1 to 40, preferably 1 to 20, more preferably 1 to 10 C atoms or a branched or cyclic alkyl group having 3 to 40, preferably 3 to 20, more preferably 3 to 10 C atoms, each of which may be substituted by one or more radicals R, or an aromatic or heteroaromatic ring system having 5 to 18 aromatic ring atoms, which may in each case be substituted by one or more radicals R. Particularly preferably, R² to R⁵ stand on each occurrence, identically or differently, for H, a straight-chain alkyl group having 1 to 10 C atoms or a branched or cyclic alkyl group having 3 to 10 C atoms, each of which may be substituted by one or more radicals R, or an aromatic or heteroaromatic ring system having 5 to 18 aromatic ring atoms, which may in each case be substituted by one or more radicals R. Very particularly preferably, R² to R⁵ stand for H.

In accordance with a preferred embodiment, R stands on each occurrence, identically or differently, for H, D, F, CN, N(Ar)₂, Si(R')₃, a straight-chain alkyl, alkoxy or thioalkyl group having 1 to 40, preferably 1 to 20, more preferably 1 to 10 C atoms or branched or cyclic alkyl, alkoxy or thioalkyl group having 3 to 40, preferably 3 to 20, more preferably 3 to 10 C atoms, each of which may be substituted by one or more radicals R', where in each case one or more non-adjacent CH₂ groups may be replaced by R'C=CR', C=C, C=O, C=S, SO, SO₂, O or S and where one or more H atoms may be replaced by D, F, CI, Br, I, CN or NO₂, an aromatic or heteroaromatic ring system having 5 to 60, preferably 5 to 40, more preferably 5 to 25, even more preferably 5 to 18 aromatic ring atoms, which may in each case be substituted by one or more radicals R', or an aryloxy group having 5 to 60, preferably 5 to 40, more preferably 5 to 25, even more preferably 5 to 18 aromatic ring atoms, which may be substituted by one or more radicals R', where two adjacent substituents R may form a mono- or polycyclic, aliphatic ring system or aromatic ring system, which may be substituted by one or more radicals R'.

In accordance with a preferred embodiment, R' stands on each occurrence, identically or differently, for H, D, F, CI, Br, I, CN, a straight-chain alkyl group having 1 to 10 C atoms or branched or cyclic alkyl group having 3 to 10 C atoms, or an aromatic or heteroaromatic ring system having 5 to 18 C atoms.

The following compounds are examples of compounds of formula (1):

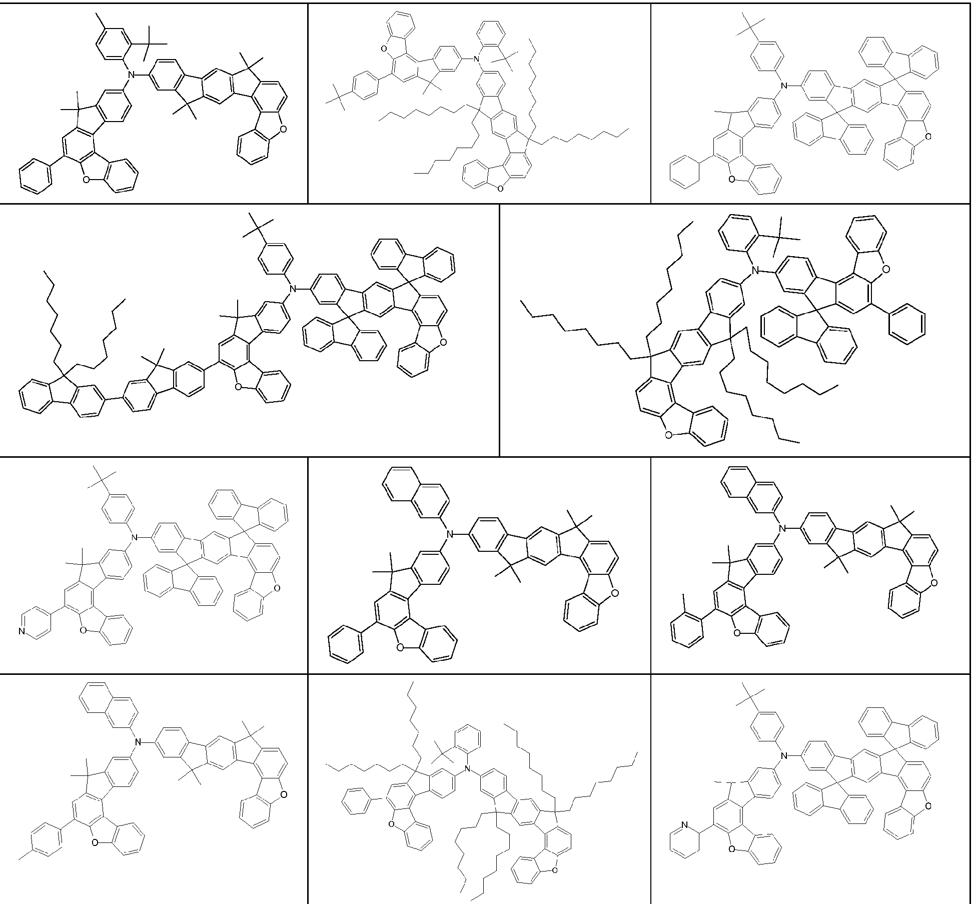

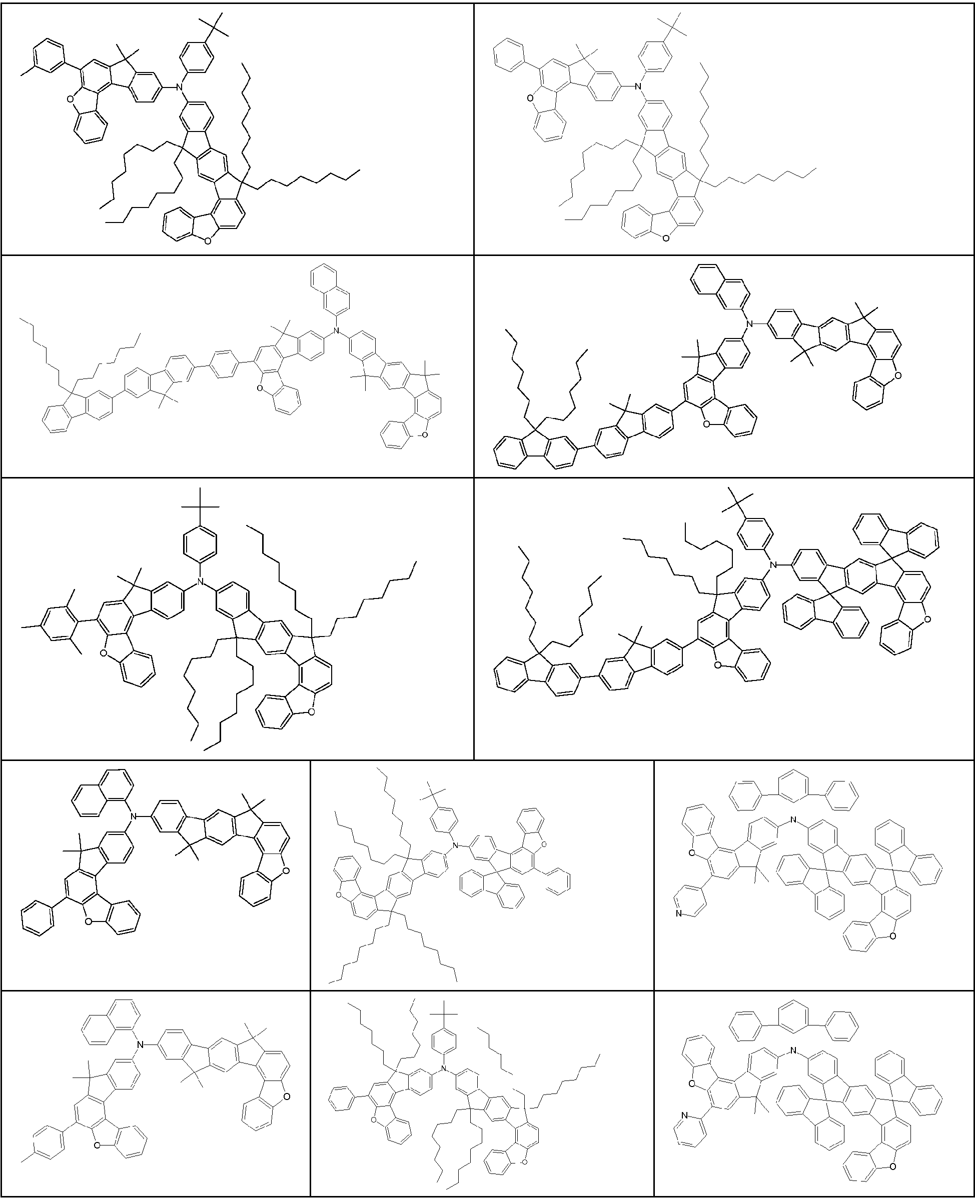

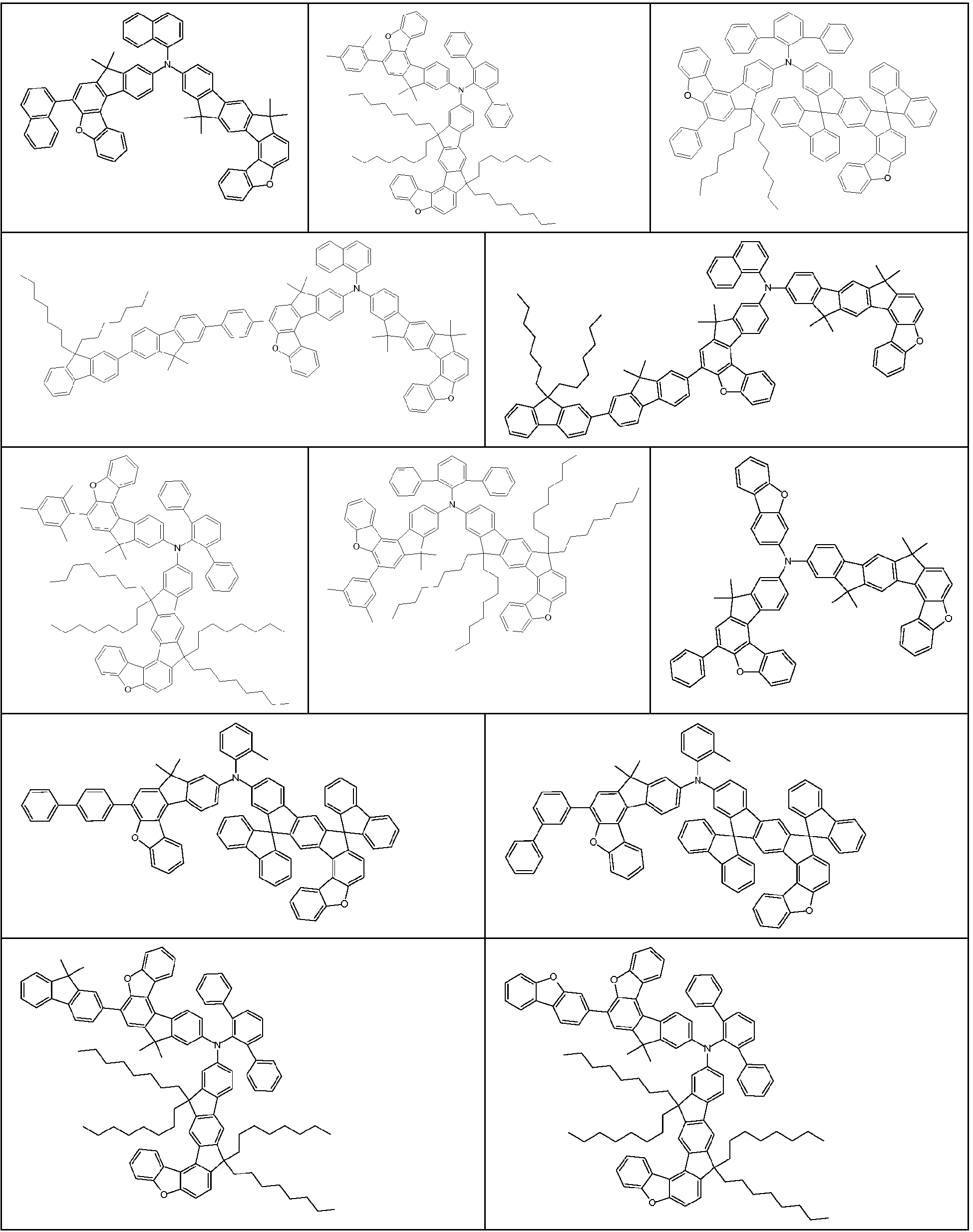

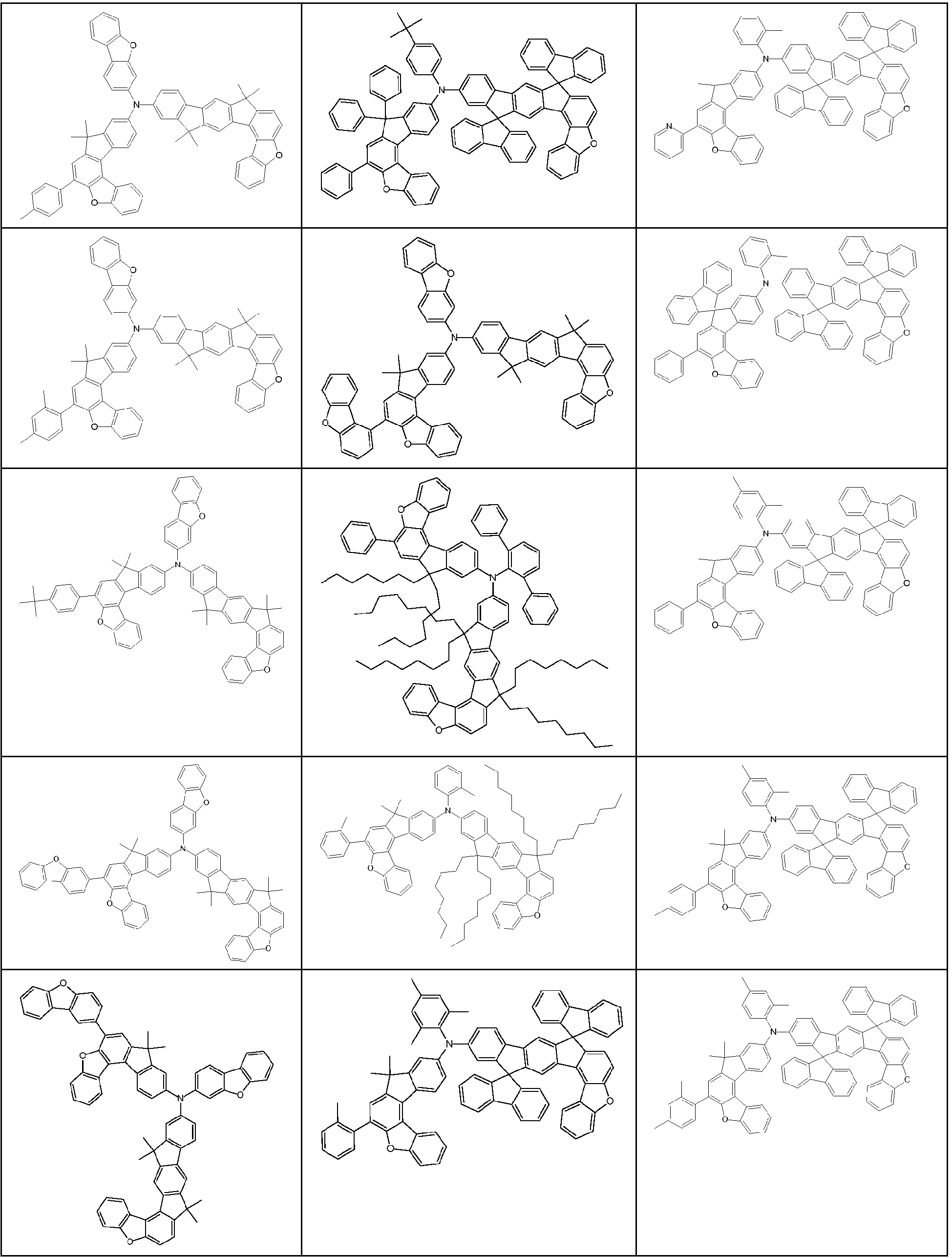

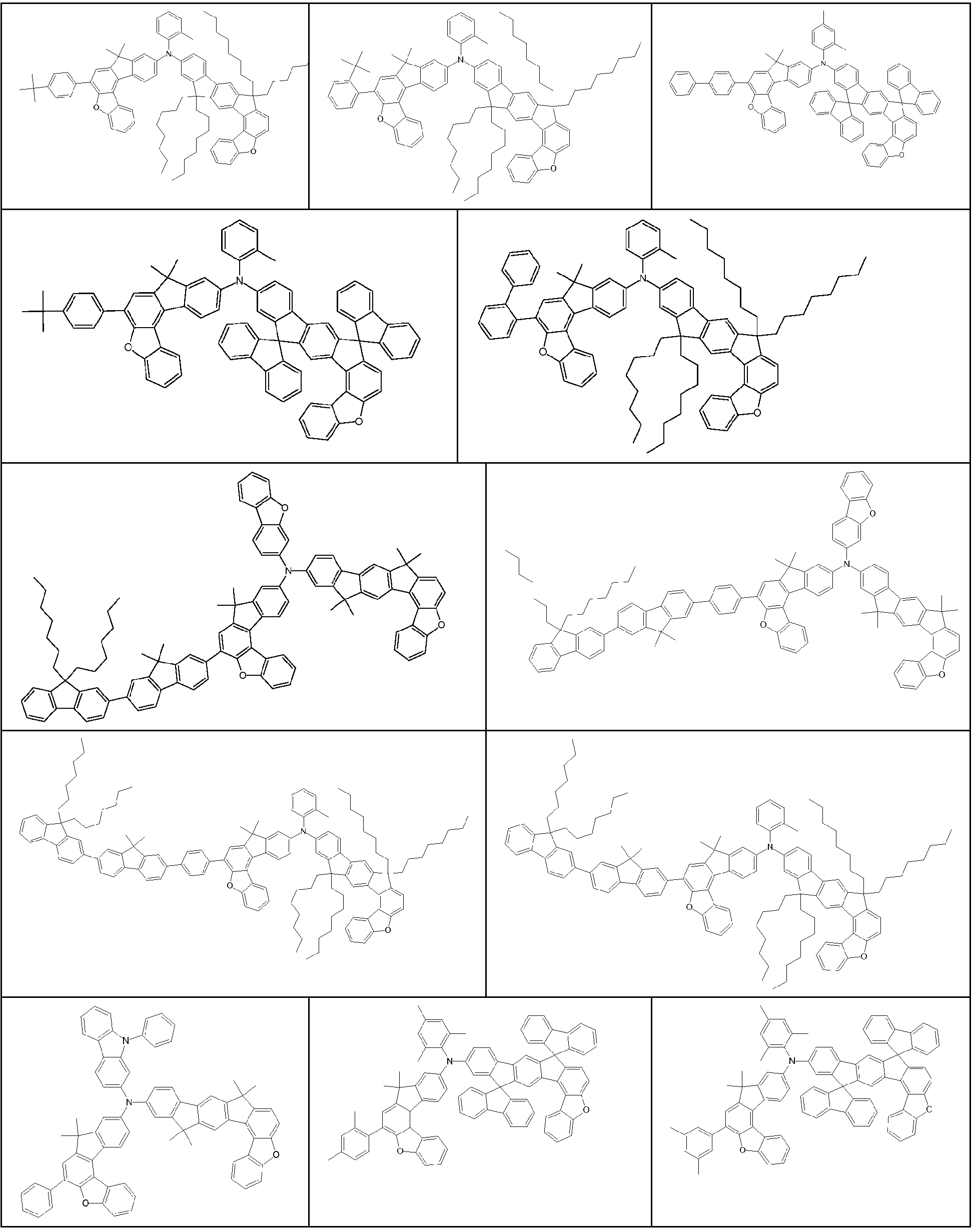

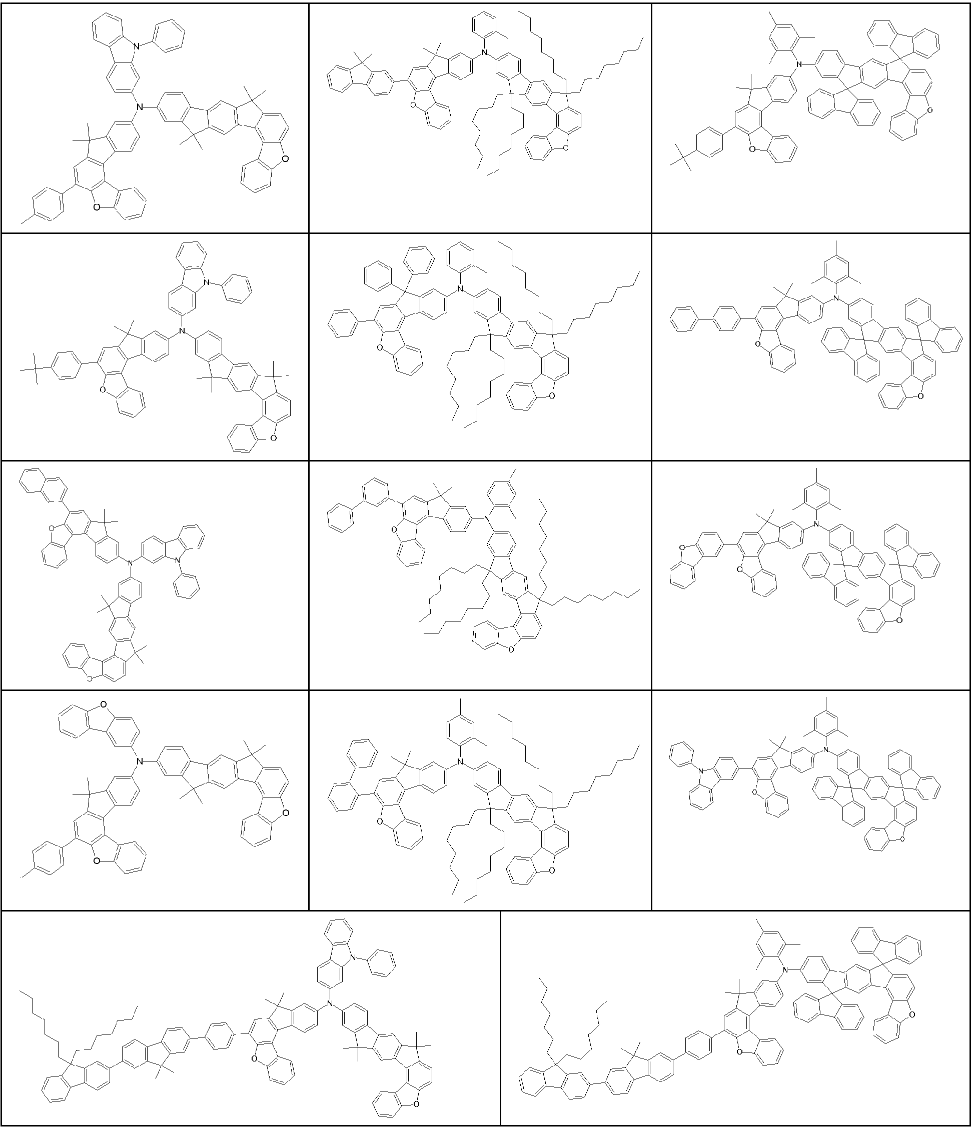

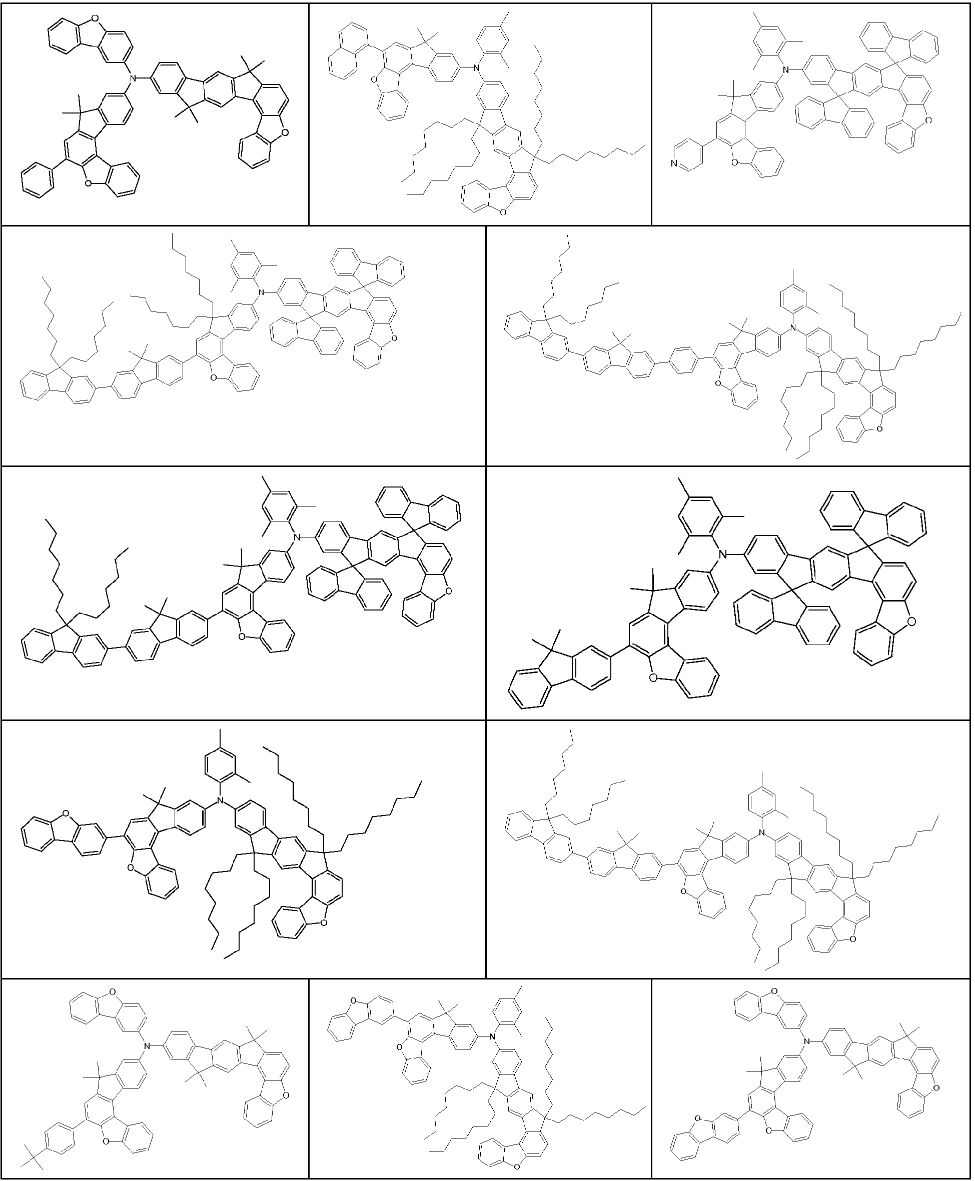

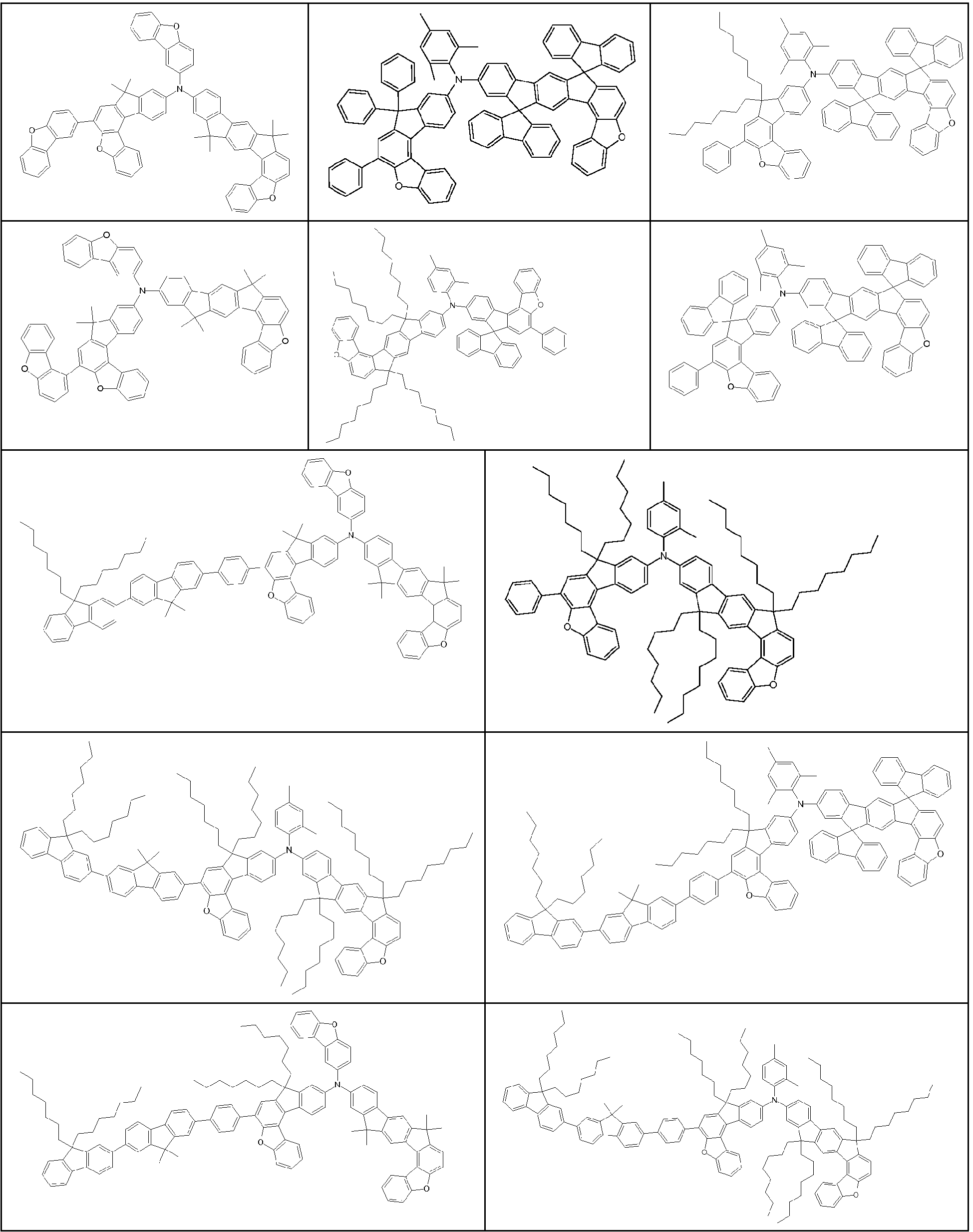

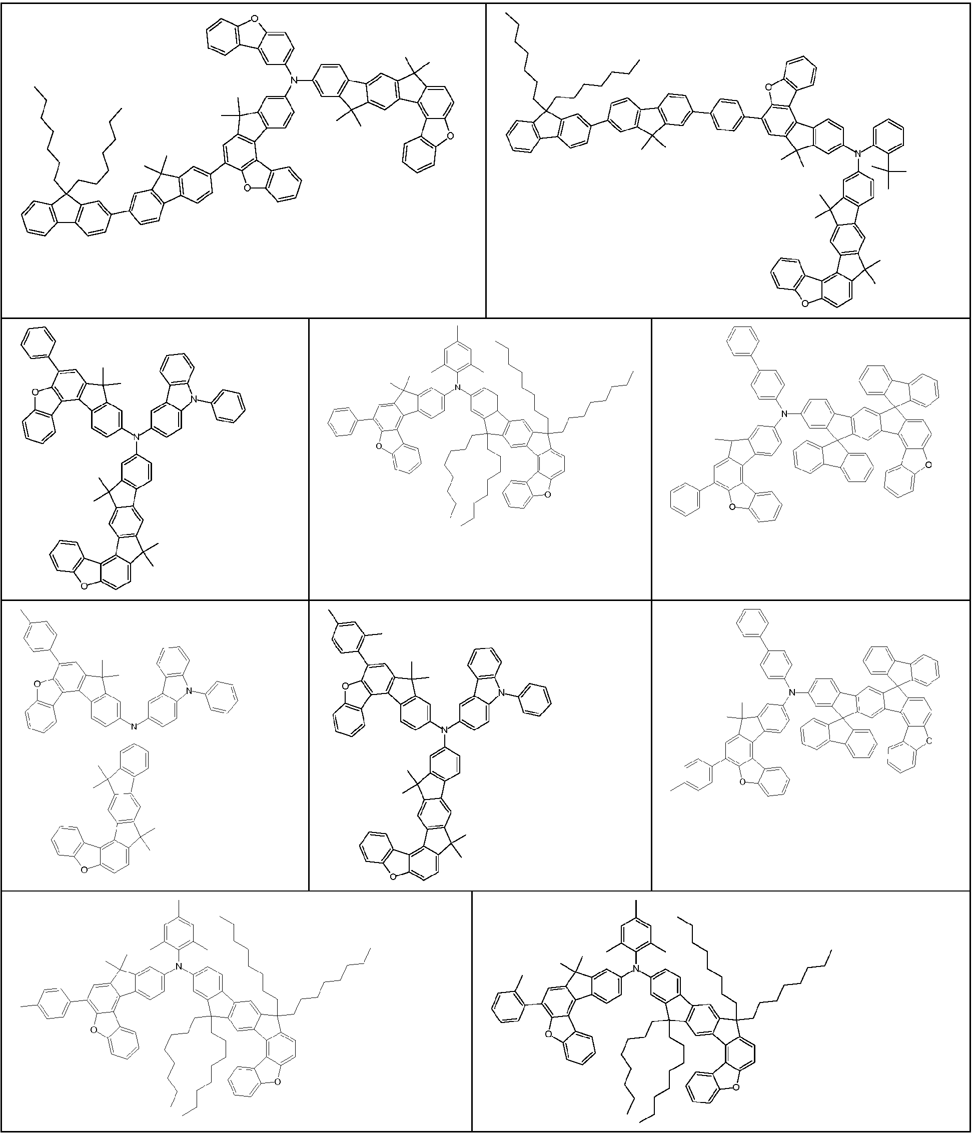

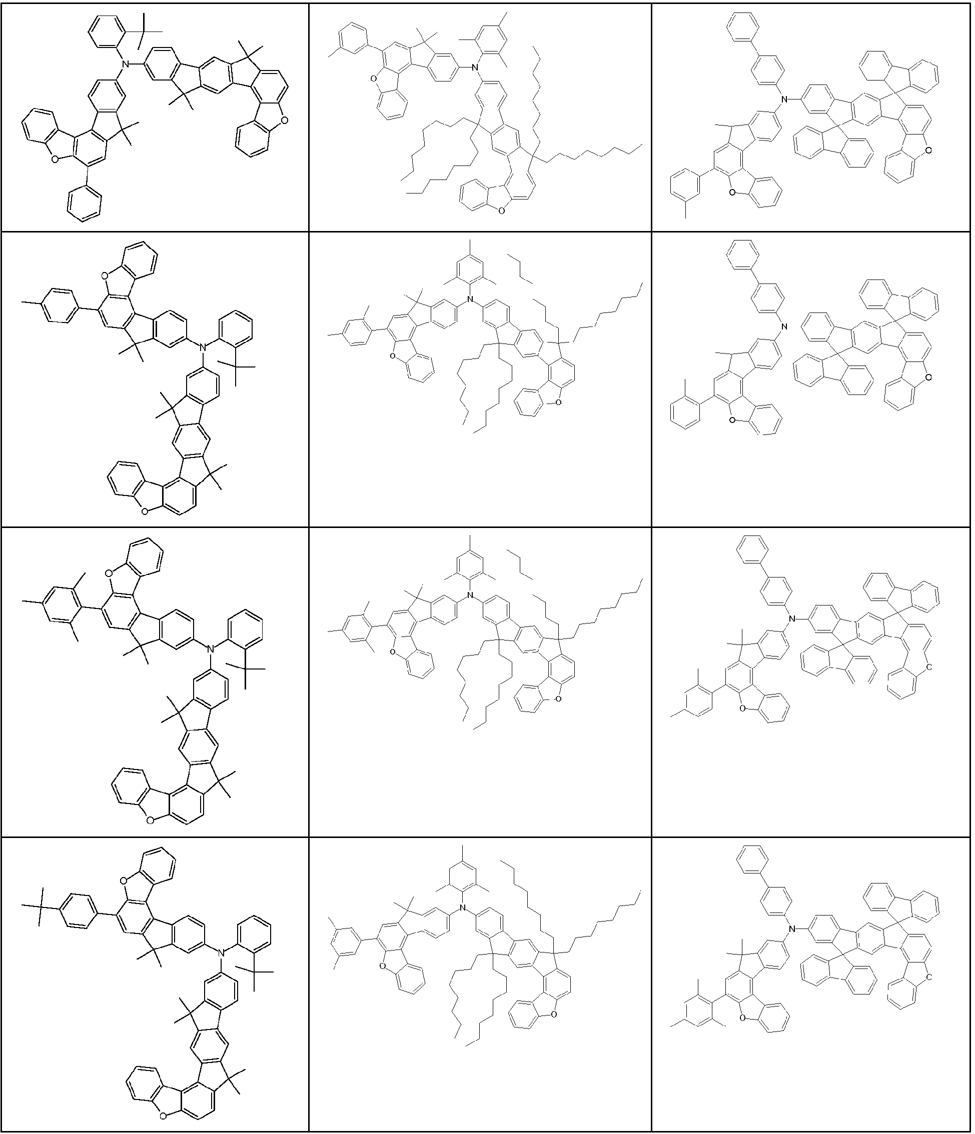

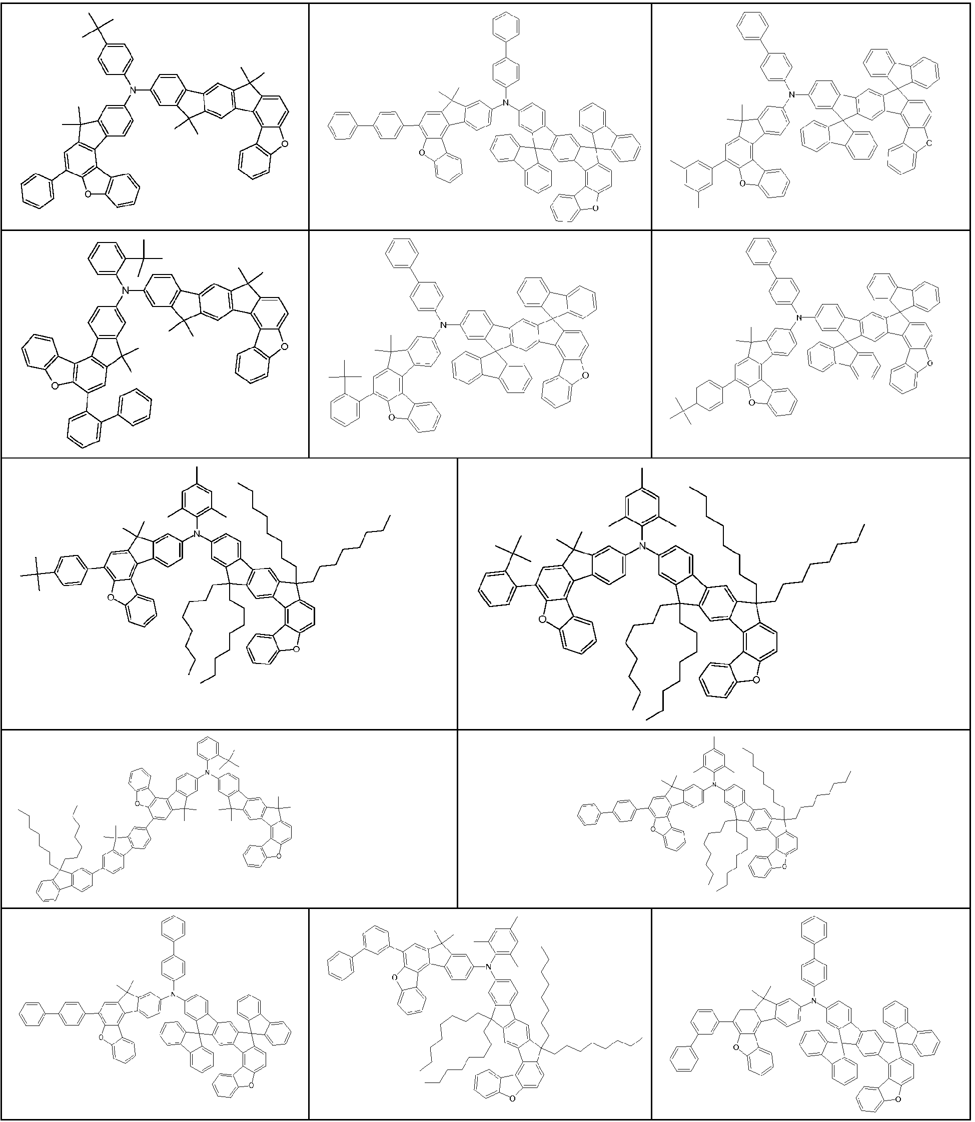

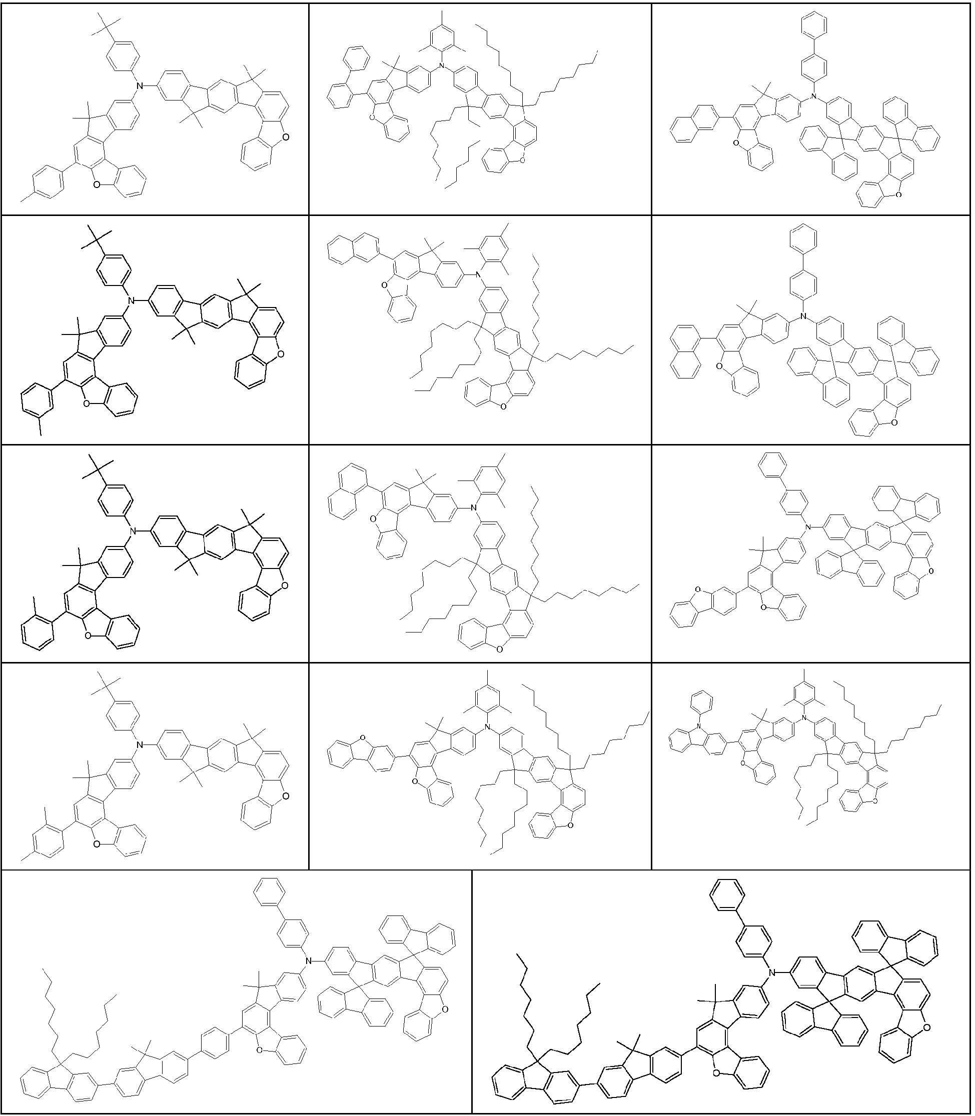

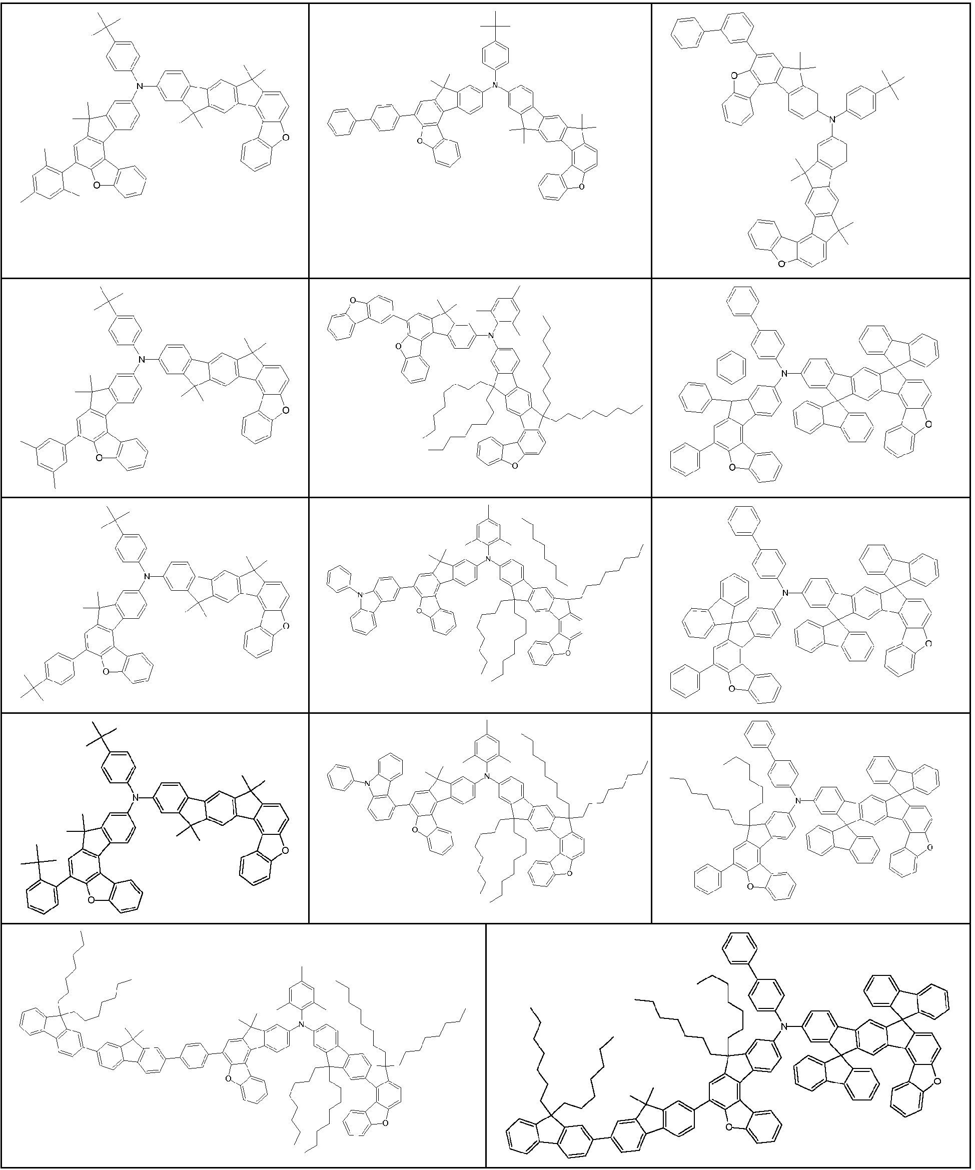

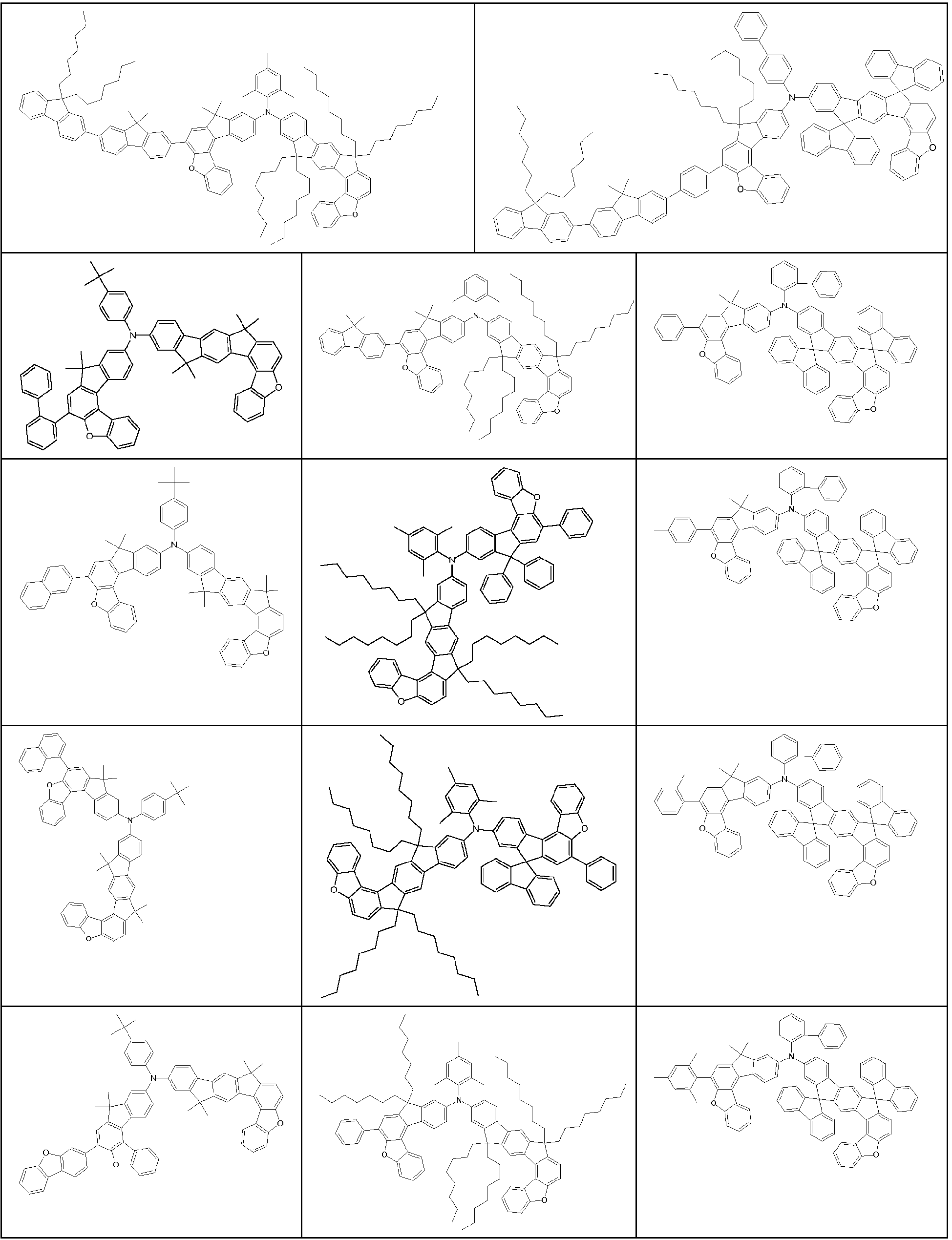

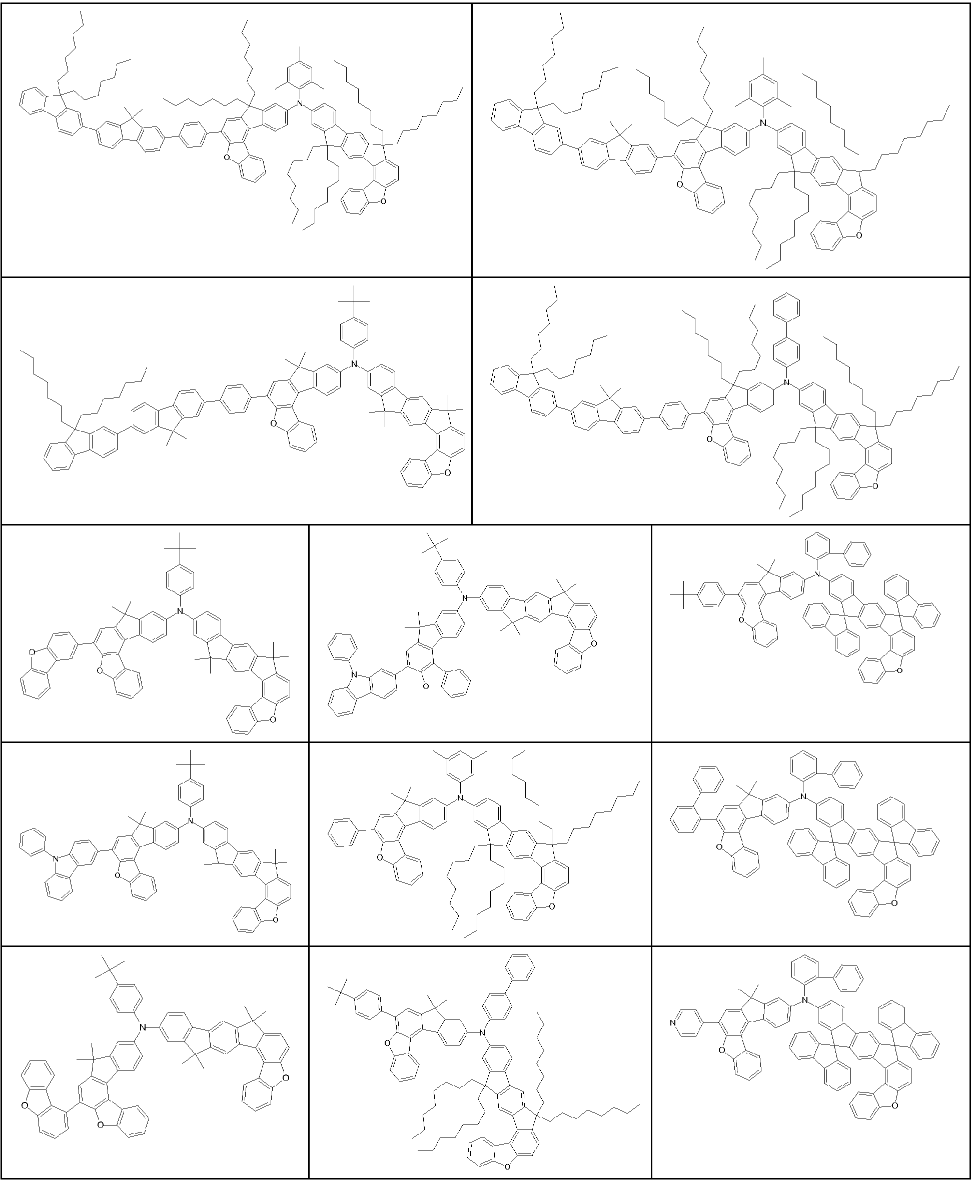

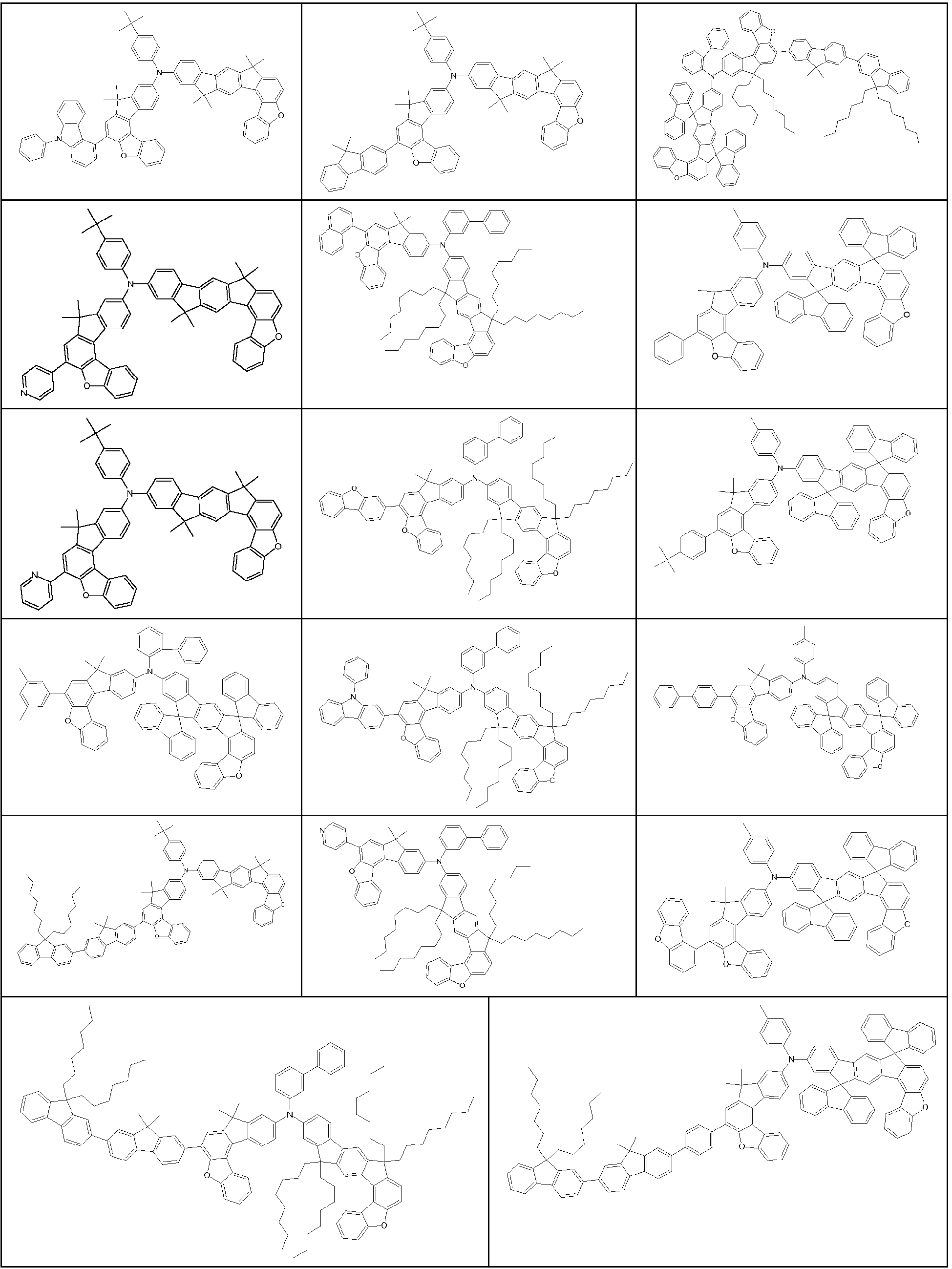

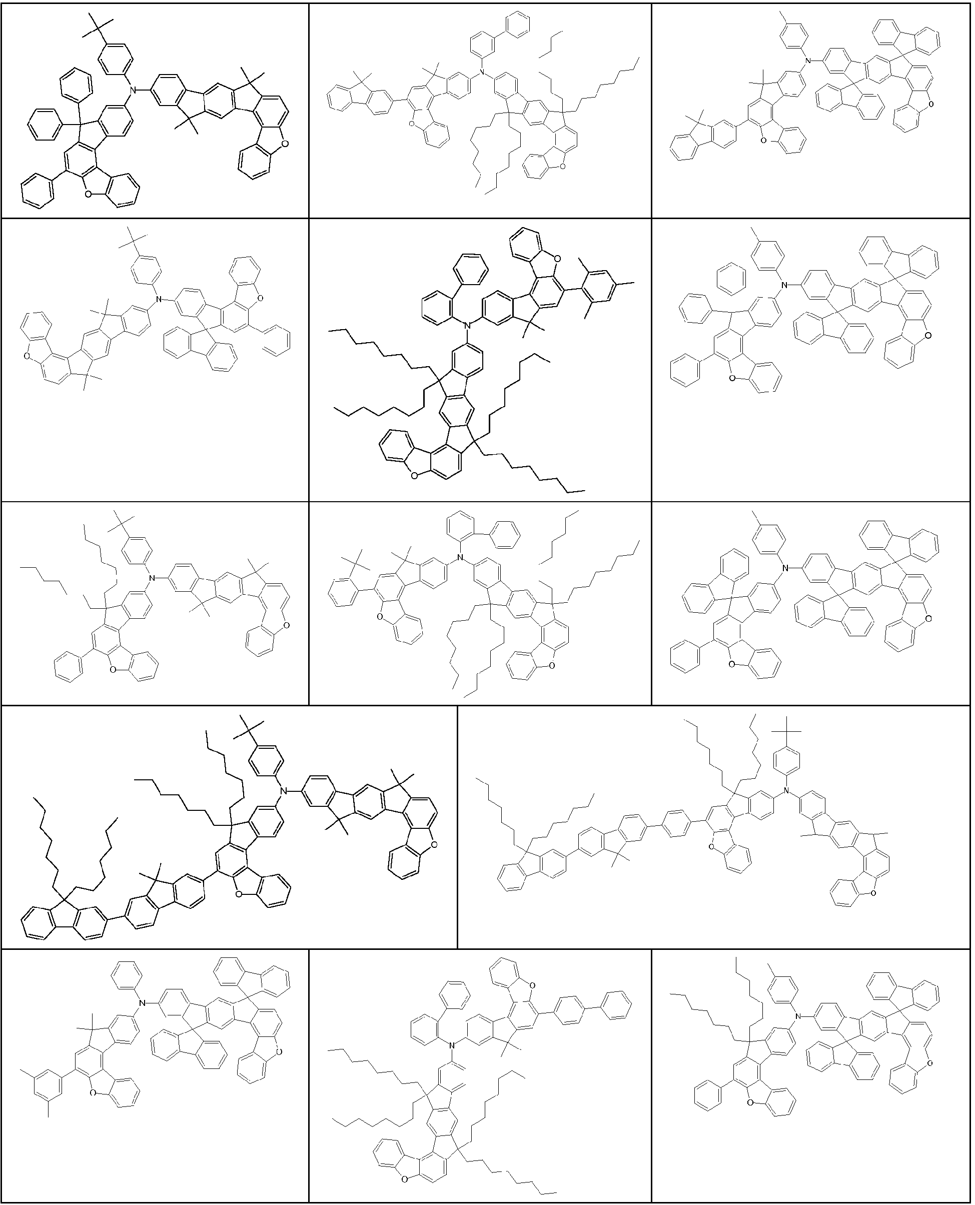

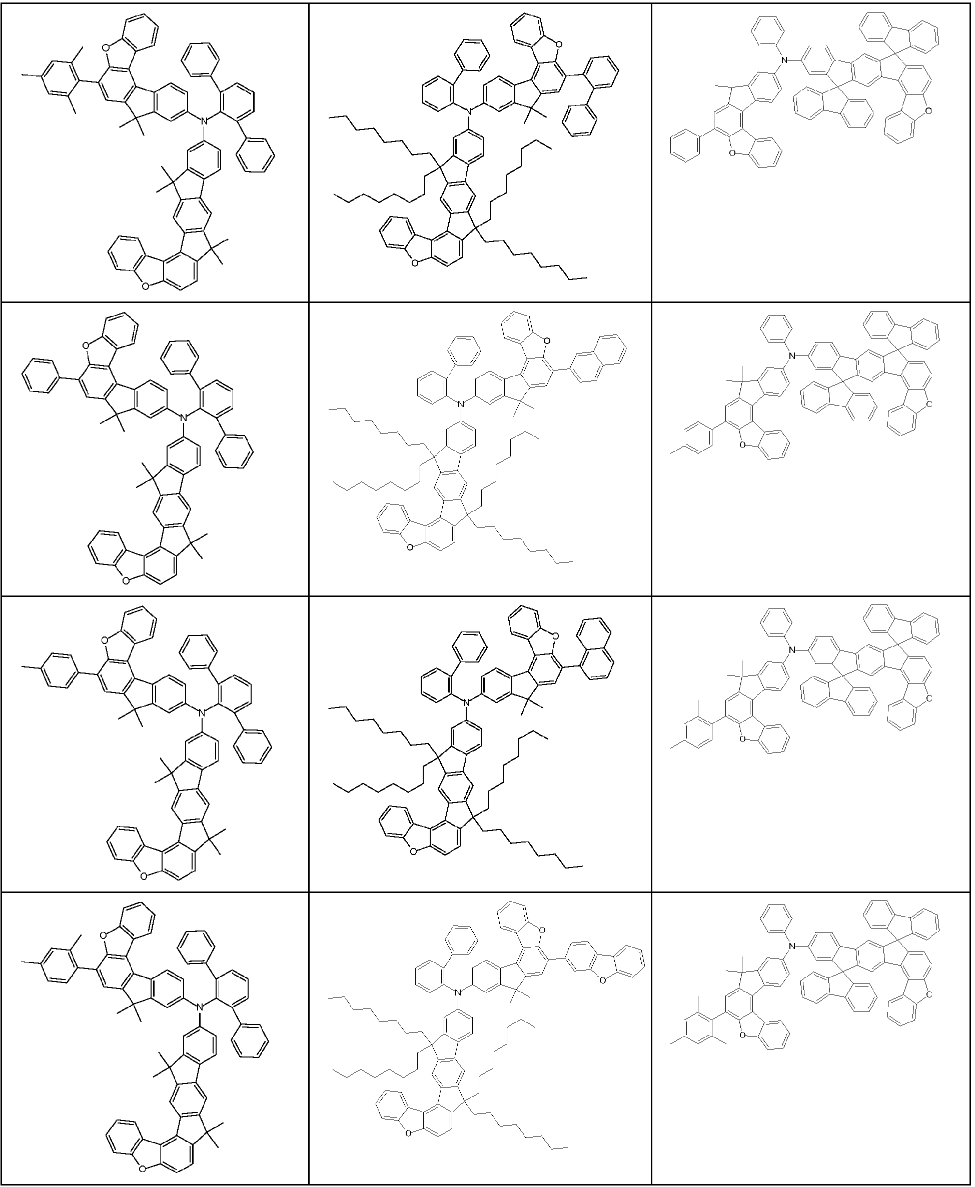

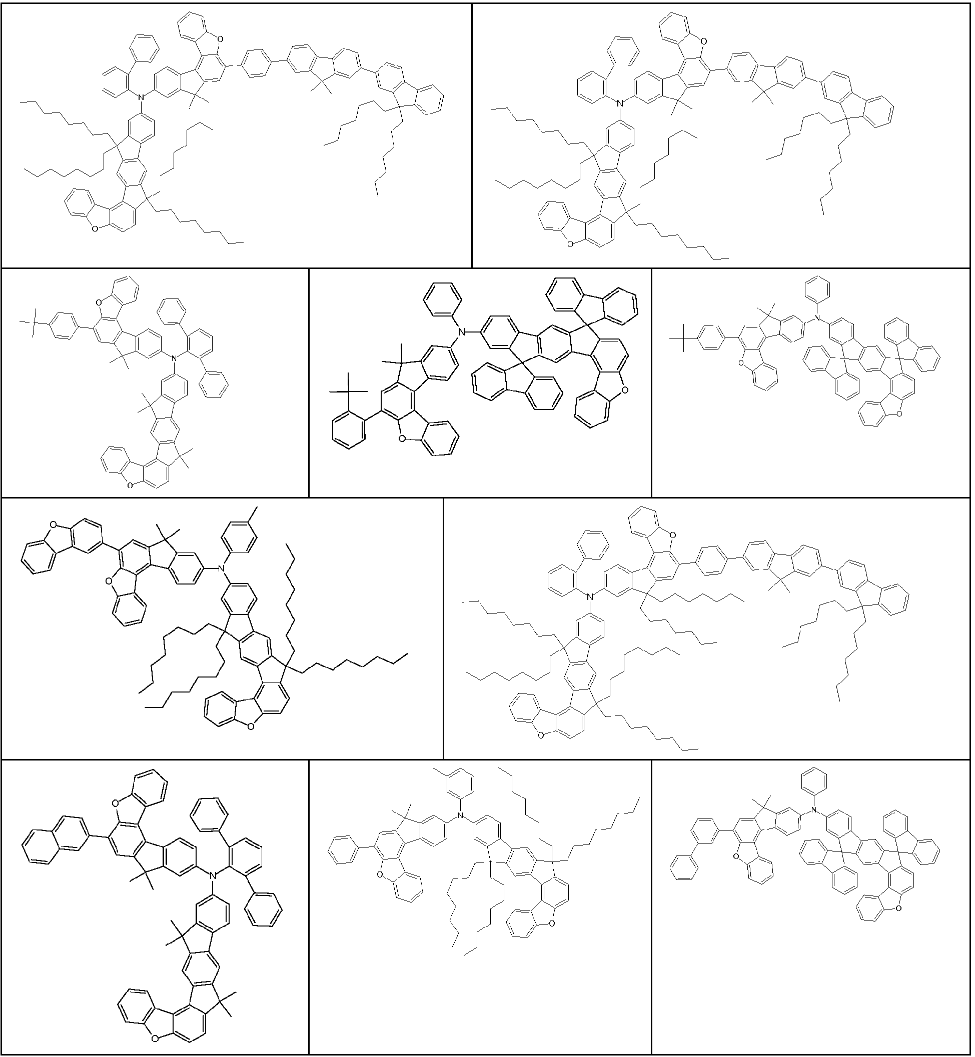

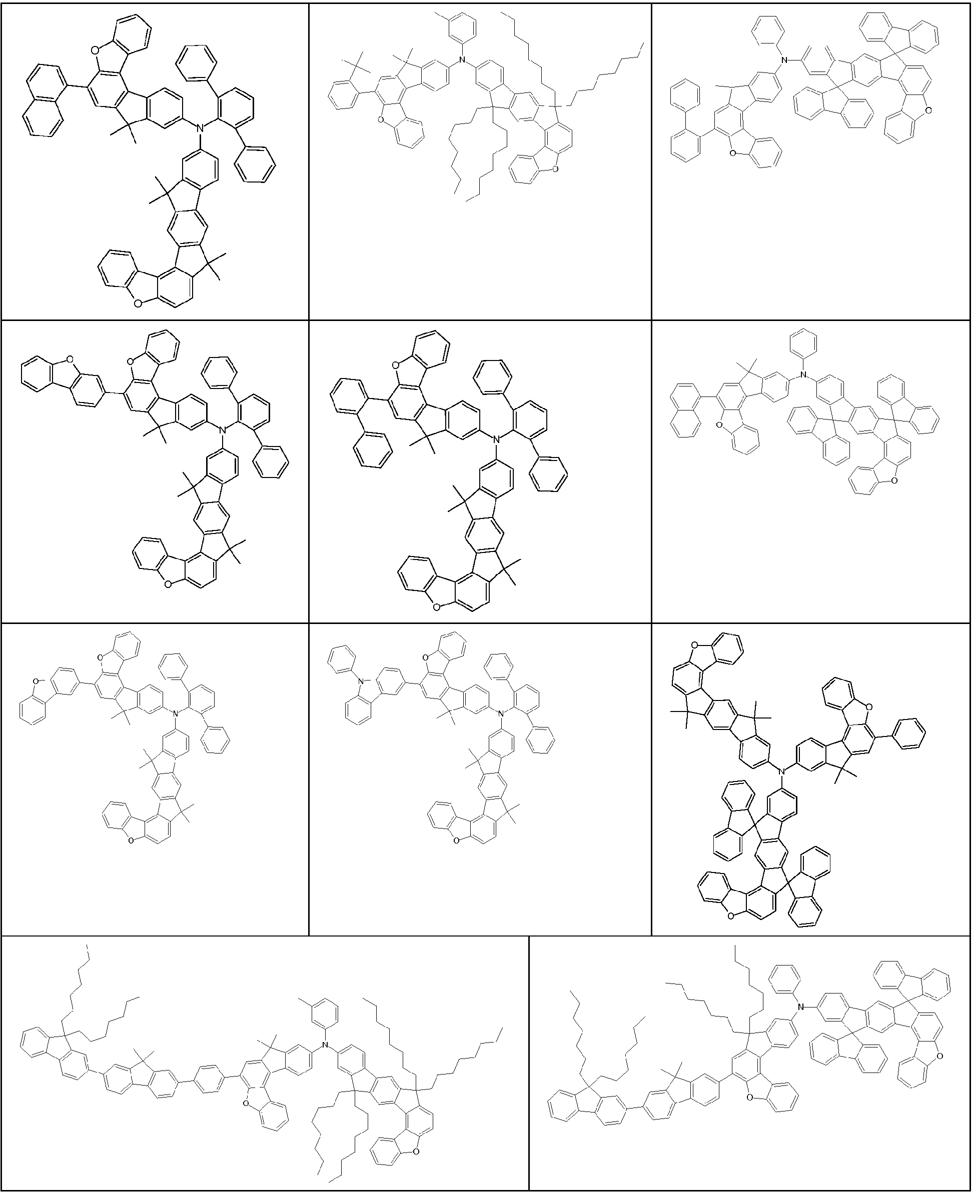

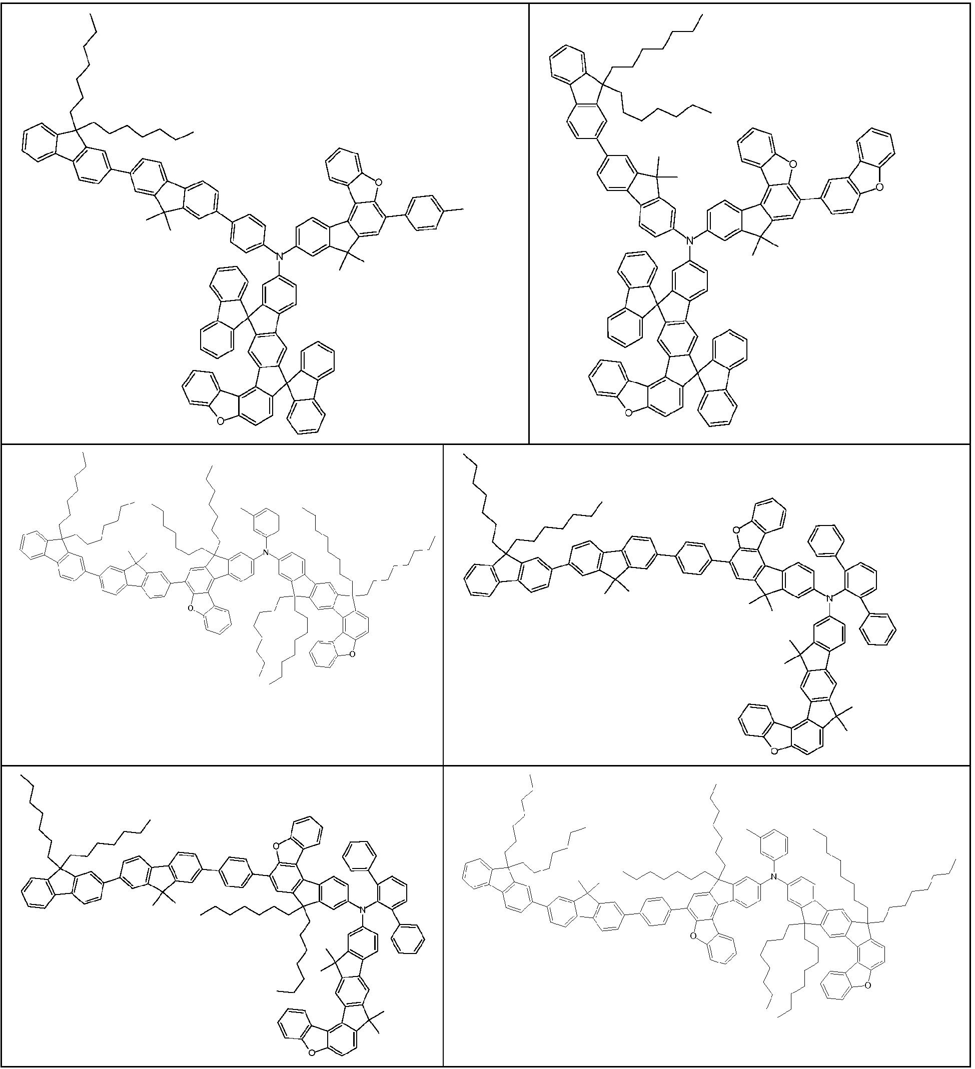

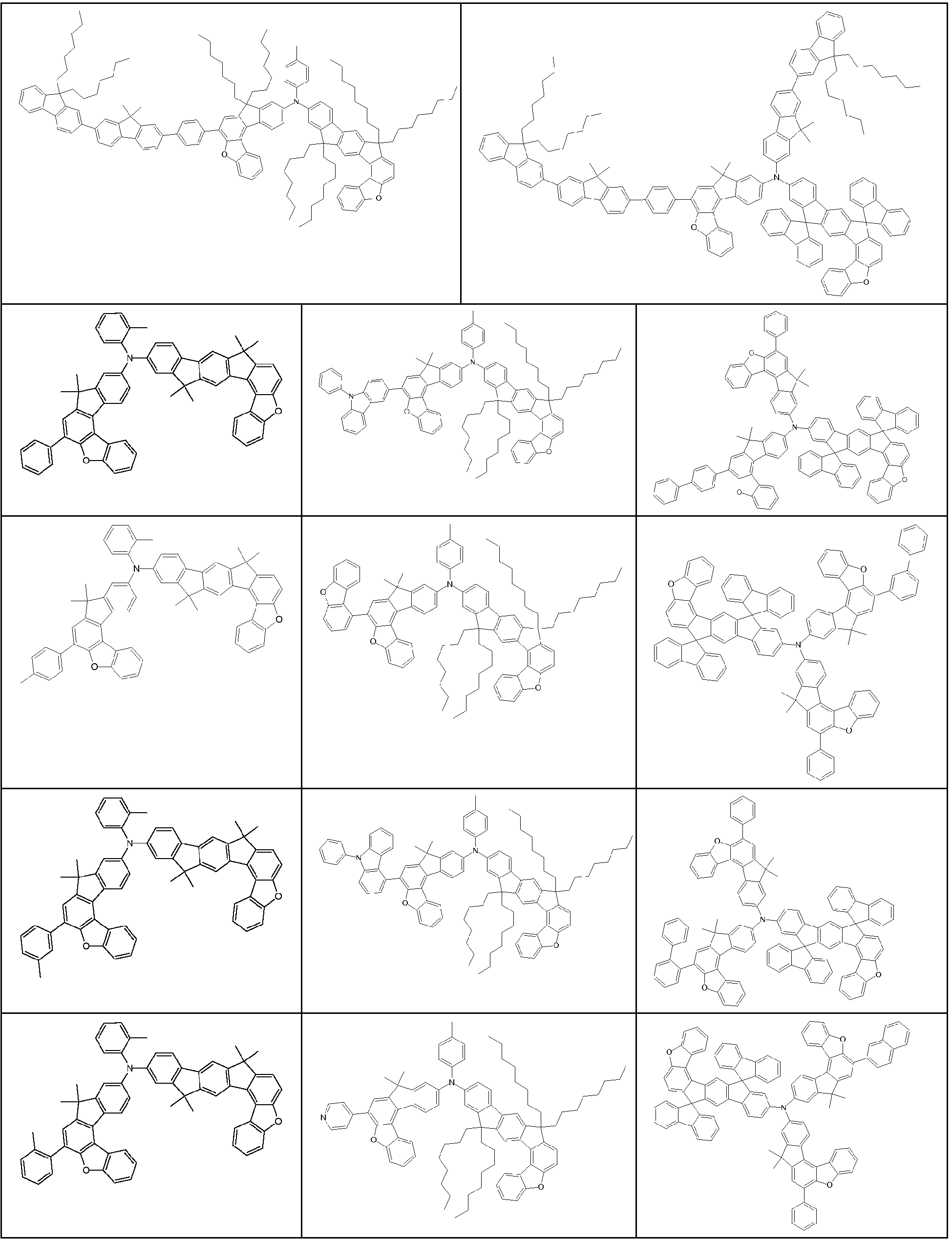

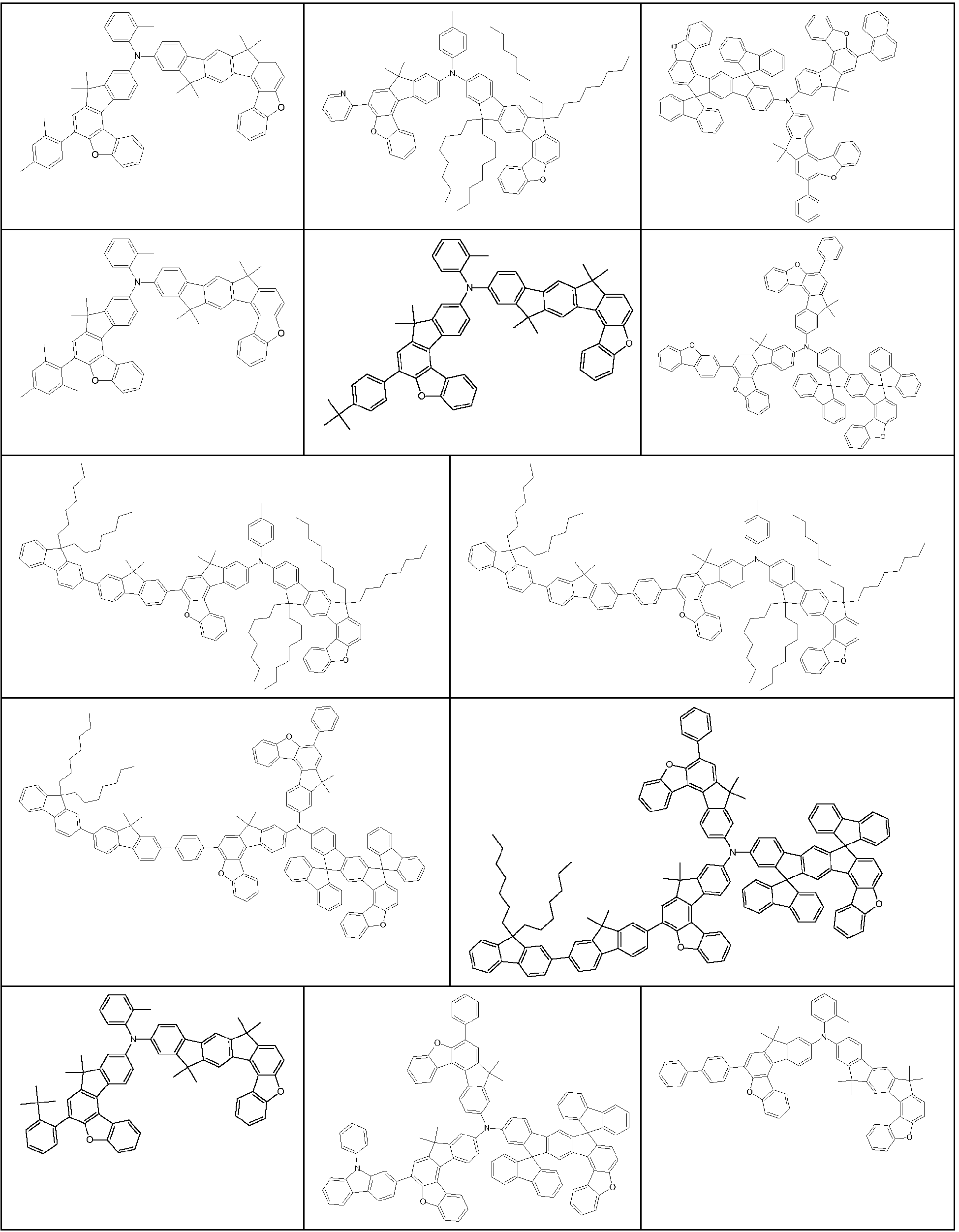

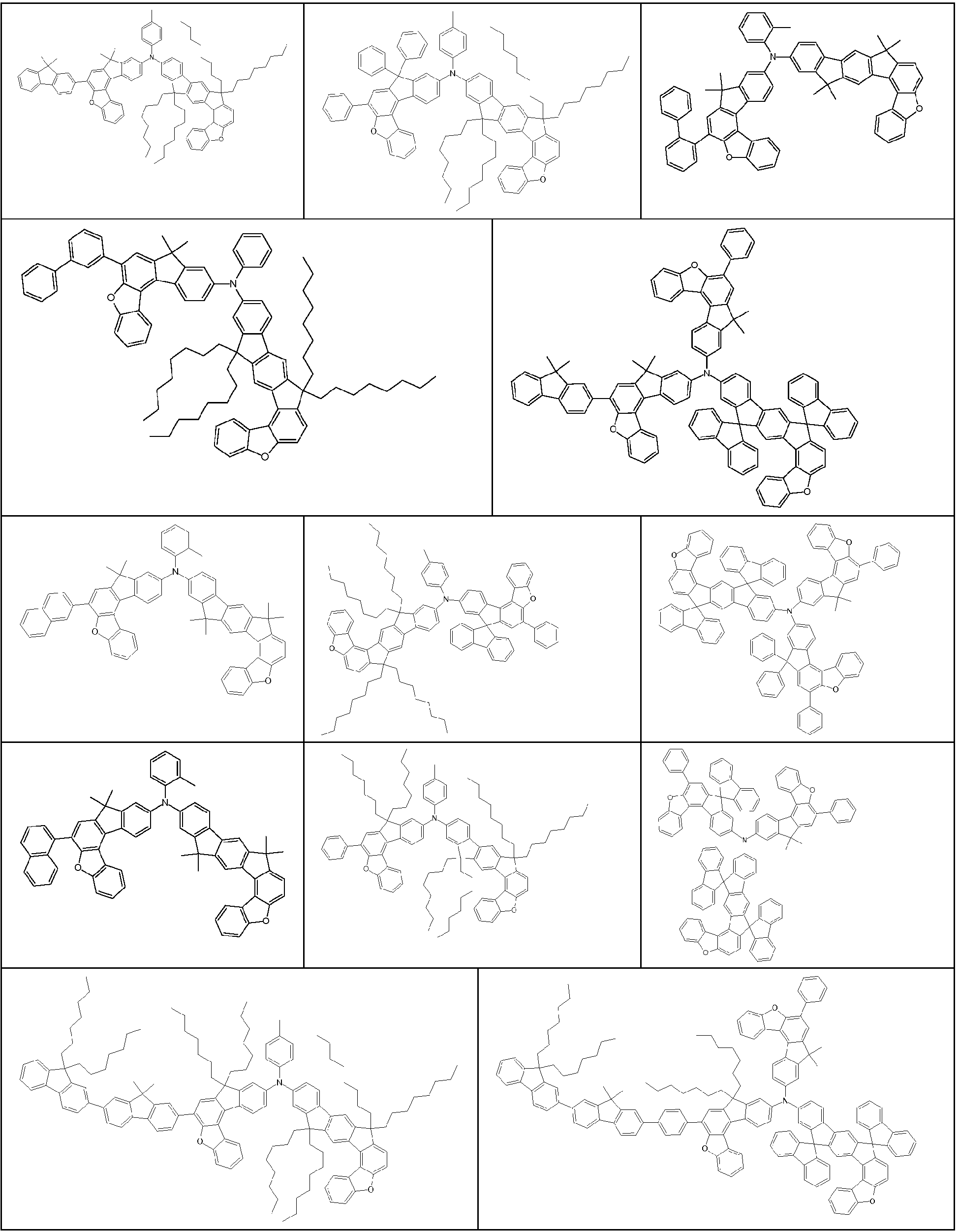

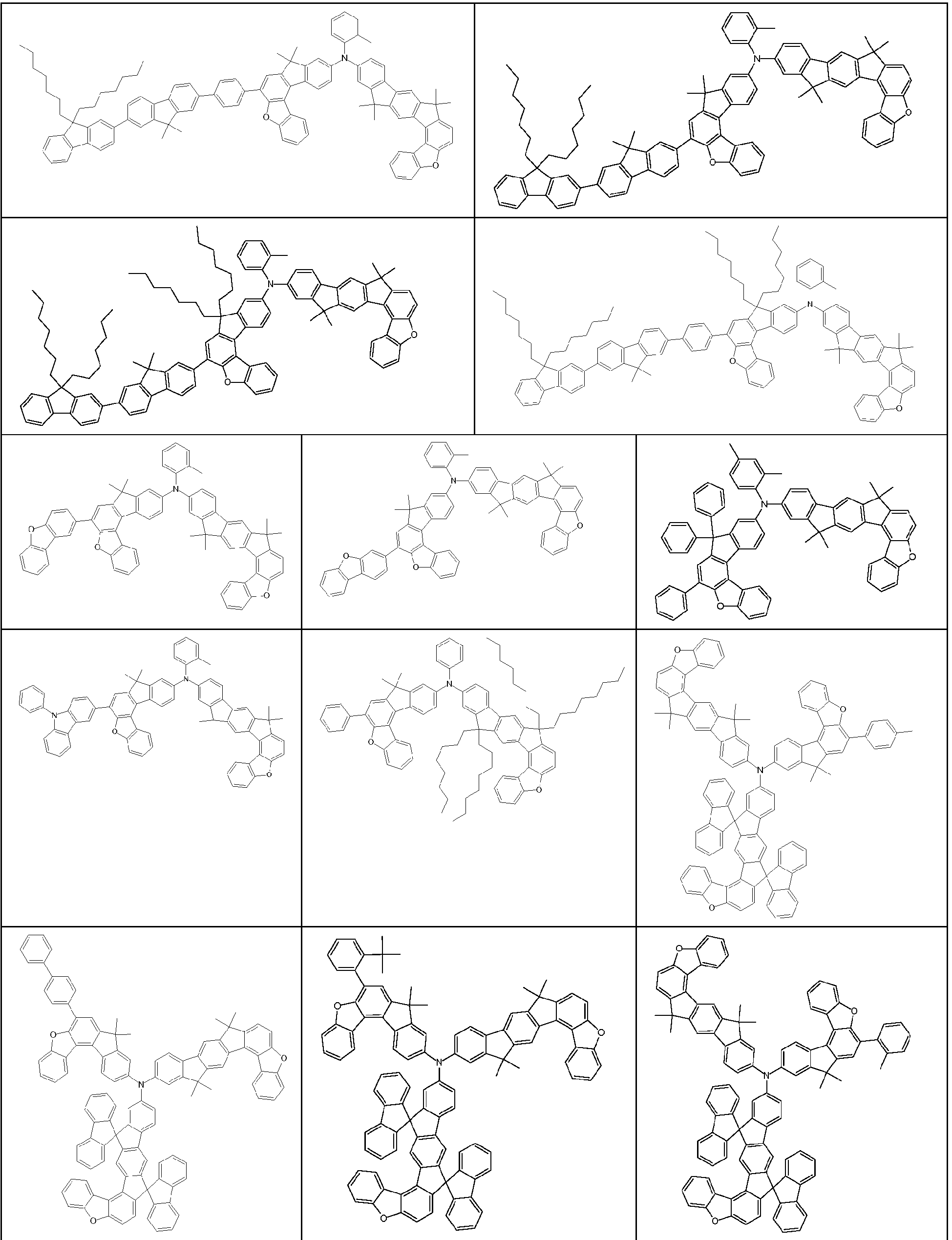

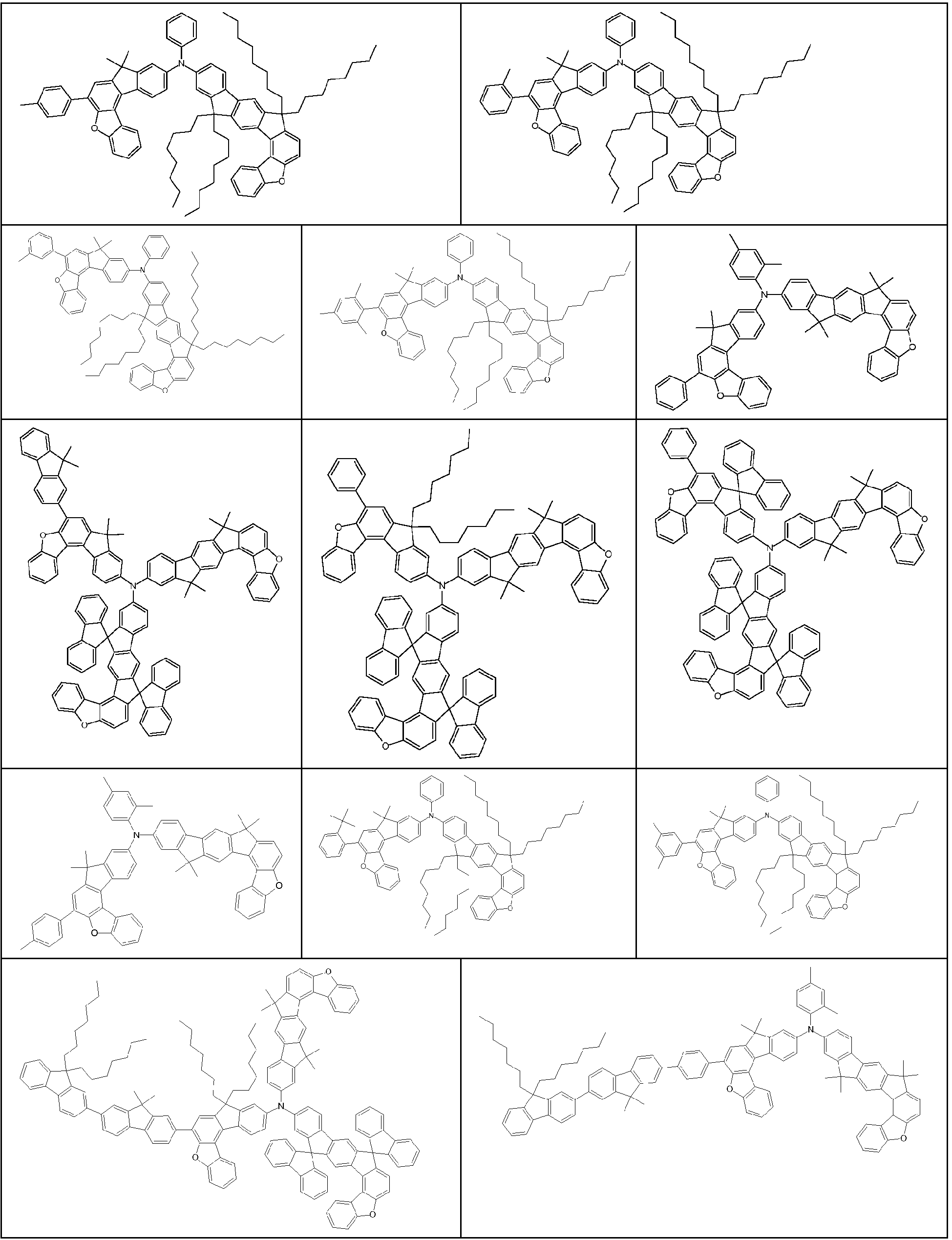

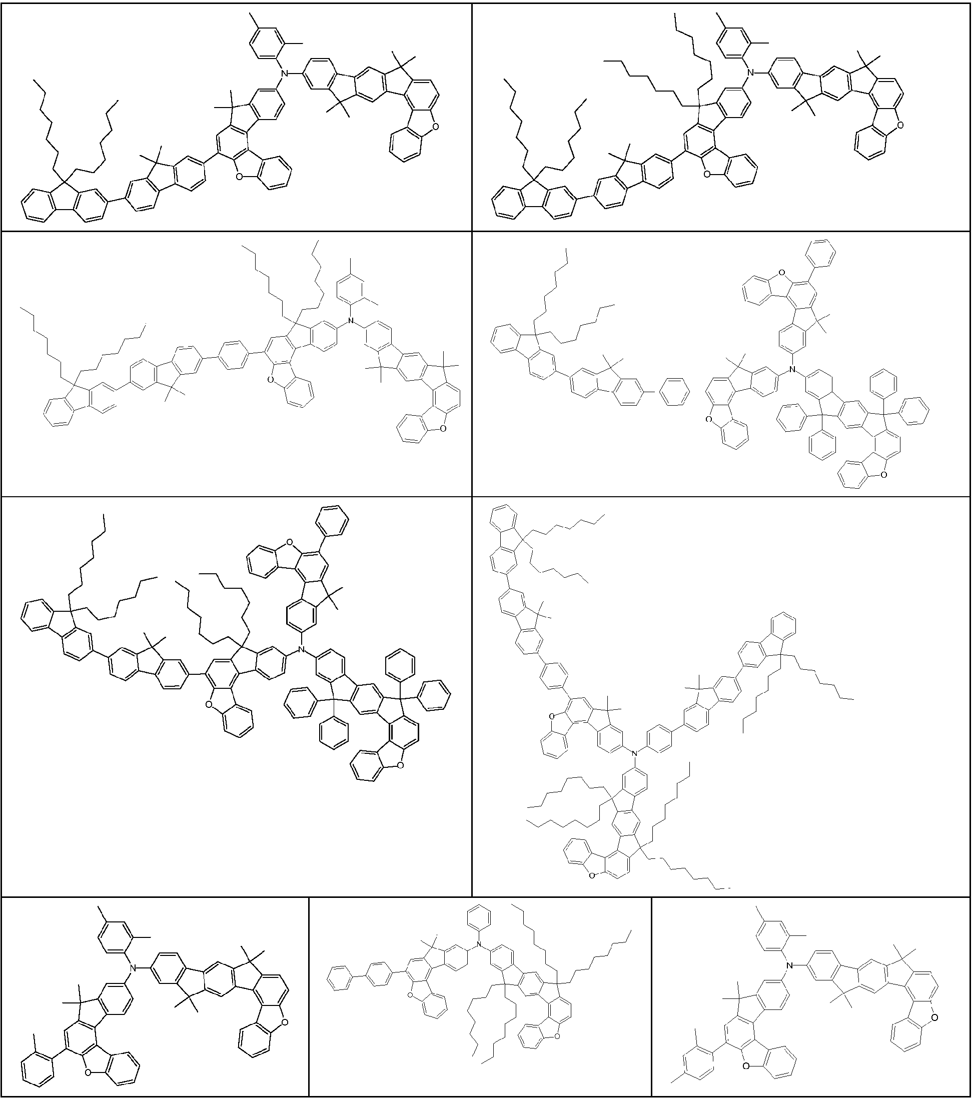

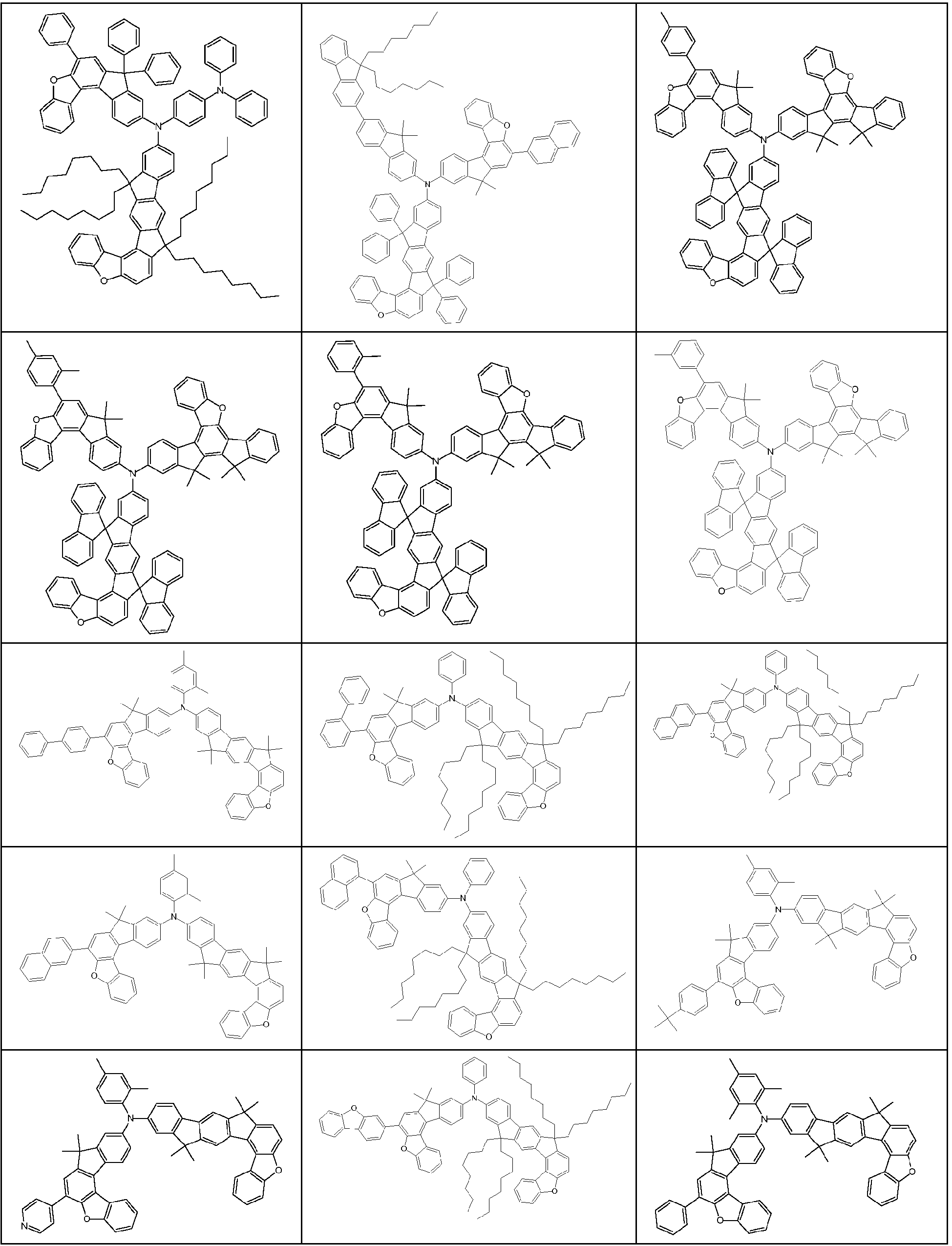

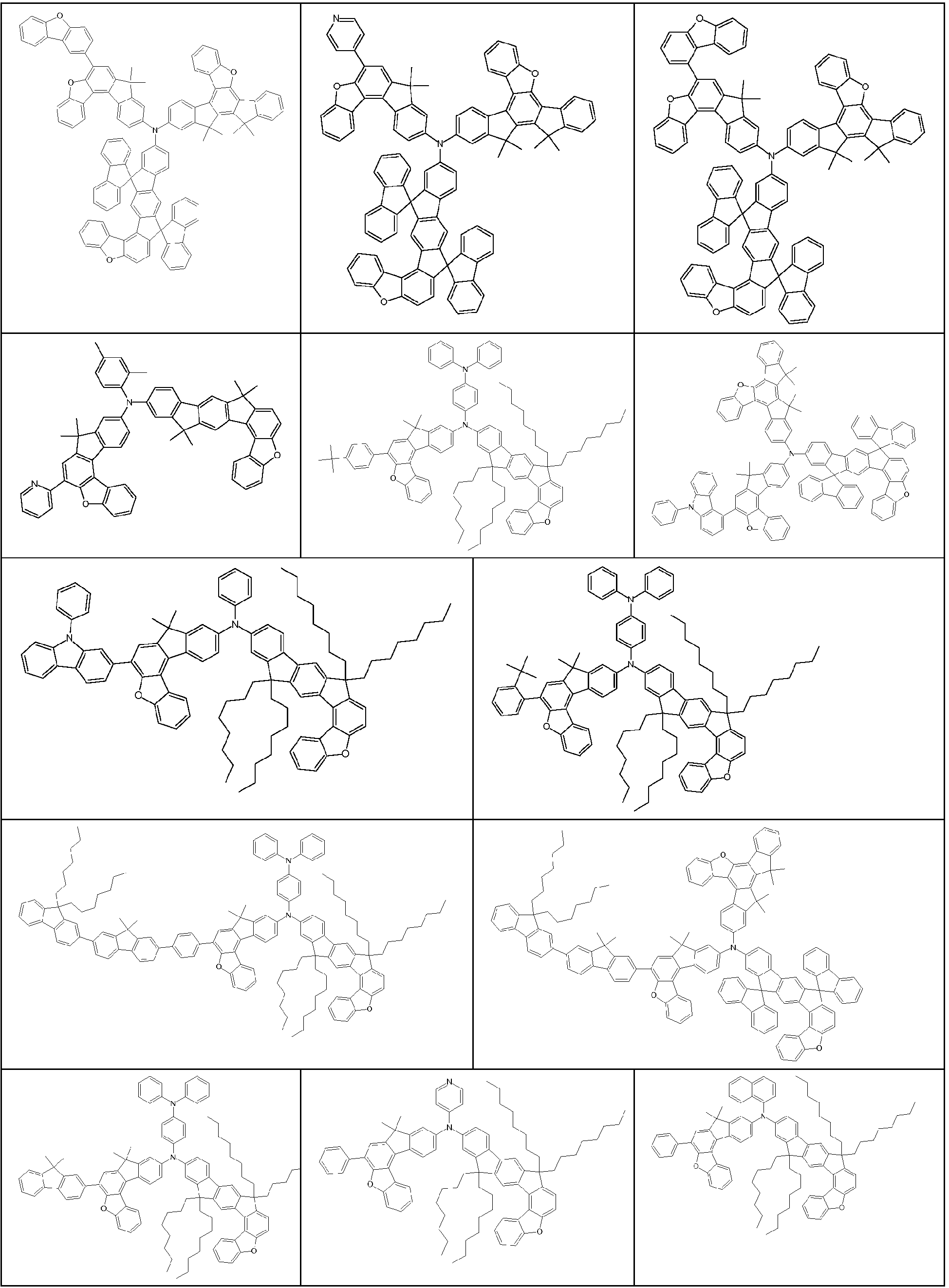

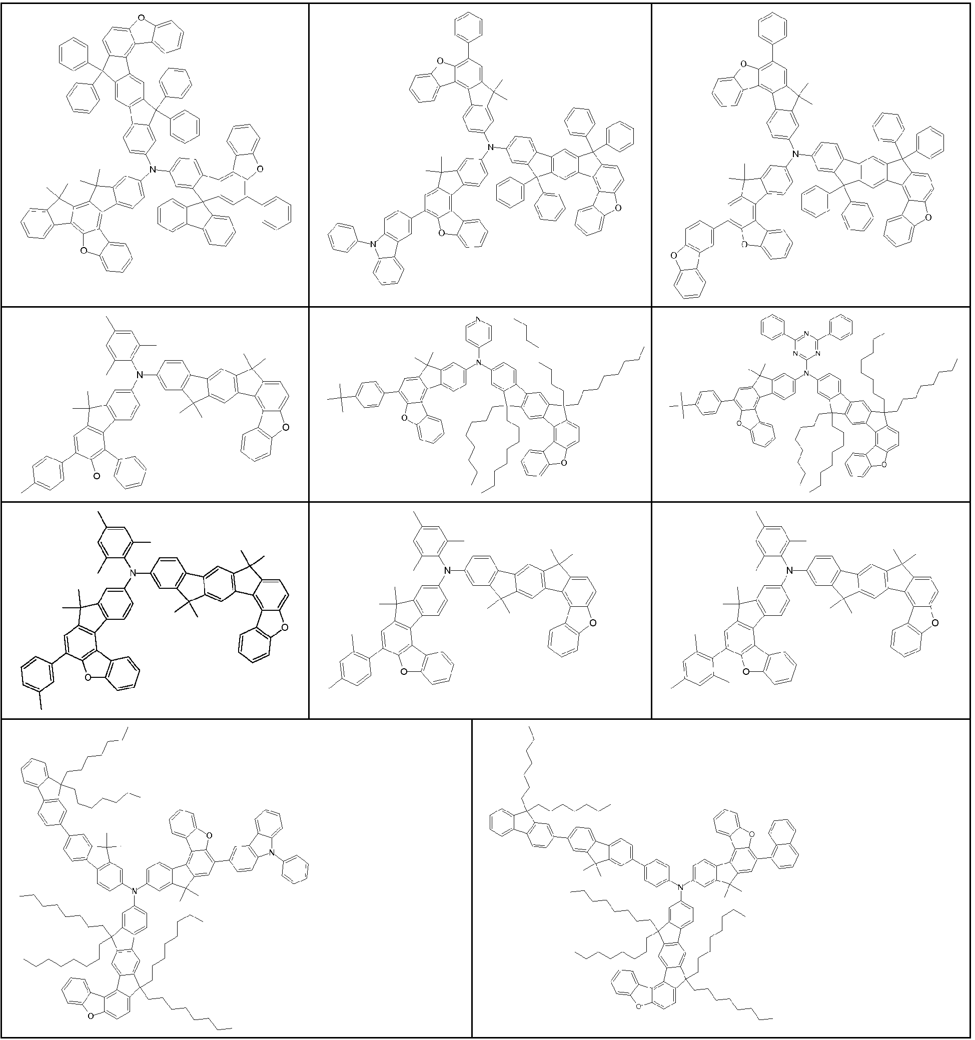

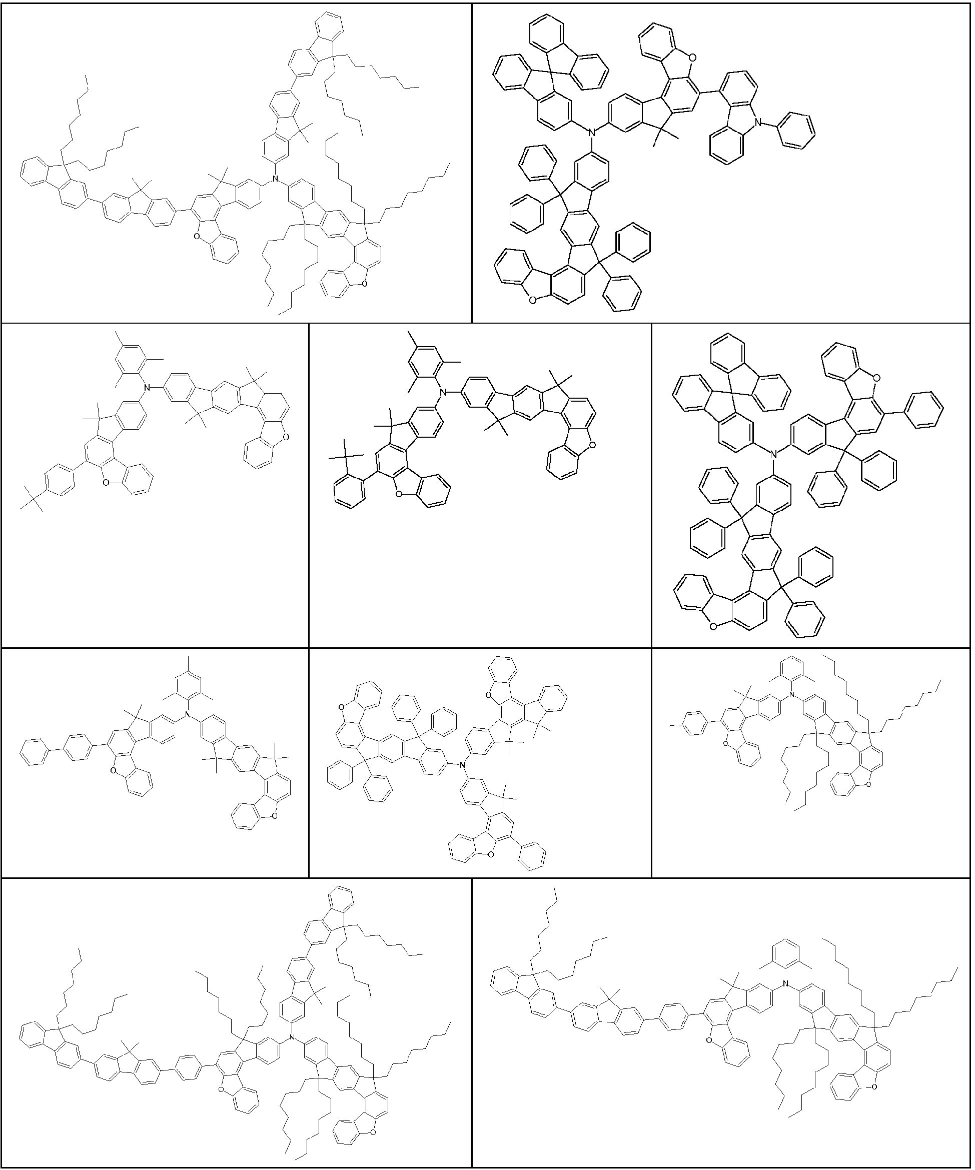

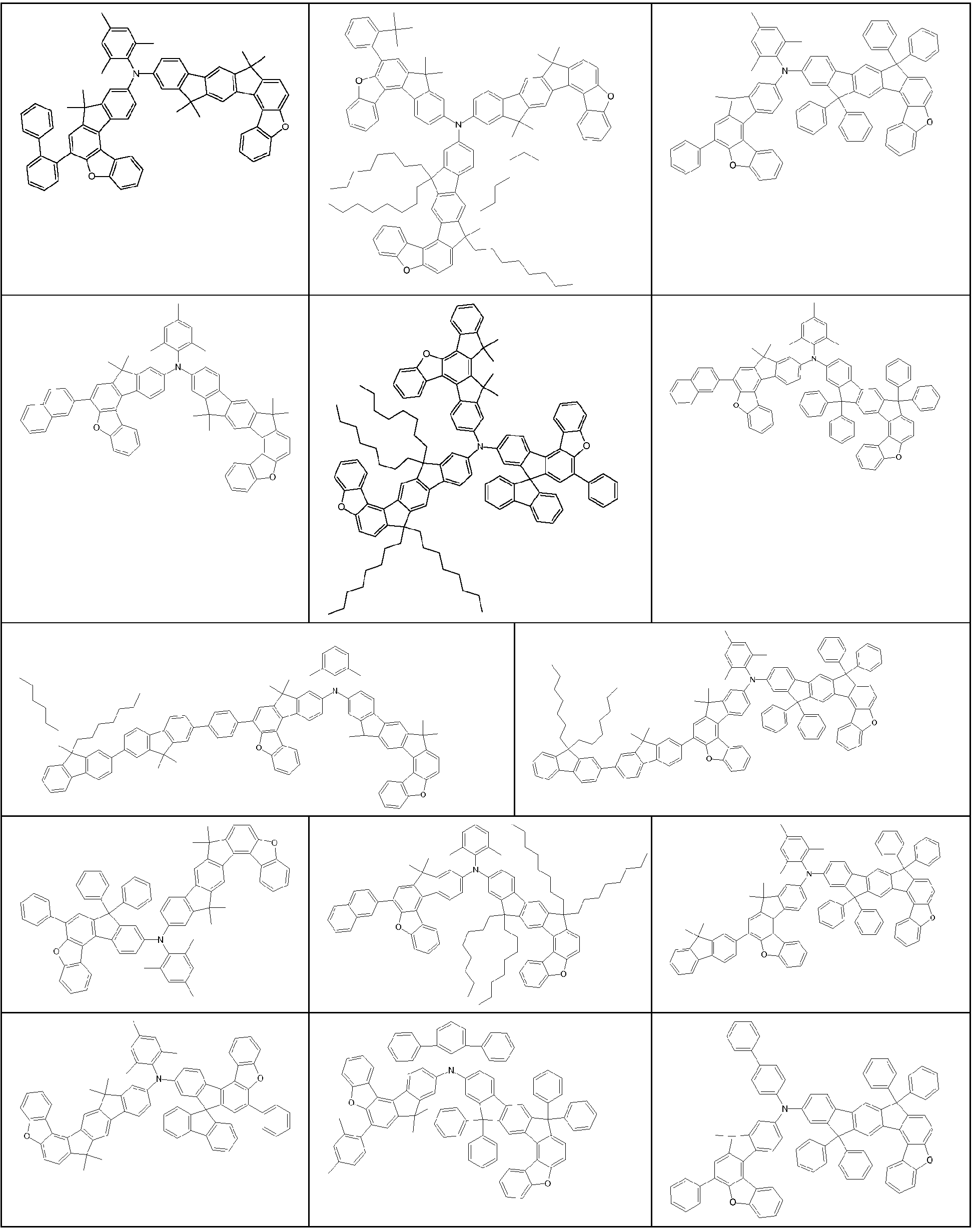

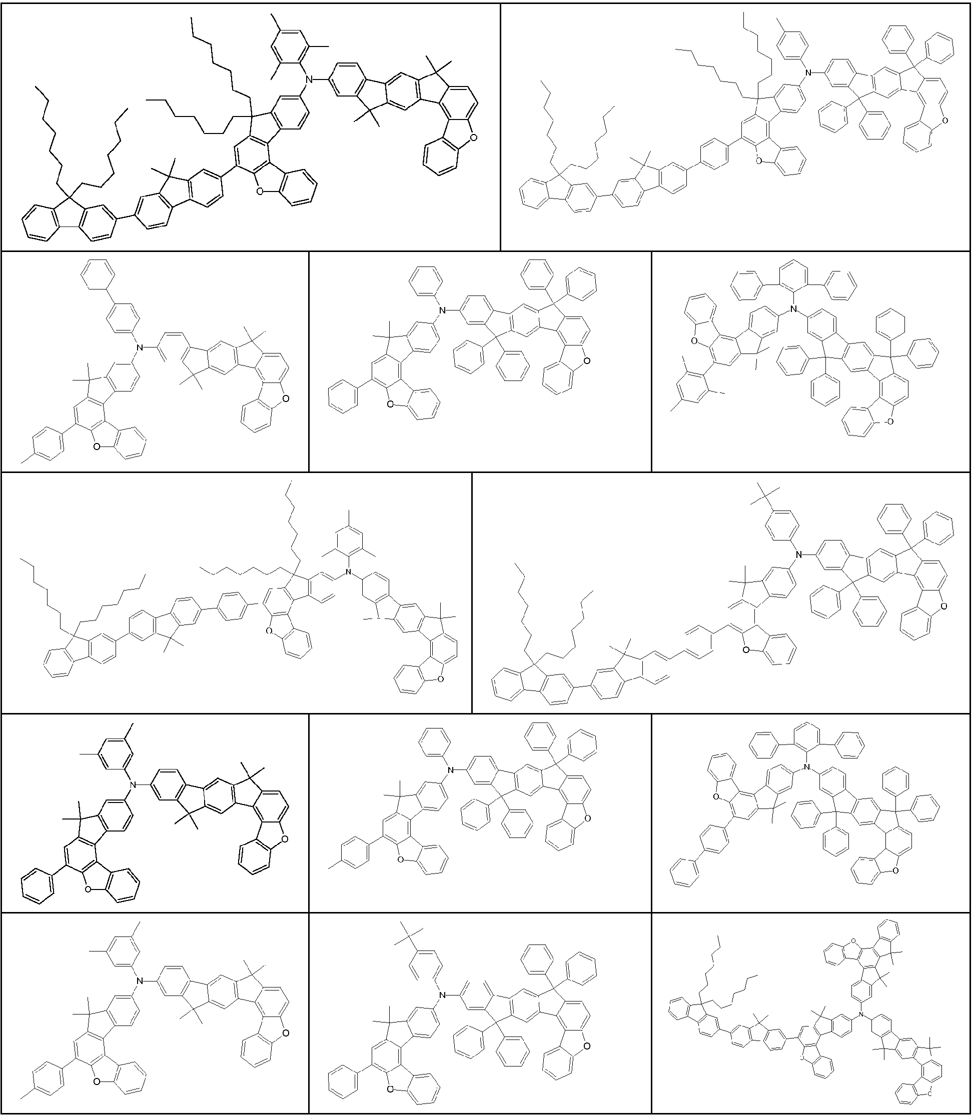

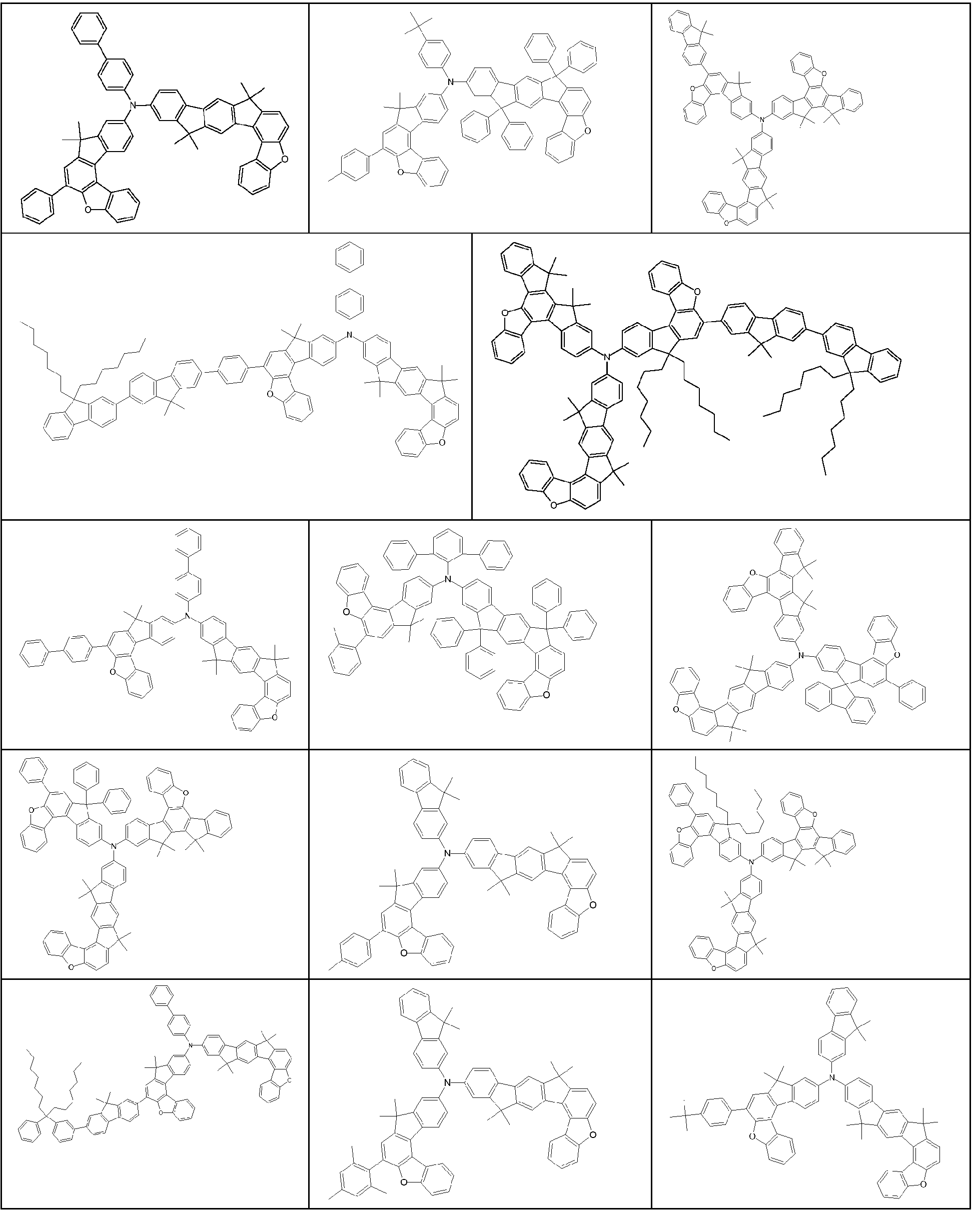

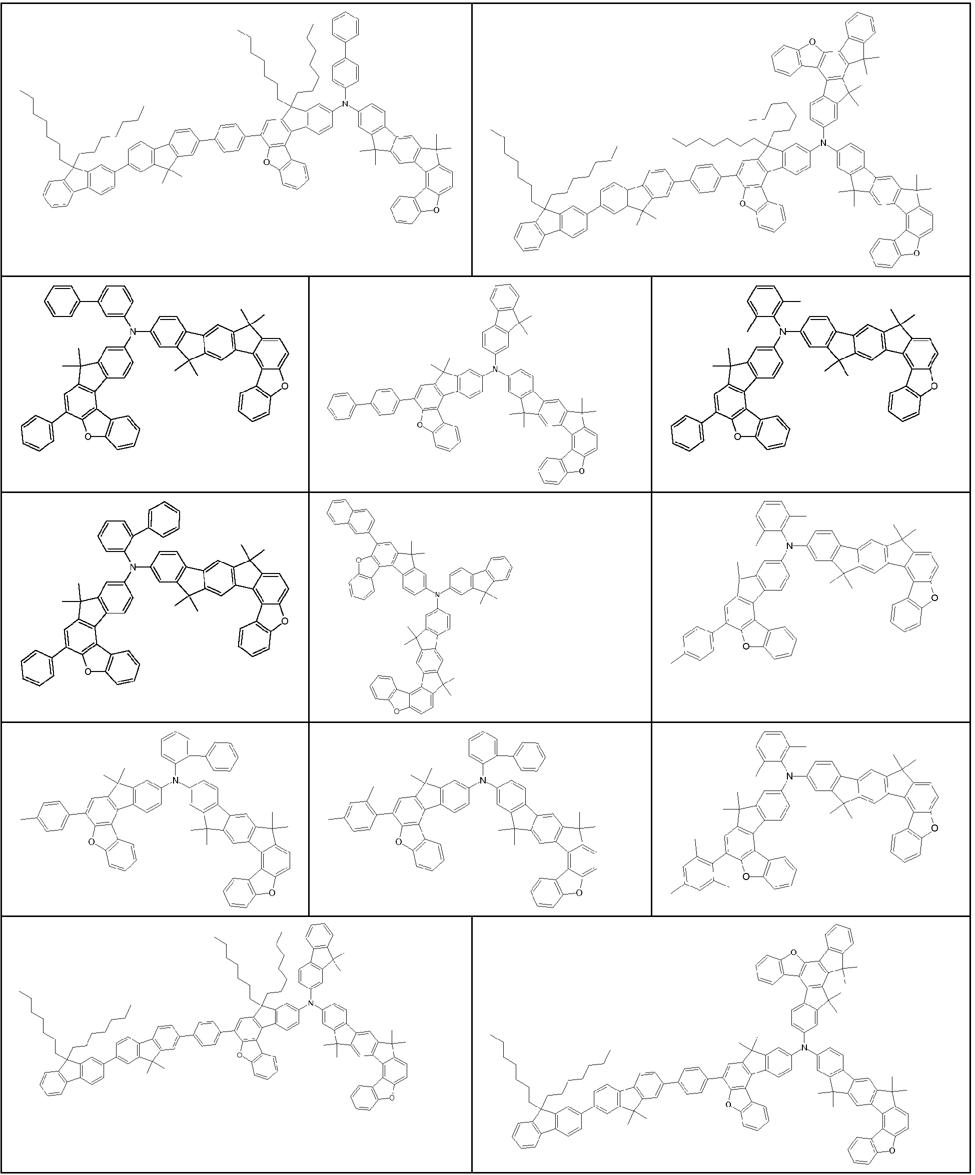

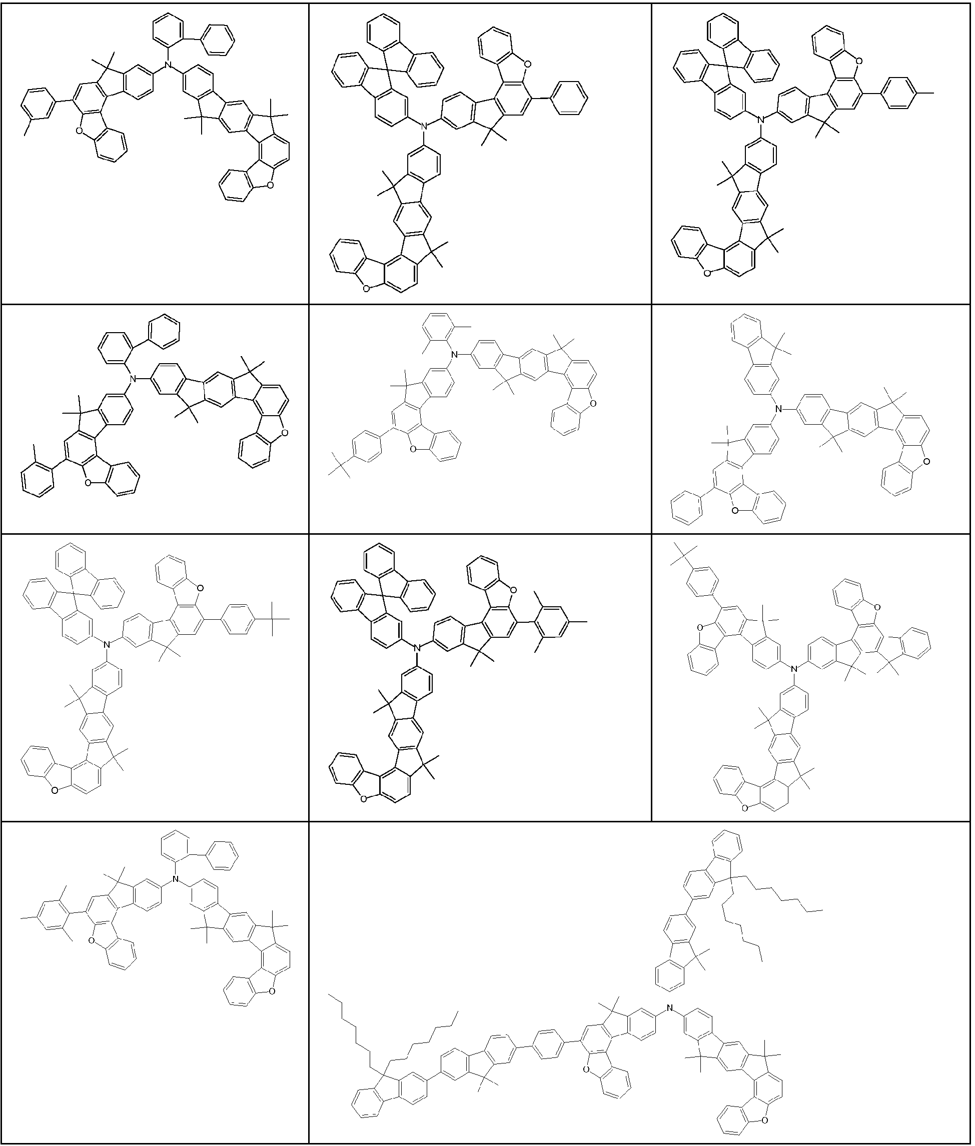

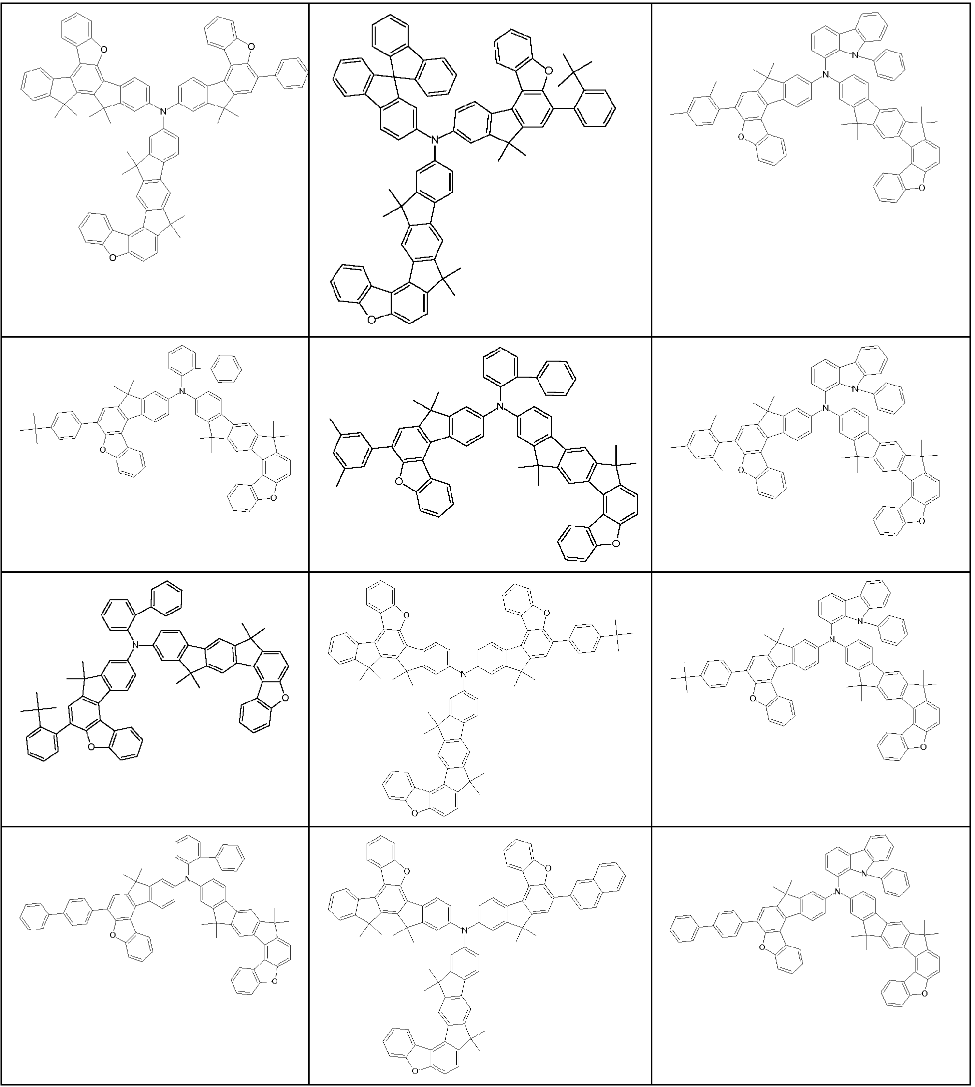

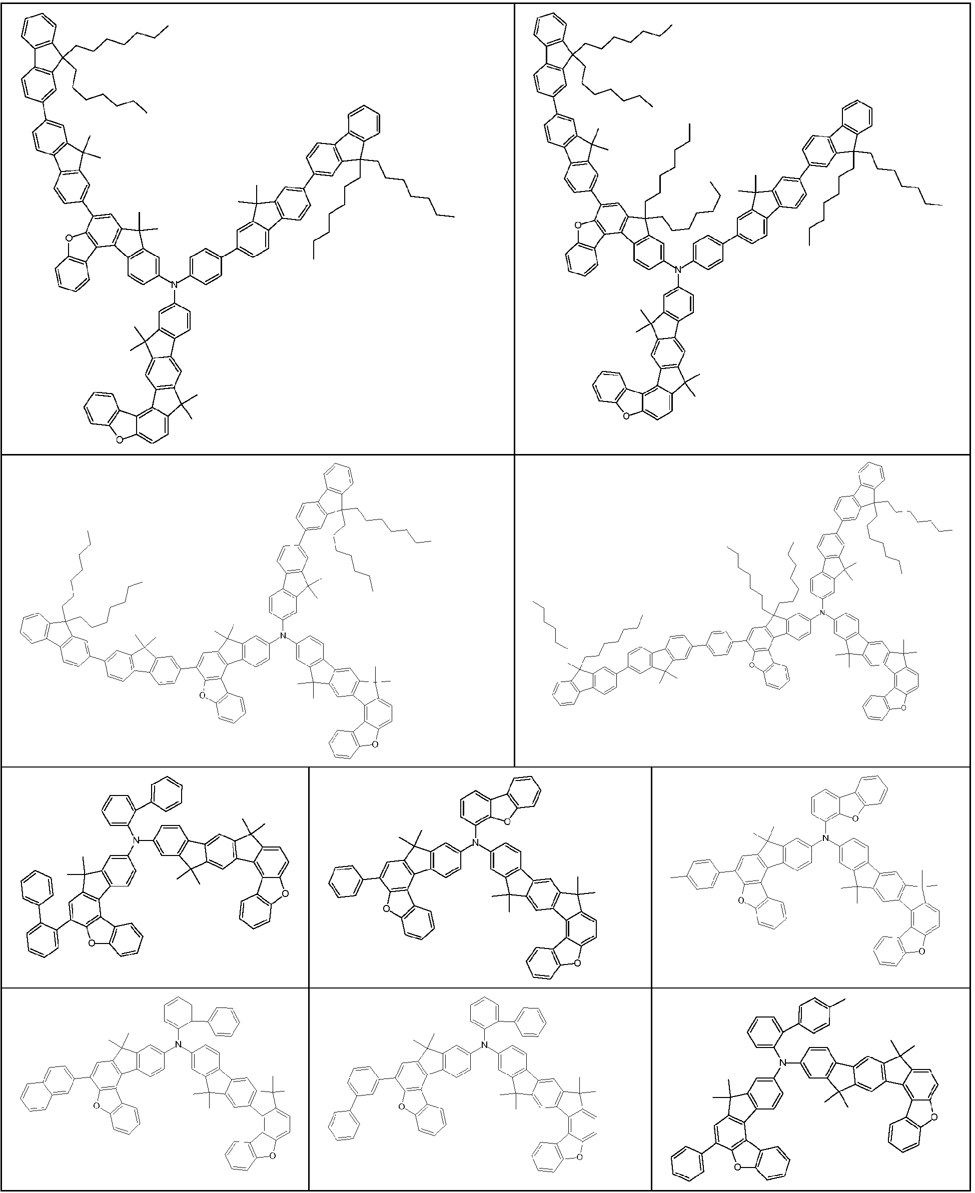

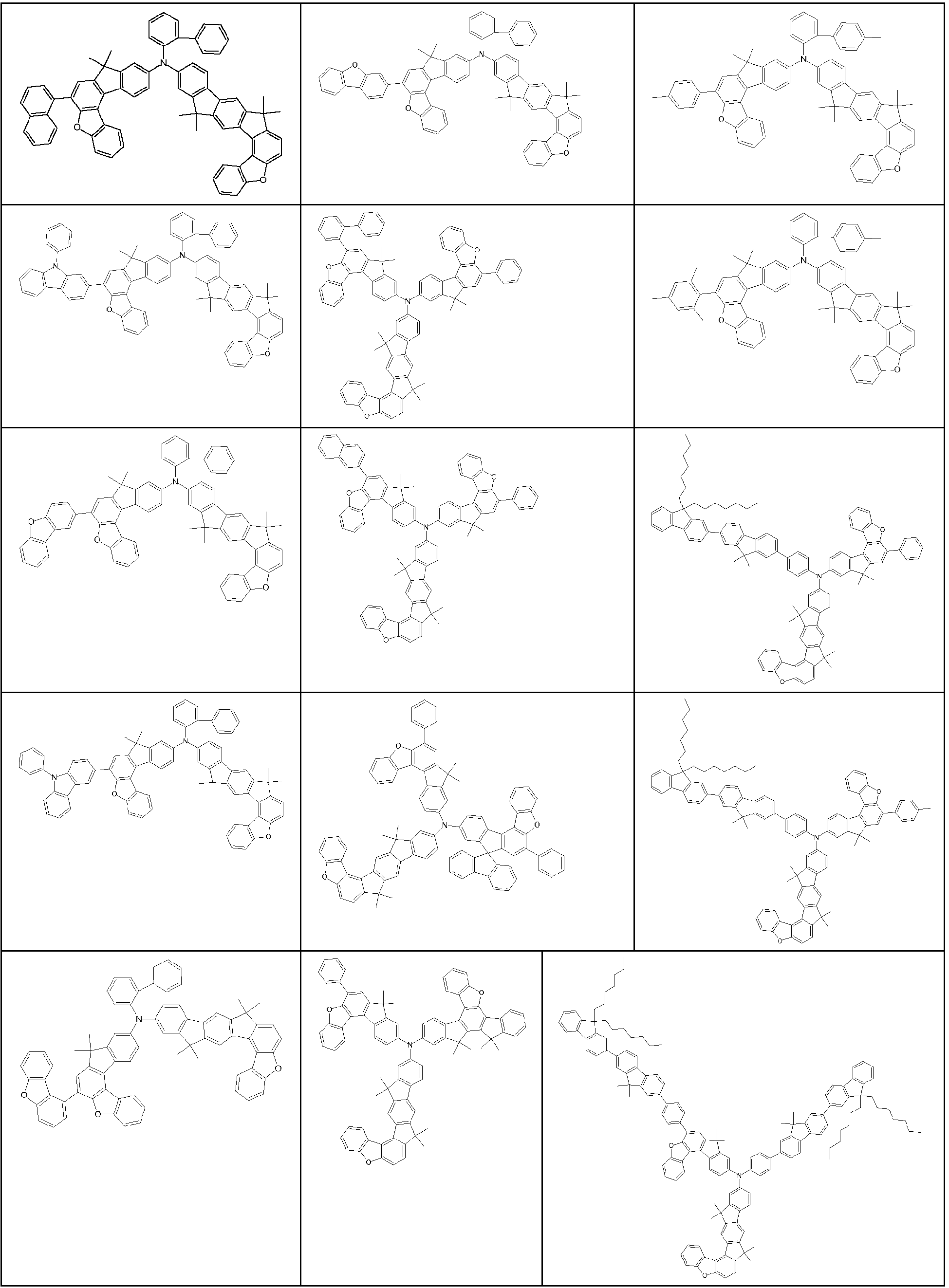

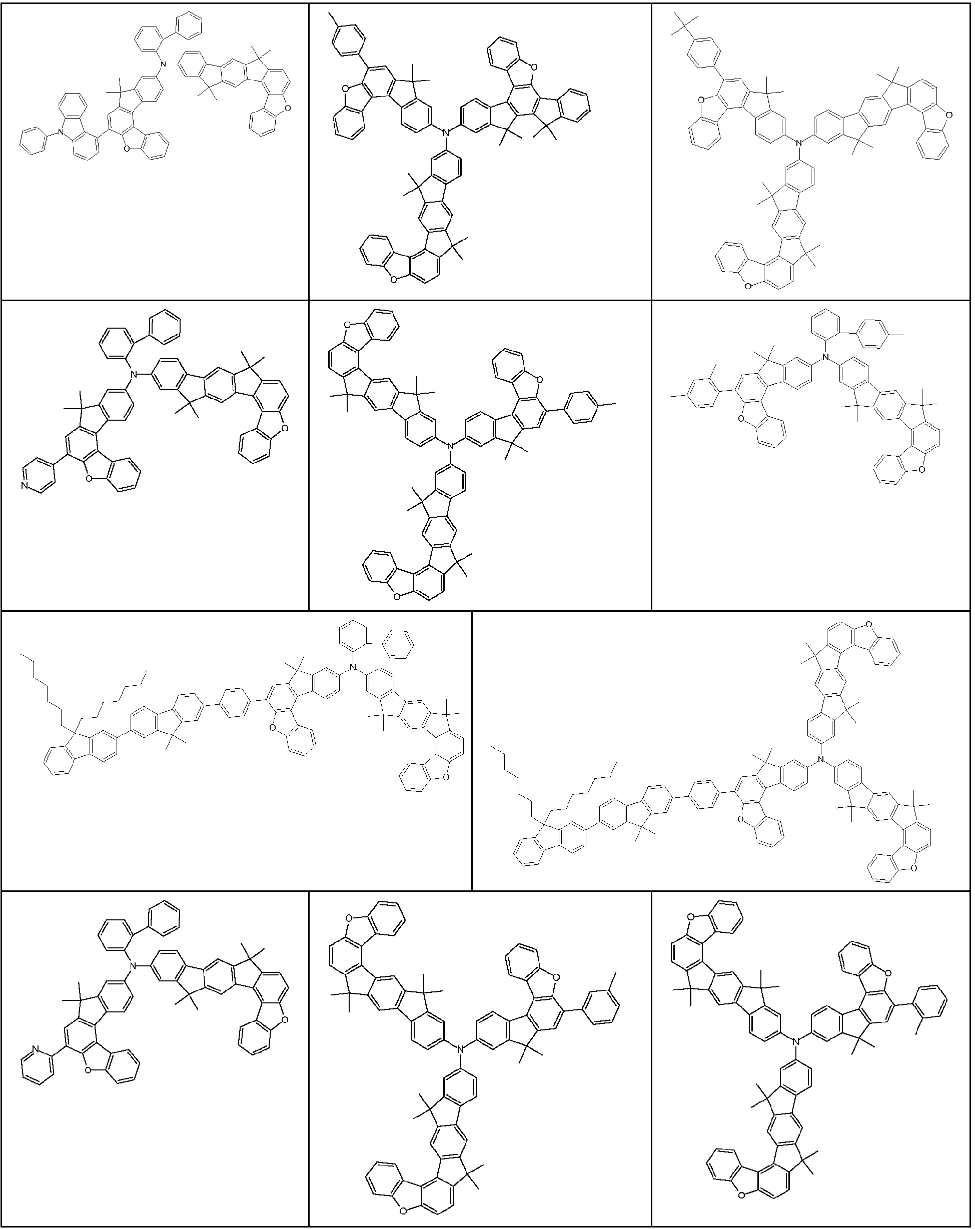

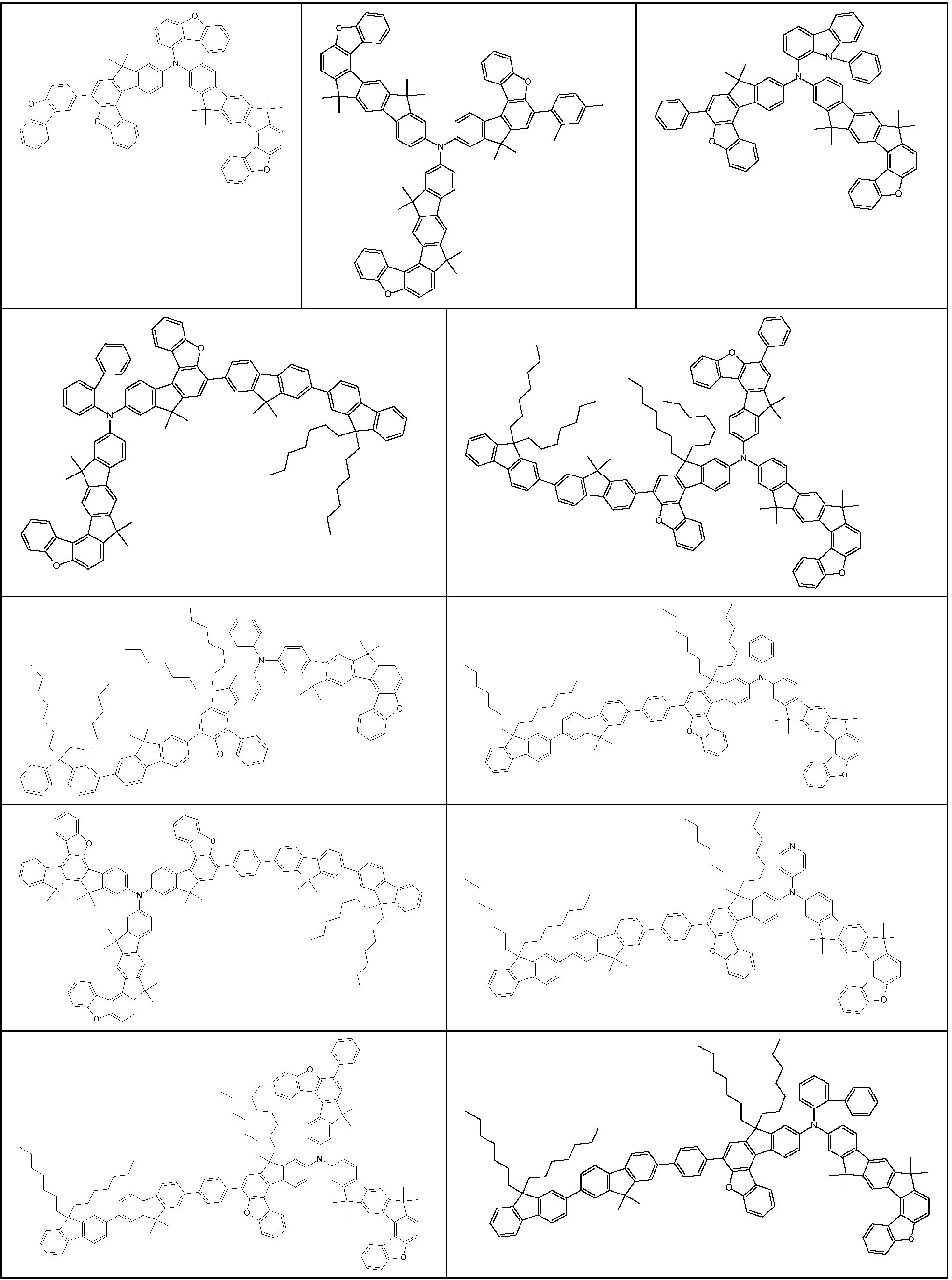

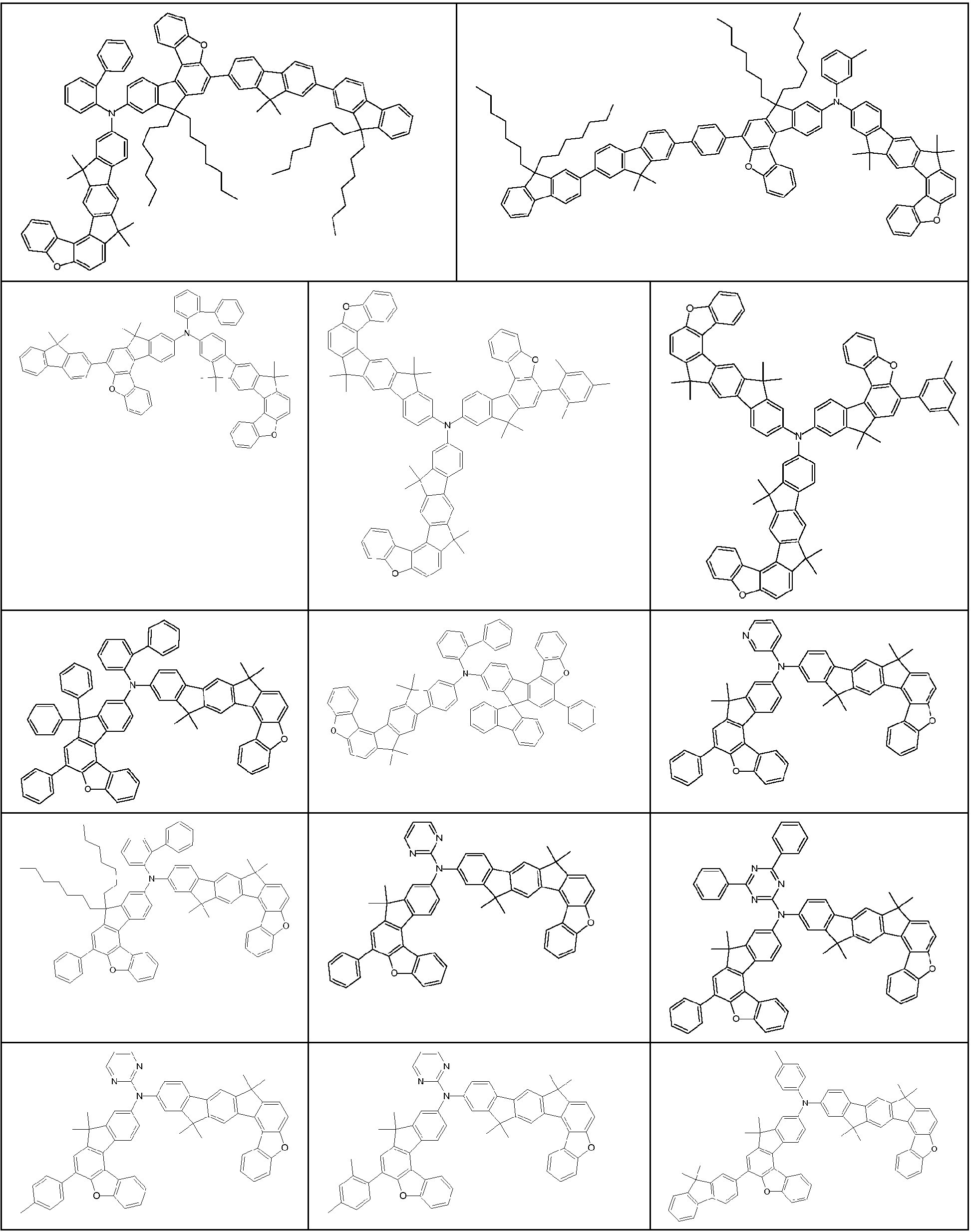

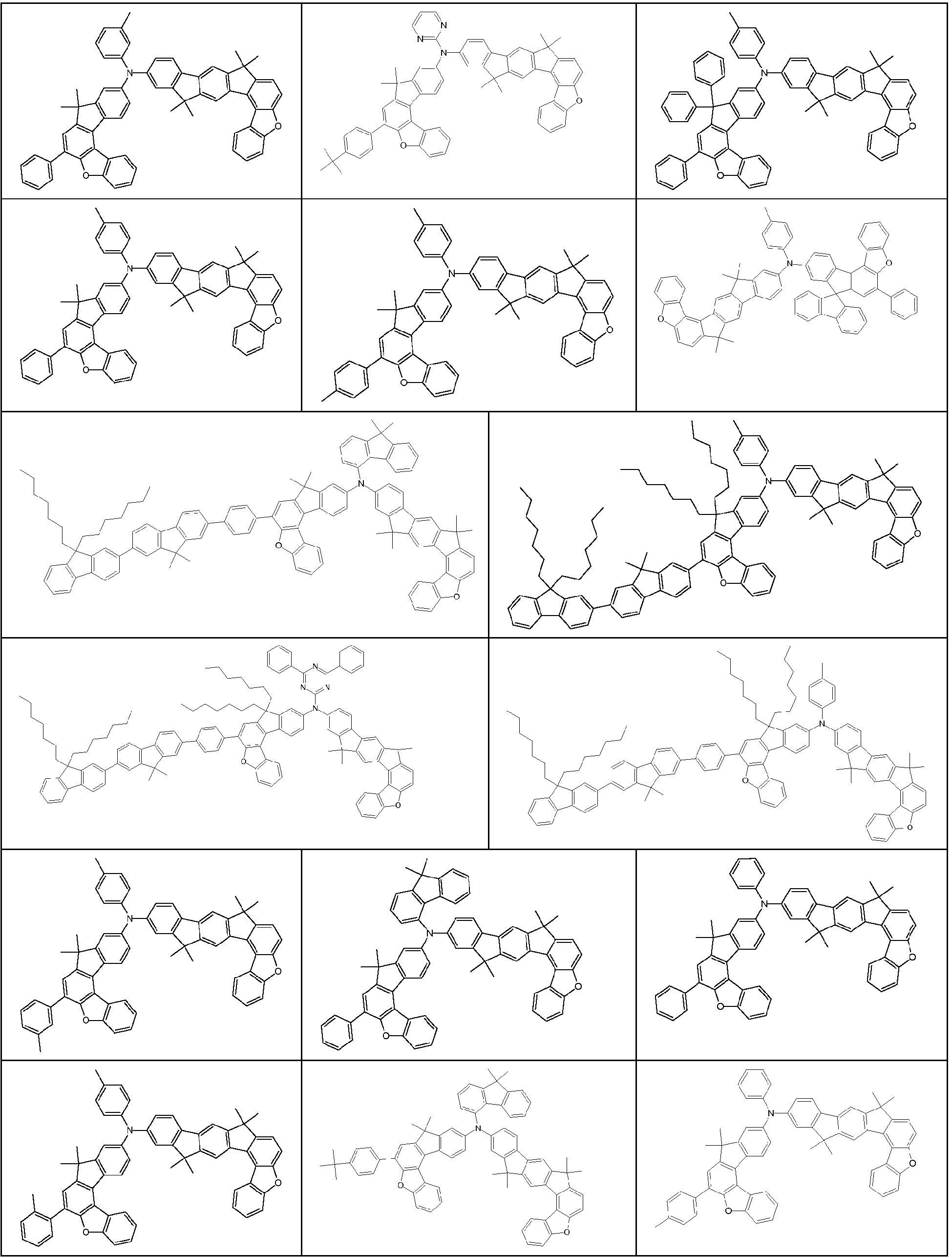

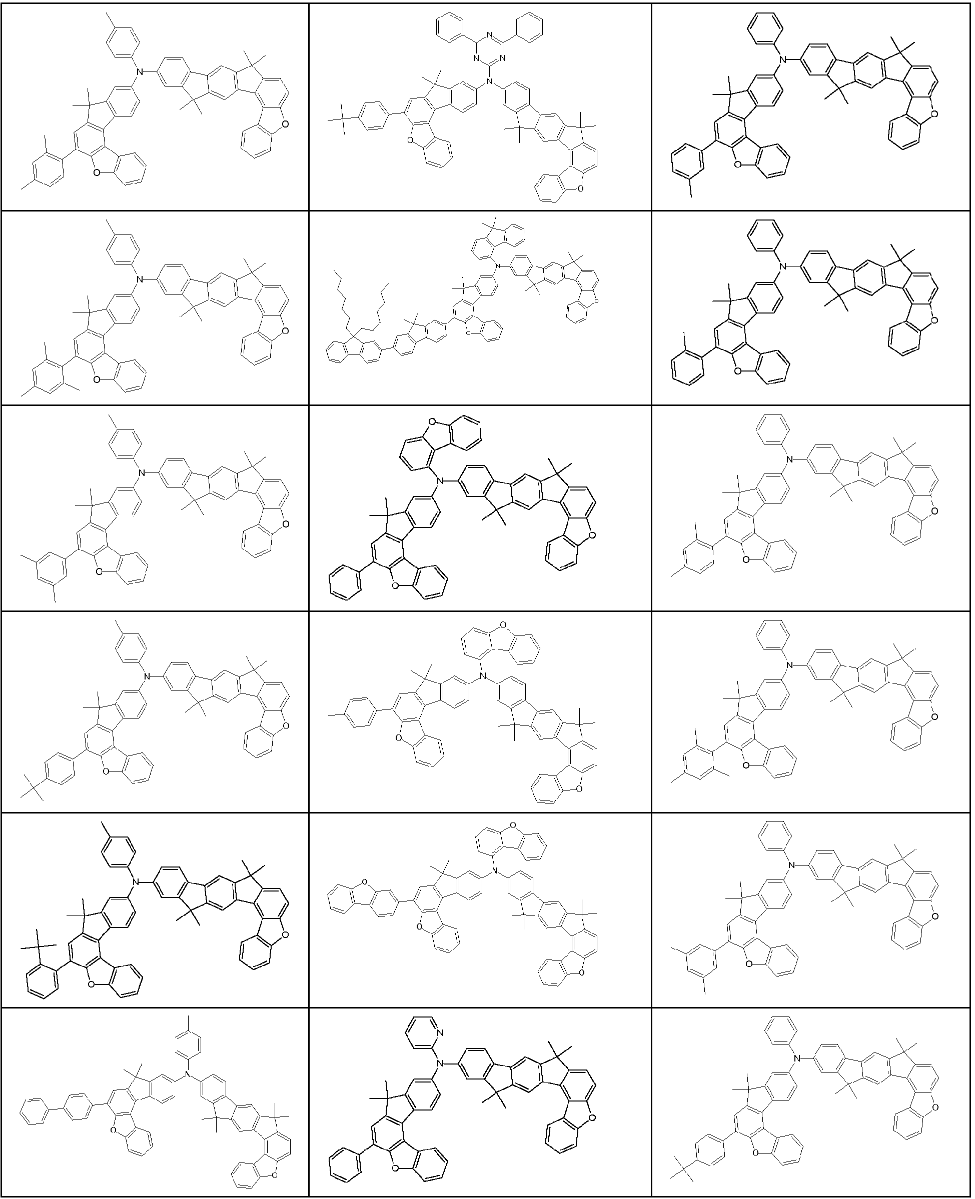

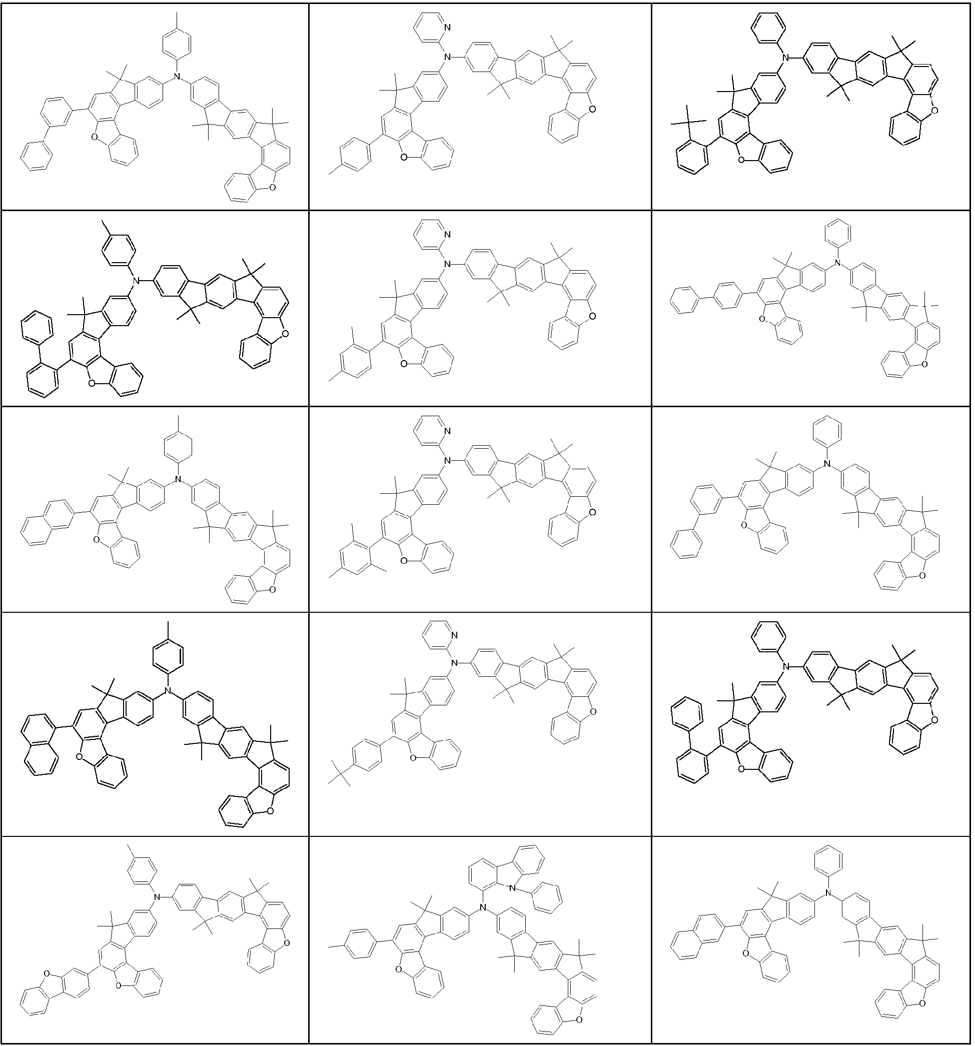

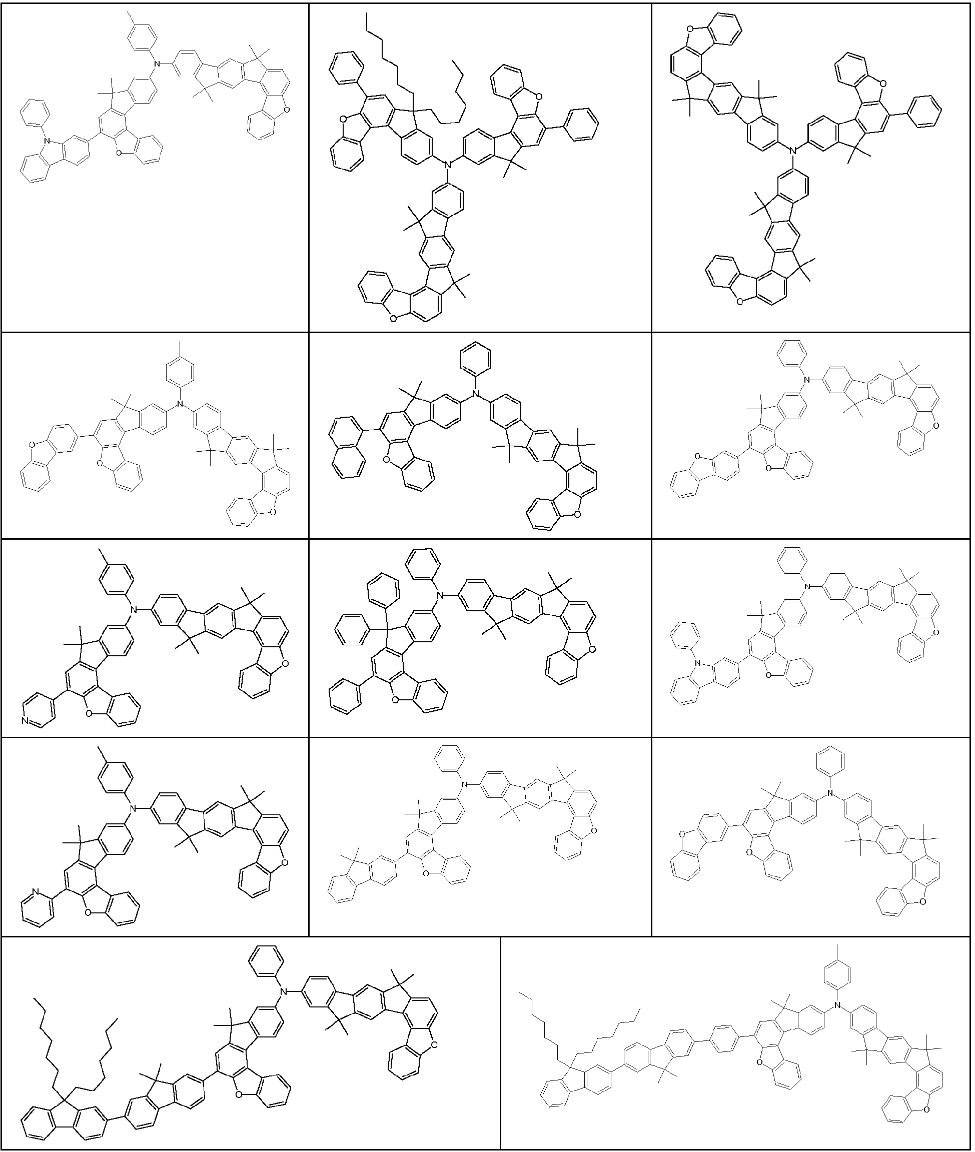

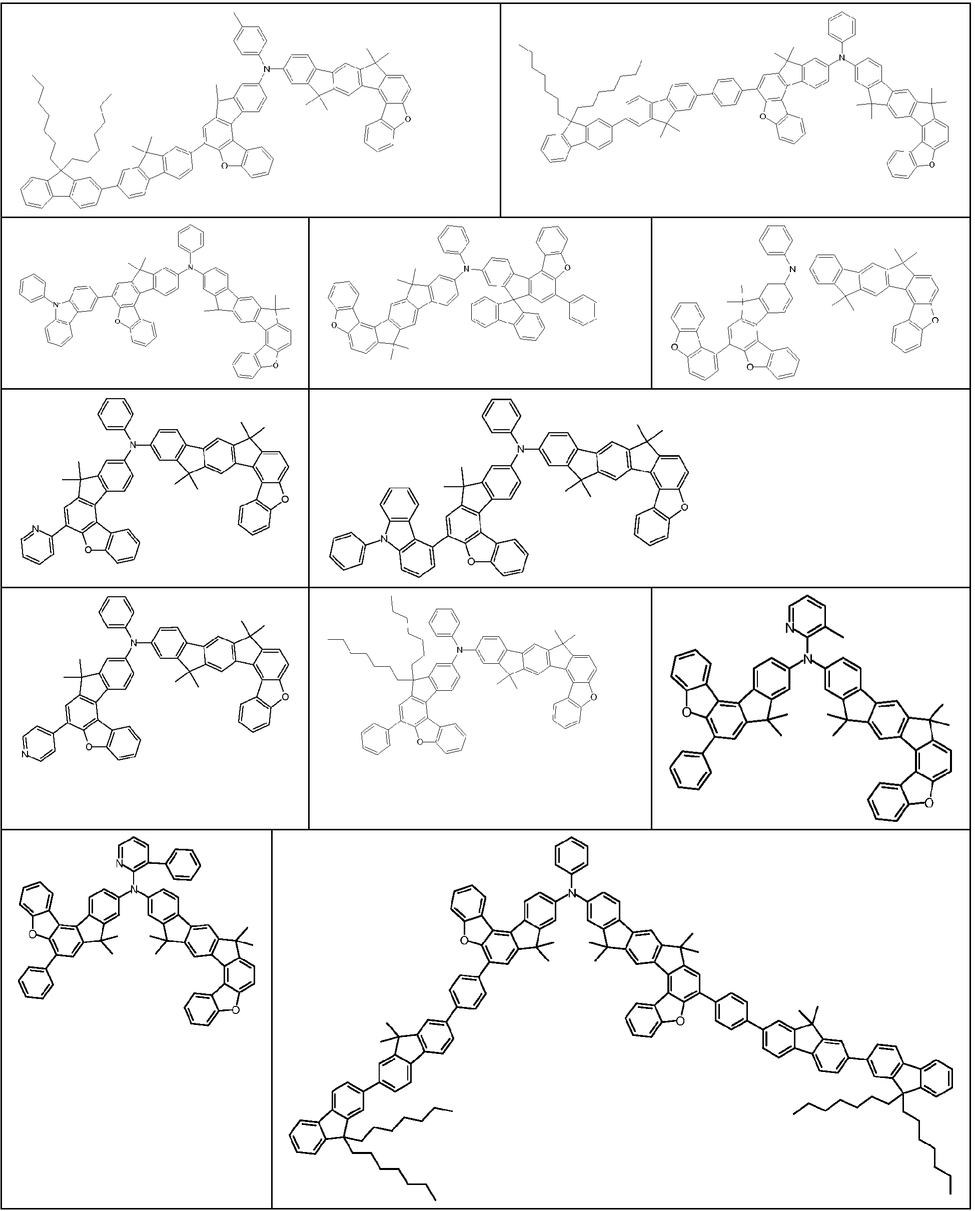

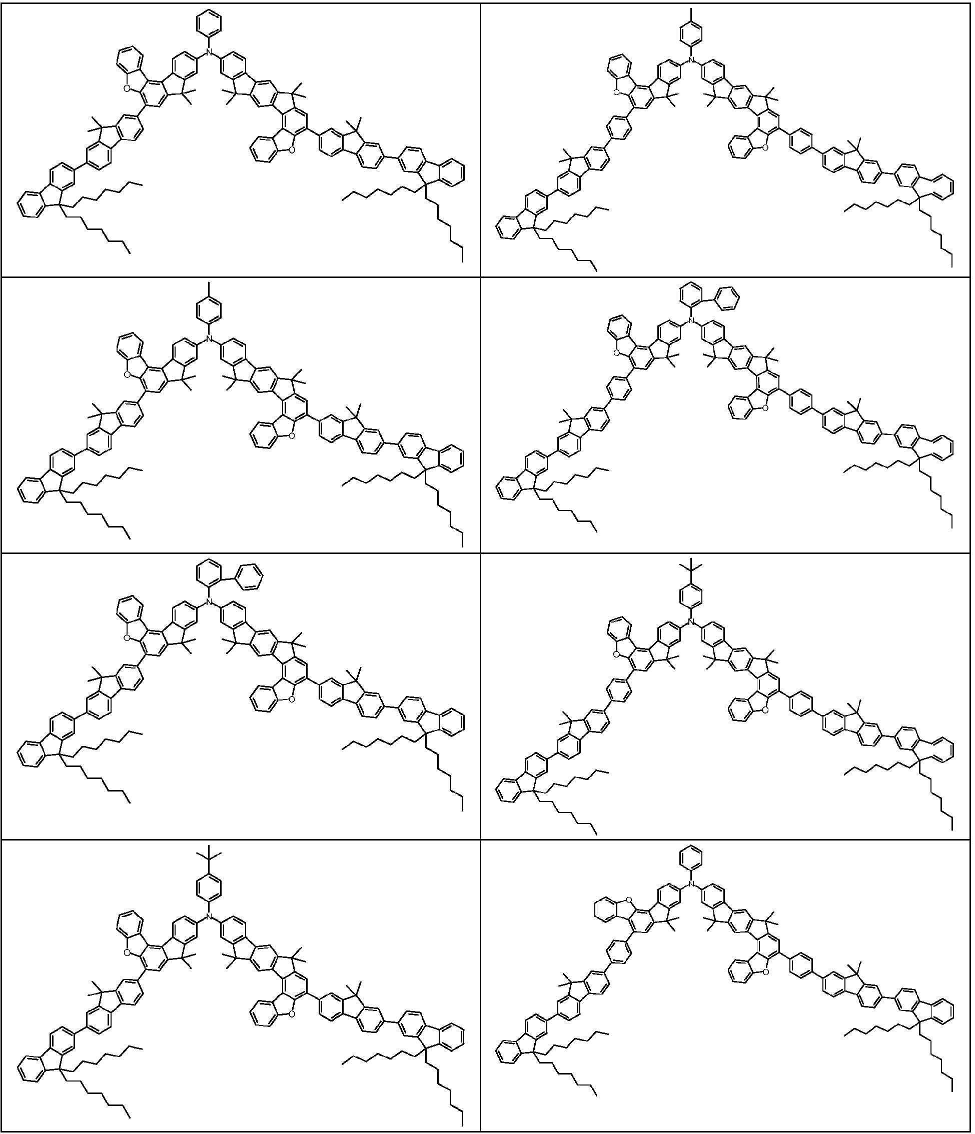

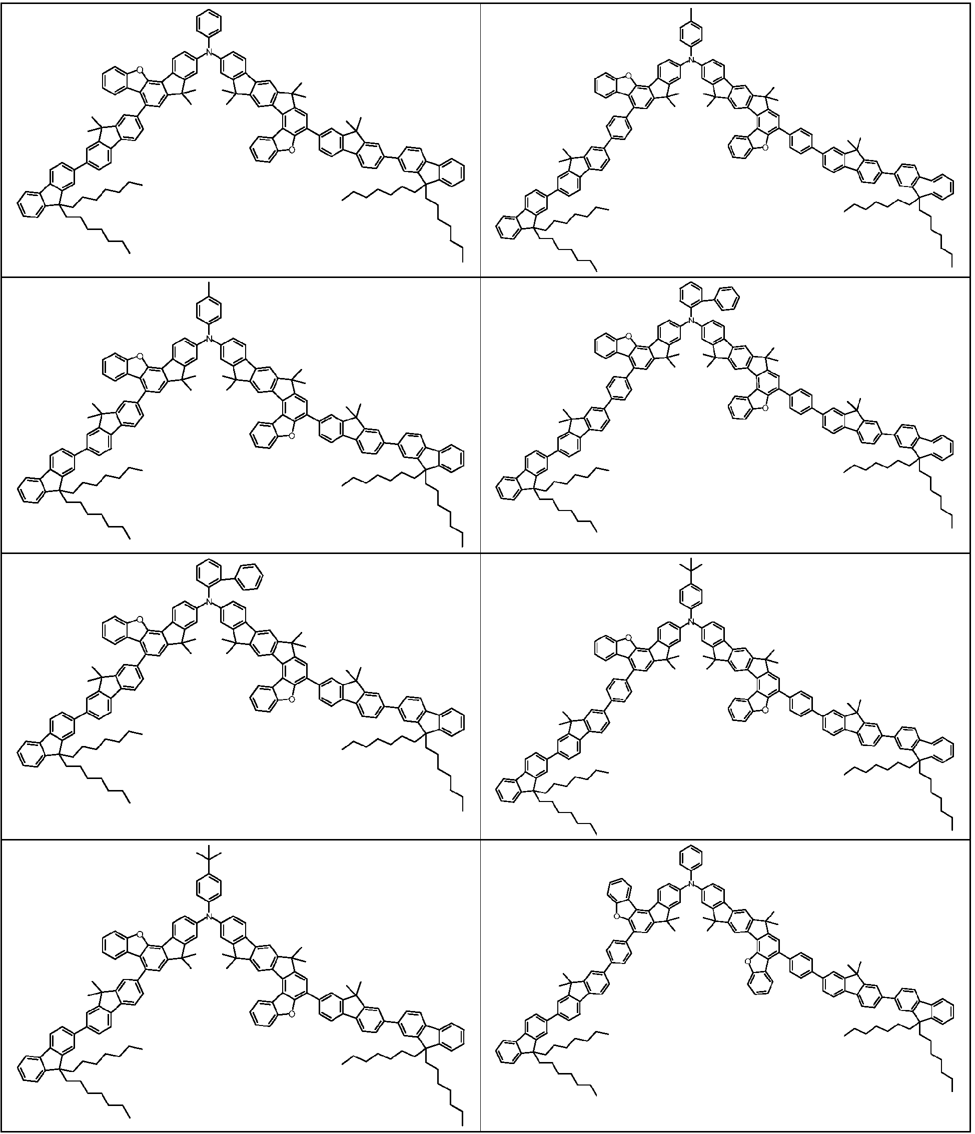

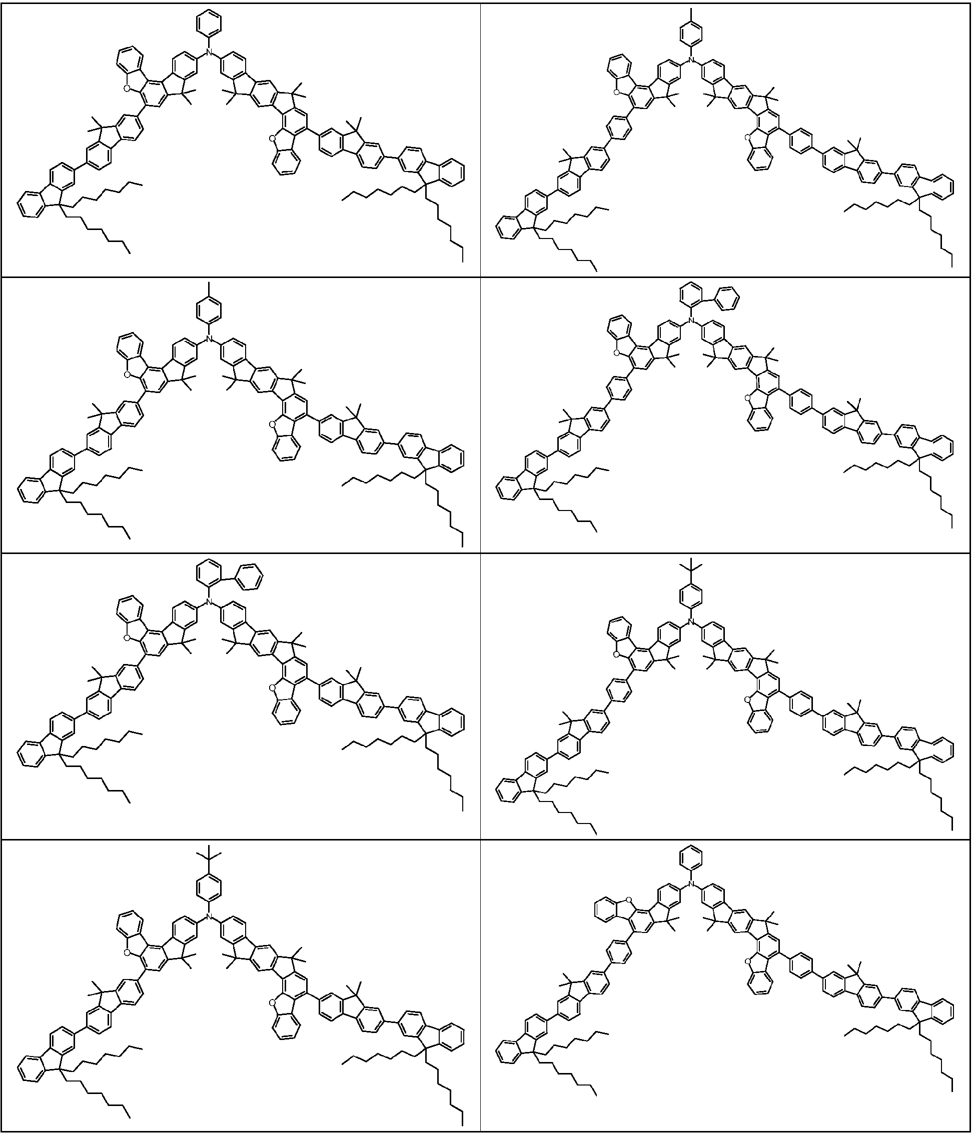

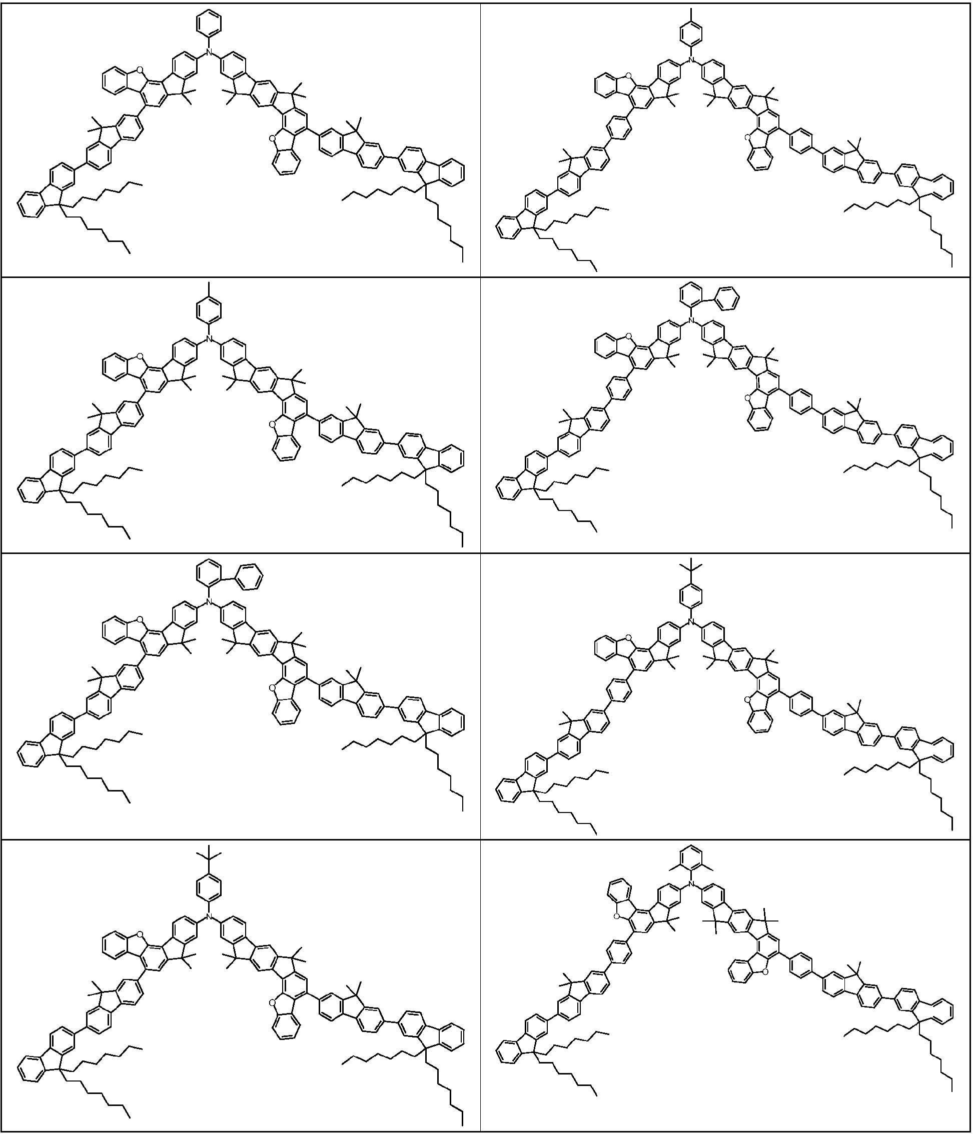

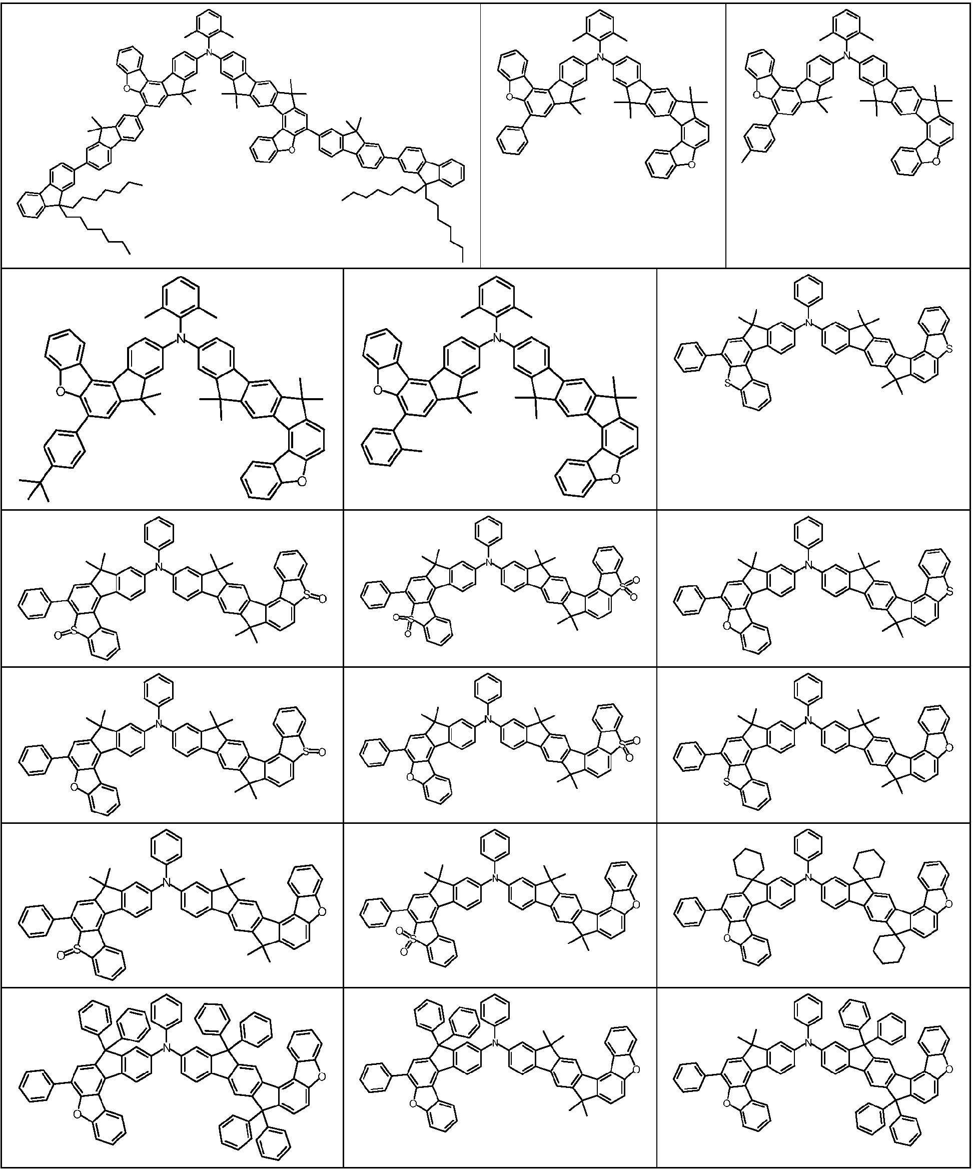

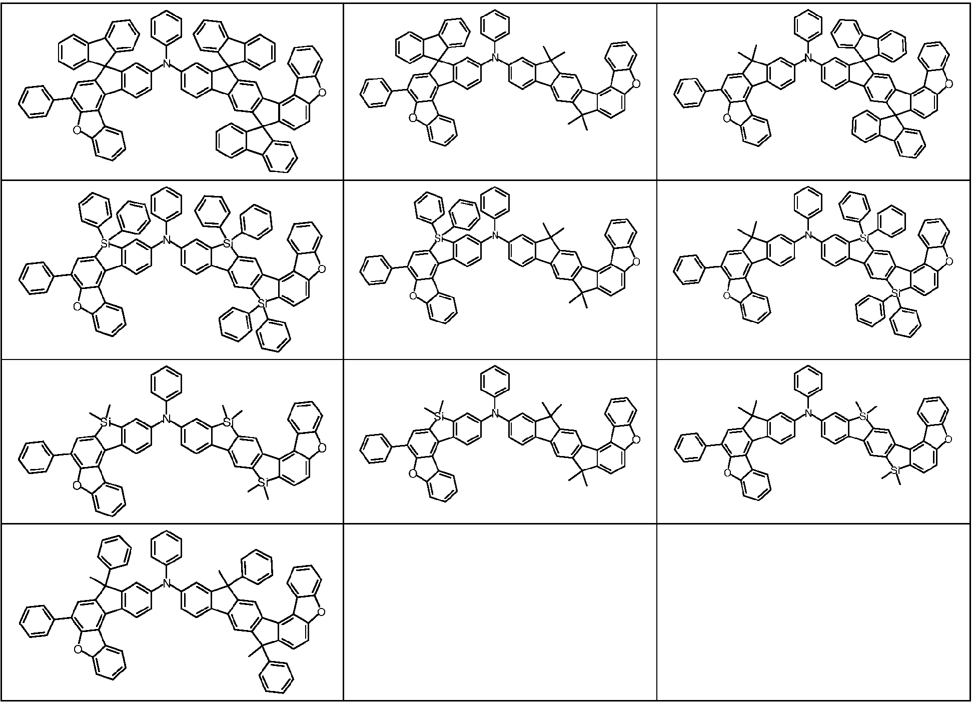

The compounds according to the invention can be prepared by synthesis steps known to the person skilled in the art, such as, for example, bromination, Suzuki coupling, Ullmann coupling, Hartwig-Buchwald coupling, etc. An example of a suitable synthesis process is depicted in general terms in Scheme 1 below.

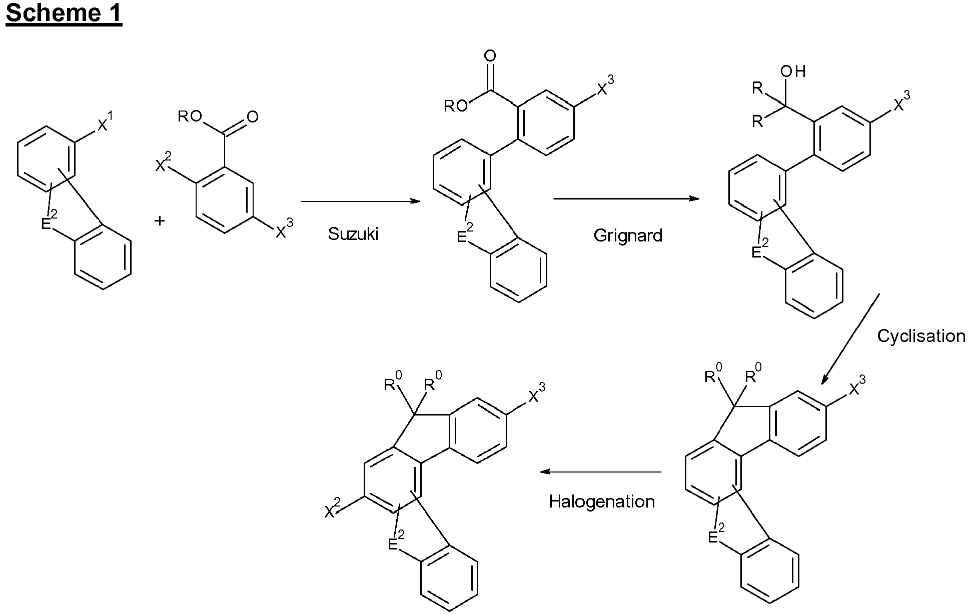

### Step 1 - Synthesis of the first intermediate

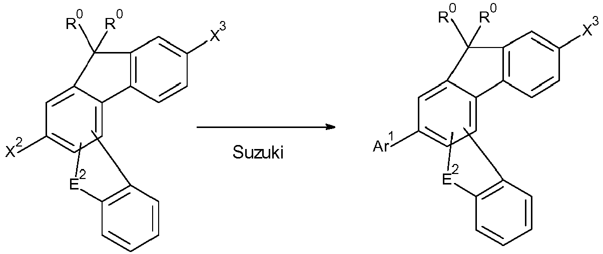

### Step 2 - Synthesis of the second intermediate

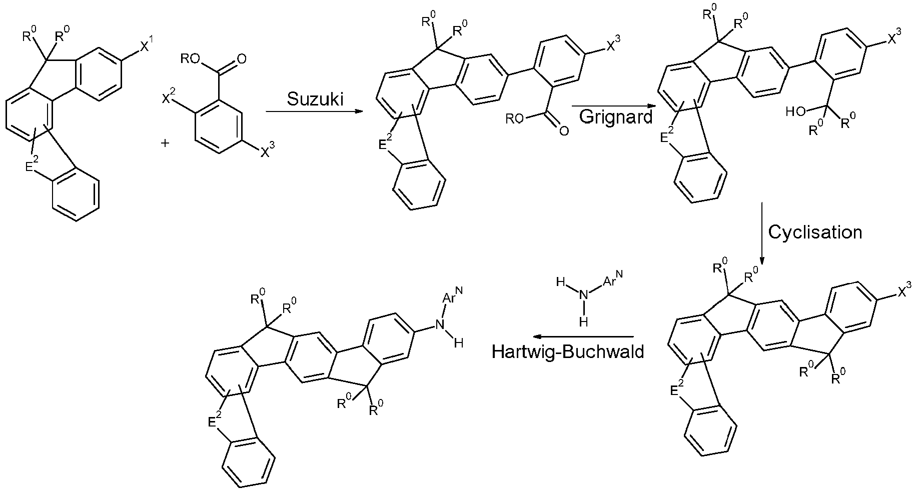

### Step 3 - Synthesis of compounds of formula (1)

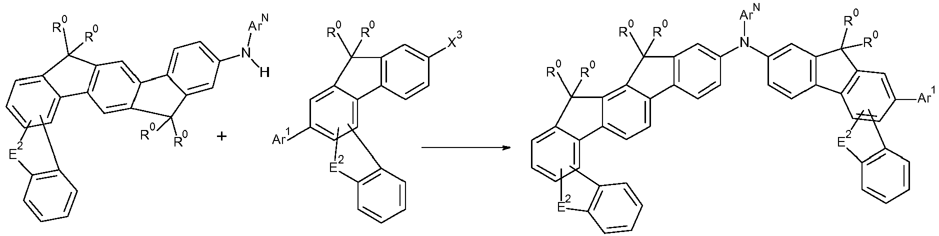

### Scheme 2

### Step 1 - Synthesis of the first intermediate (Same as step 1 of scheme 1)

### Step 2 - Synthesis of the second intermediate

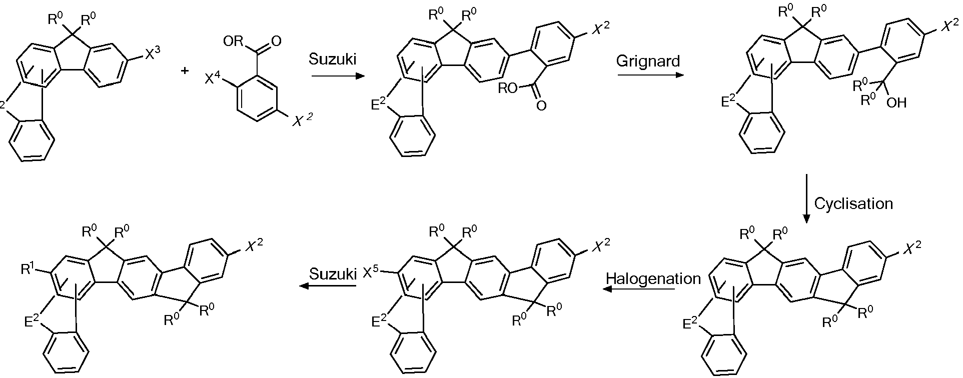

### Step 3 - Synthesis of third intermediate

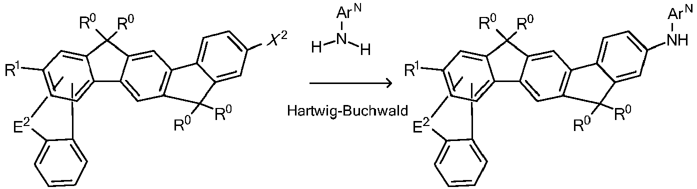

### Step 4 - Synthesis of compounds of formula (1)

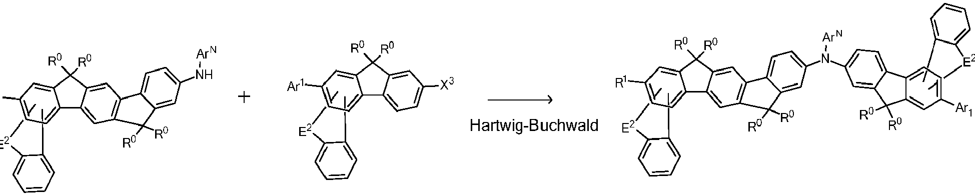

In Schemes 1 and 2, the symbols Ar¹, Ar^{N}, E², R¹, R⁰ and R have the same meaning as above, the symbols X¹, X² and X³ represent a leaving group (like a halogen or a boronic ester).

The present invention therefore relates to a process for the synthesis of the compounds according to the invention, comprising a step where an aminated oxadiindenofluorene derivative reacts with an indenodibenzofuran derivative.

For the processing of the compounds according to the invention from the liquid phase, for example by spin coating or by printing processes, formulations of the compounds according to the invention are necessary. These formulations can be, for example, solutions, dispersions or emulsions. It may be preferred to use mixtures of two or more solvents for this purpose. Suitable and preferred solvents are, for example, toluene, anisole, o-, m- or p-xylene, methyl benzoate, mesitylene, tetralin, veratrol, THF, methyl-THF, THP, chlorobenzene, dioxane, phenoxytoluene, in particular 3-phenoxytoluene, (-)-fenchone, 1,2,3,5-tetramethylbenzene, 1,2,4,5-tetramethylbenzene, 1-methylnaphthalene, 2-methylbenzothiazole, 2-phenoxyethanol, 2-pyrrolidinone, 3-methylanisole, 4-methylanisole, 3,4-dimethylanisole, 3,5-dimethylanisole, acetophenone, α-terpineol, benzothiazole, butyl benzoate, cumene, cyclohexanol, cyclohexanone, cyclohexylbenzene, decalin, dodecylbenzene, ethyl benzoate, indane, methyl benzoate, NMP, p-cymene, phenetole, 1,4-diisopropylbenzene, dibenzyl ether, diethylene glycol butyl methyl ether, triethylene glycol butyl methyl ether, diethylene glycol dibutyl ether, triethylene glycol dimethyl ether, diethylene glycol monobutyl ether, tripropylene glycol dimethyl ether, tetraethylene glycol dimethyl ether, 2-isopropylnaphthalene, pentylbenzene, hexylbenzene, heptylbenzene, octylbenzene, 1,1-bis(3,4-dimethylphenyl)ethane or mixtures of these solvents.

The present invention therefore furthermore relates to a formulation comprising a compound according to the invention and at least one further compound. The further compound may be, for example, a solvent, in particular one of the above-mentioned solvents or a mixture of these solvents. However, the further compound may also be at least one further organic or inorganic compound which is likewise employed in the electronic device, for example an emitting compound, in particular a phosphorescent dopant, and/or a further matrix material. Suitable emitting compounds and further matrix materials are indicated below in connection with the organic electroluminescent device. This further compound may also be polymeric.

The compounds and mixtures according to the invention are suitable for use in an electronic device. An electronic device here is taken to mean a device which comprises at least one layer which comprises at least one organic compound. However, the component here may also comprise inorganic materials or also layers built up entirely from inorganic materials.

The present invention therefore furthermore relates to the use of the compounds or mixtures according to the invention in an electronic device, in particular in an organic electroluminescent device.

The present invention again furthermore relates to an electronic device comprising at least one of the compounds or mixtures according to the invention mentioned above. The preferences stated above for the compound also apply to the electronic devices.

The electronic device is preferably selected from the group consisting of organic electroluminescent devices (OLEDs, PLEDs), organic integrated circuits (O-ICs), organic field-effect transistors (O-FETs), organic thin-film transistors (O-TFTs), organic light-emitting transistors (O-LETs), organic solar cells (O-SCs), organic dye-sensitised solar cells, organic optical detectors, organic photoreceptors, organic field-quench devices (O-FQDs), light-emitting electrochemical cells (LECs), organic laser diodes (O-lasers) and "organic plasmon emitting devices" (D. M. Koller et al., Nature Photonics 2008, 1-4), preferably organic electroluminescent devices (OLEDs, PLEDs), in particular phosphorescent OLEDs.

The organic electroluminescent device comprises a cathode, an anode and at least one emitting layer. Apart from these layers, it may also comprise further layers, for example in each case one or more hole-injection layers, hole-transport layers, hole-blocking layers, electron-transport layers, electron-injection layers, exciton-blocking layers, electron-blocking layers and/or charge-generation layers. It is likewise possible for interlayers, which have, for example, an exciton-blocking function, to be introduced between two emitting layers. However, it should be pointed out that each of these layers does not necessarily have to be present. The organic electroluminescent device here may comprise one emitting layer or a plurality of emitting layers. If a plurality of emission layers are present, these preferably have in total a plurality of emission maxima between 380 nm and 750 nm, resulting overall in white emission, i.e. various emitting compounds which are able to fluoresce or phosphoresce are used in the emitting layers. Particular preference is given to systems having three emitting layers, where the three layers exhibit blue, green and orange or red emission (for the basic structure see, for example, WO 2005/011013). These can be fluorescent or phosphorescent emission layers or hybrid systems, in which fluorescent and phosphorescent emission layers are combined with one another.

The compound according to the invention in accordance with the embodiments indicated above can be employed in various layers, depending on the precise structure and on the substitution. Preference is given to an organic electroluminescent device comprising a compound of the formula (1) or in accordance with the preferred embodiments as fluorescent emitters, emitters showing TADF (Thermally Activated Delayed Fluorescence), matrix material for fluorescent emitters. Particularly preferred is an organic electroluminescent device comprising a compound of the formula (1) or in accordance with the preferred embodiments as fluorescent emitters, more particularly blue-emitting fluorescent compound.

The compounds of formula (1) can also be employed in an electron-transport layer and/or in an electron-blocking or exciton-blocking layer and/or in a hole-transport layer, depending on the precise substitution. The preferred embodiments indicated above also apply to the use of the materials in organic electronic devices.

The compound according to the invention is particularly suitable for use as blue-emitting emitter compound. The electronic device concerned may comprise a single emitting layer comprising the compound according to the invention or it may comprise two or more emitting layers. The further emitting layers here may comprise one or more compounds according to the invention or alternatively other compounds.

If the compound according to the invention is employed as a fluorescent emitting compound in an emitting layer, it is preferably employed in combination with one or more matrix materials. A matrix material here is taken to mean a material which is present in the emitting layer, preferably as the principal component, and which does not emit light on operation of the device.

The proportion of the emitting compound in the mixture of the emitting layer is between 0.1 and 50.0%, preferably between 0.5 and 20.0%, particularly preferably between 1.0 and 10.0%. Correspondingly, the proportion of the matrix material or matrix materials is between 50.0 and 99.9%, preferably between 80.0 and 99.5%, particularly preferably between 90.0 and 99.0%.

The specifications of the proportions in % are, for the purposes of the present application, taken to mean % by vol. if the compounds are applied from the gas phase and % by weight if the compounds are applied from solution.

Preferred matrix materials for use in combination with fluorescent emitting compounds are selected from the classes of the oligoarylenes (for example 2,2',7,7'-tetraphenylspirobifluorene in accordance with EP 676461 or dinaphthylanthracene), in particular the oligoarylenes containing condensed aromatic groups, the oligoarylenevinylenes (for example DPVBi or spiro-DPVBi in accordance with EP 676461), the polypodal metal complexes (for example in accordance with WO 2004/081017), the hole-conducting compounds (for example in accordance with WO 2004/058911), the electronconducting compounds, in particular ketones, phosphine oxides, sulfoxides, etc. (for example in accordance with WO 2005/084081 and WO 2005/084082), the atropisomers (for example in accordance with WO 2006/048268), the boronic acid derivatives (for example in accordance with WO 2006/117052) or the benzanthracenes (for example in accordance with WO 2008/145239). Particularly preferred matrix materials are selected from the classes of the oligoarylenes, comprising naphthalene, anthracene, benzanthracene and/or pyrene or atropisomers of these compounds, the oligoarylenevinylenes, the ketones, the phosphine oxides and the sulfoxides. Very particularly preferred matrix materials are selected from the classes of the oligoarylenes, comprising anthracene, benzanthracene, benzophenanthrene and/or pyrene or atropisomers of these compounds. An oligoarylene in the sense of this invention is intended to be taken to mean a compound in which at least three aryl or arylene groups are bonded to one another.

Particularly preferred matrix materials for use in combination with the compounds of the formula (1) in the emitting layer are depicted in the following table.

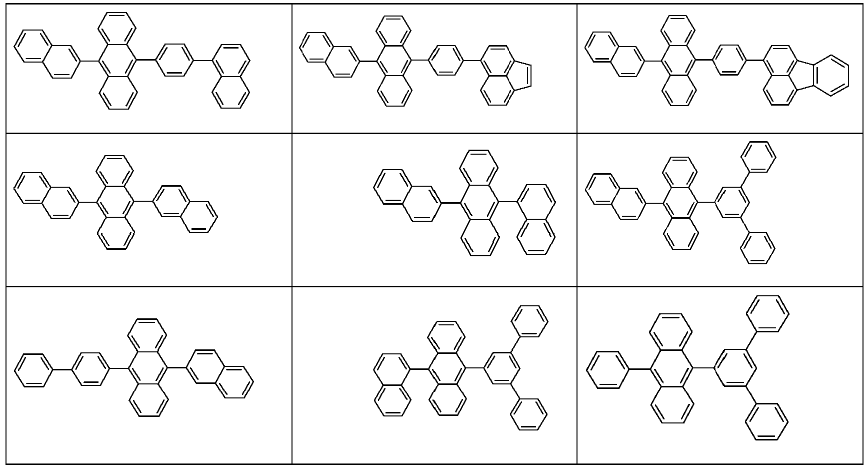

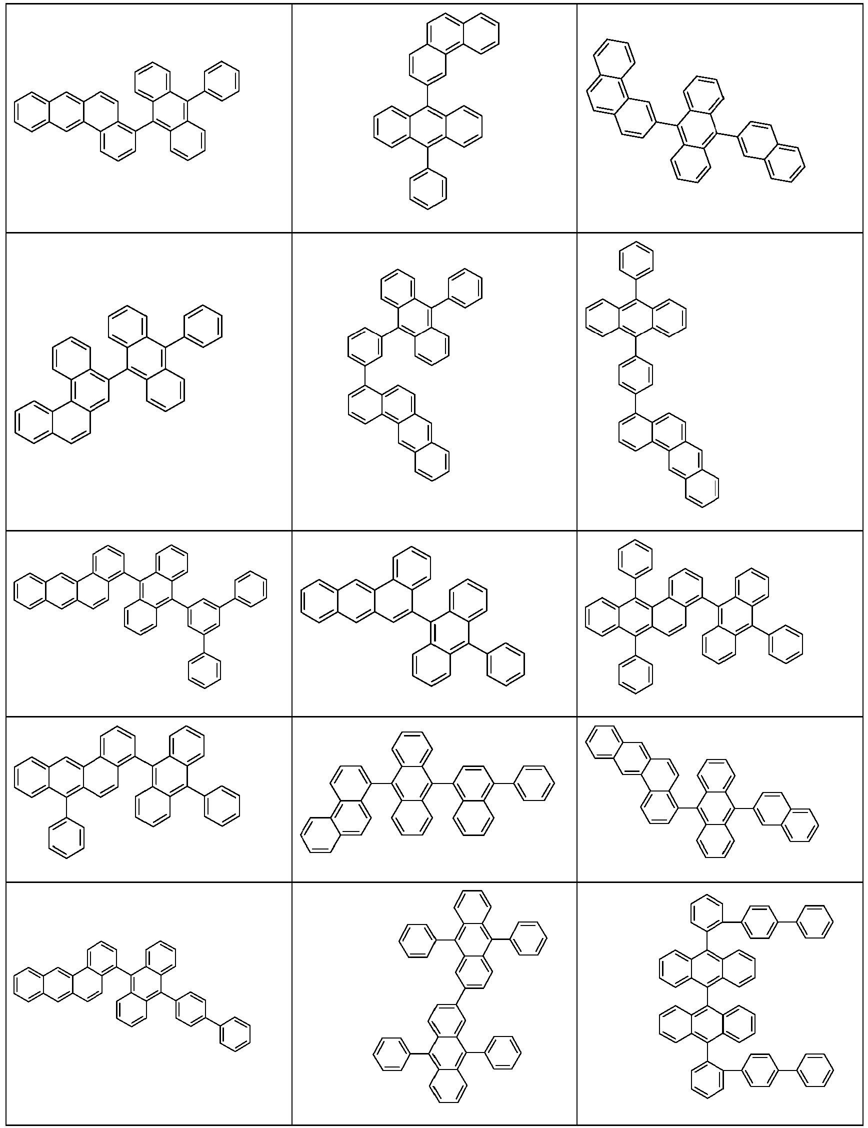

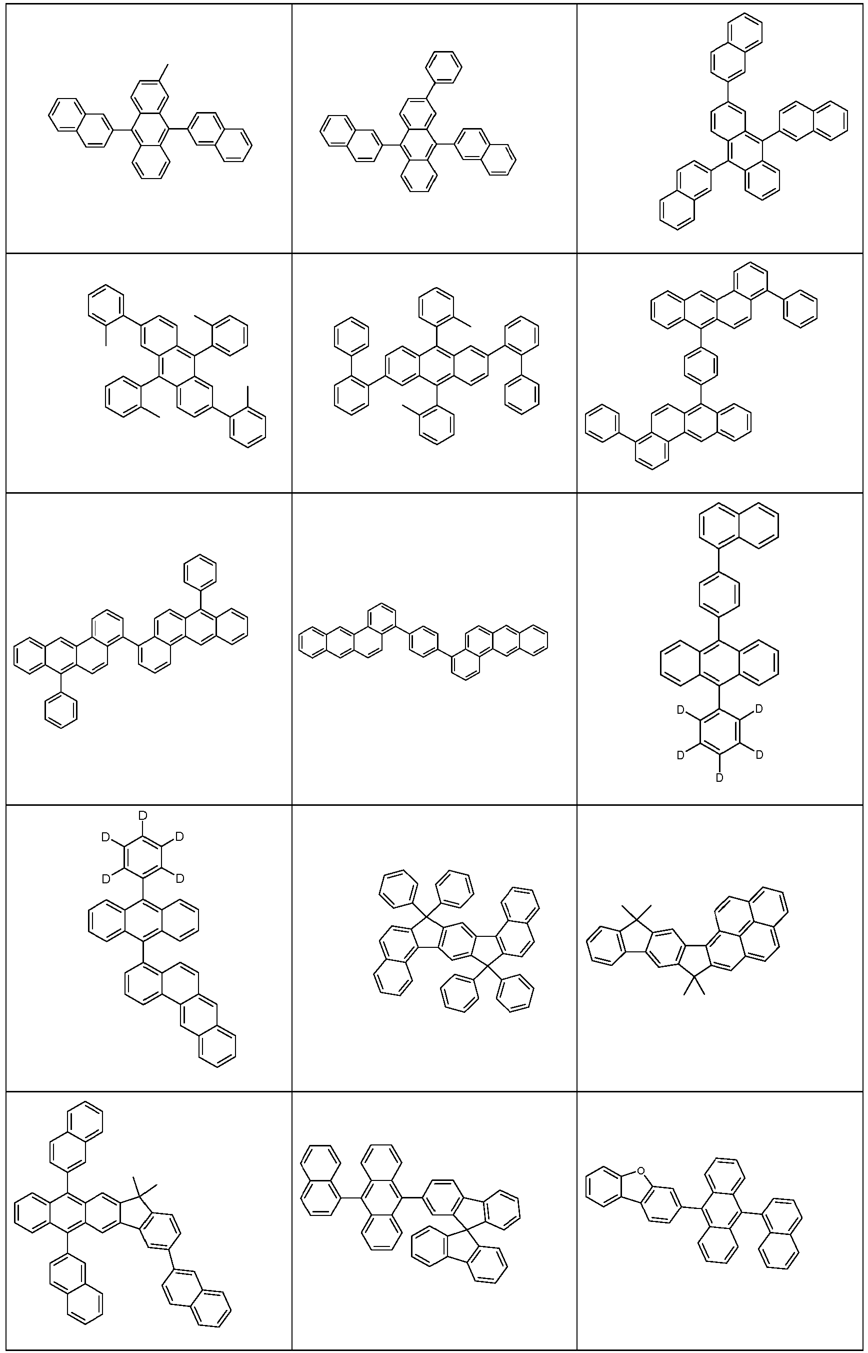

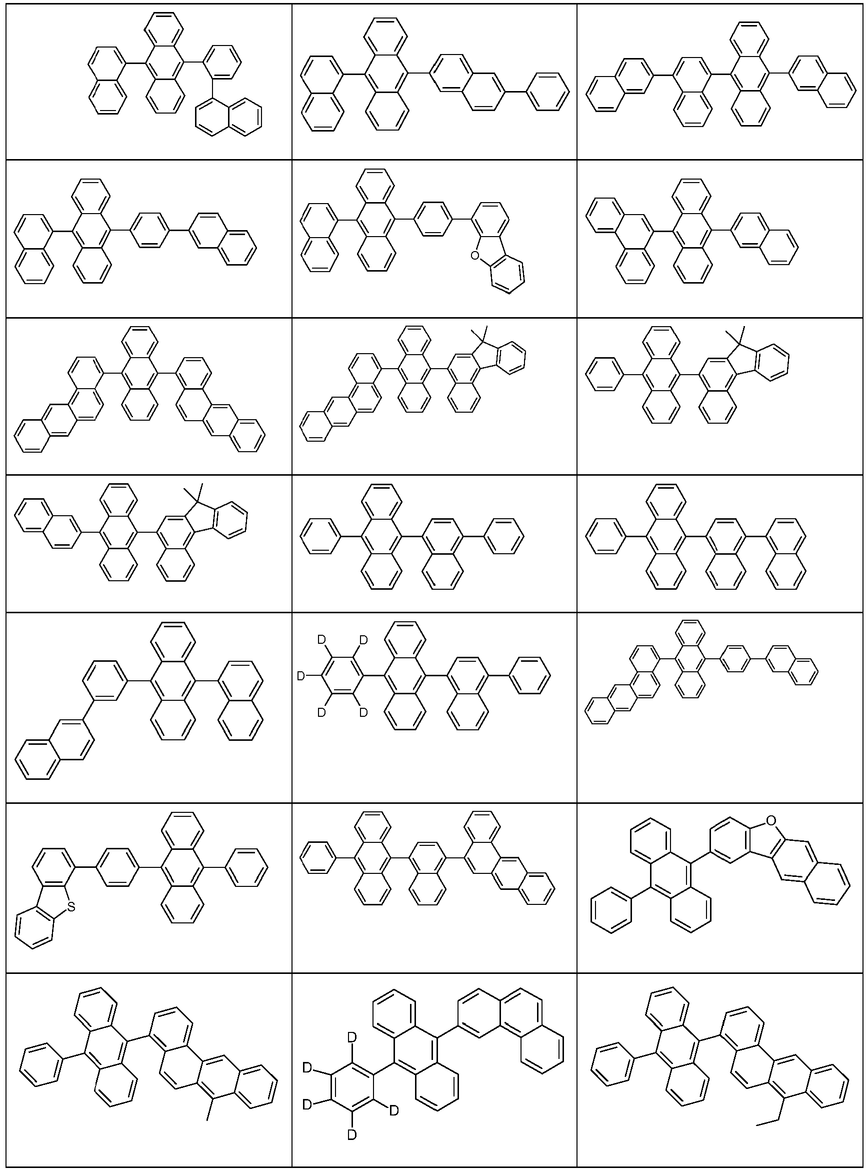

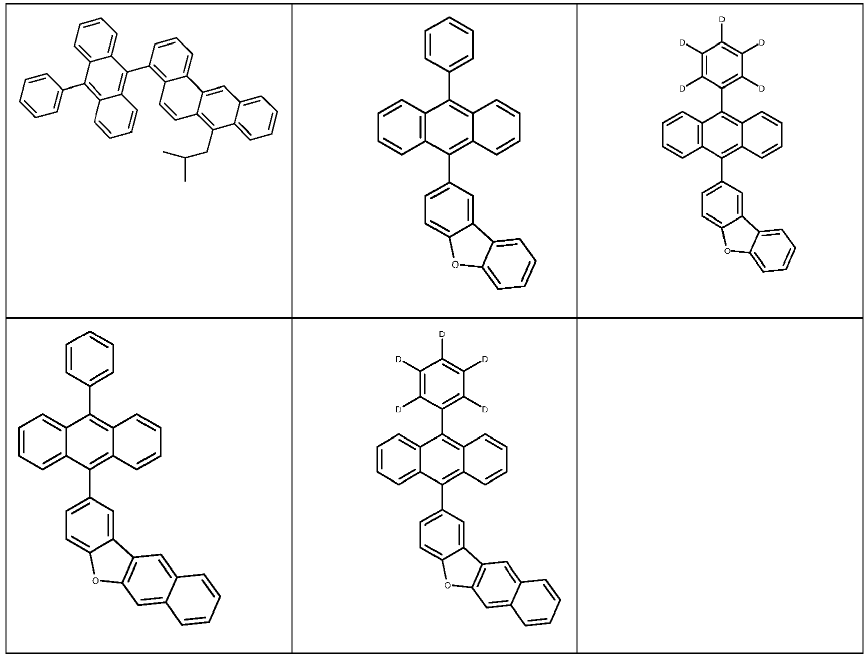

If the compound according to the invention is employed as a fluorescent emitting compound in an emitting layer, it may be employed in combination with one or more other fluorescent emitting compounds.

Preferred fluorescent emitters, besides the compounds according to the invention, are selected from the class of the arylamines. An arylamine in the sense of this invention is taken to mean a compound which contains three substituted or unsubstituted aromatic or heteroaromatic ring systems bonded directly to the nitrogen. At least one of these aromatic or heteroaromatic ring systems is preferably a condensed ring system, particularly preferably having at least 14 aromatic ring atoms. Preferred examples thereof are aromatic anthracenamines, aromatic anthracenediamines, aromatic pyrenamines, aromatic pyrenediamines, aromatic chrysenamines or aromatic chrysenediamines. An aromatic anthracenamine is taken to mean a compound in which one diarylamino group is bonded directly to an anthracene group, preferably in the 9-position. An aromatic anthracenediamine is taken to mean a compound in which two diarylamino groups are bonded directly to an anthracene group, preferably in the 9,10-position. Aromatic pyrenamines, pyrenediamines, chrysenamines and chrysenediamines are defined analogously thereto, where the diarylamino groups are preferably bonded to the pyrene in the 1-position or in the 1,6-position. Further preferred emitters are indenofluorenamines or indenofluorenediamines, for example in accordance with WO 2006/108497 or WO 2006/122630, benzoindenofluorenamines or benzoindenofluorenediamines, for example in accordance with WO 2008/006449, and dibenzoindenofluorenamines or dibenzoindenofluorenediamines, for example in accordance with WO 2007/140847, and the indenofluorene derivatives containing condensed aryl groups which are disclosed in WO 2010/012328. Still further preferred emitters are benzanthracene derivatives as disclosed in WO 2015/158409, anthracene derivatives as disclosed in WO 2017/036573, fluorene dimers like in WO 2016/150544 or phenoxazine derivatives as disclosed in WO 2017/028940 and WO 2017/028941. Preference is likewise given to the pyrenarylamines disclosed in WO 2012/048780 and WO 2013/185871. Preference is likewise given to the benzoindenofluorenamines disclosed in WO 2014/037077, the benzofluorenamines disclosed in WO 2014/106522 and the indenofluorenes disclosed in WO 2014/111269 or WO 2017/036574.

Examples of preferred fluorescent emitting compounds, besides the compounds according to the invention, which can be used in combination with the compounds of the invention in an emitting layer or which can be used in another emitting layer of the same device are depicted in the following table:

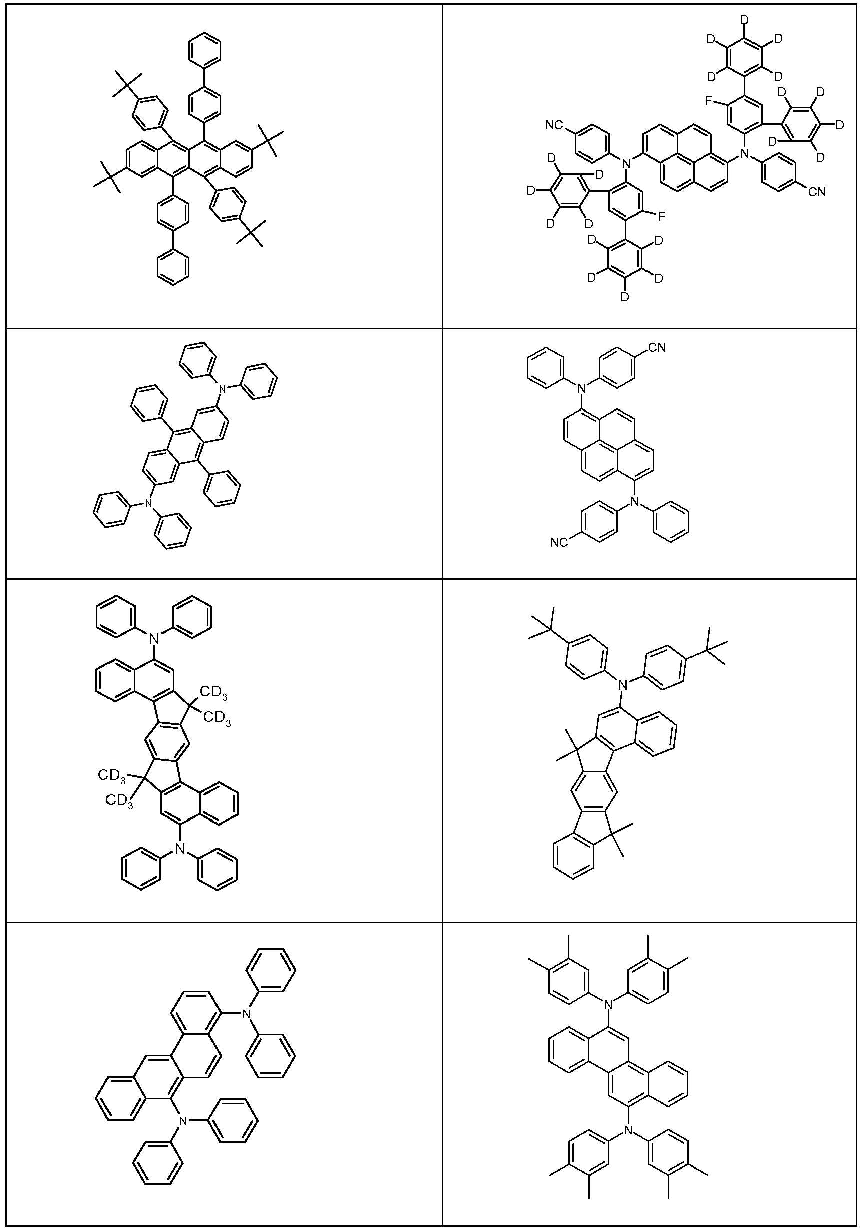

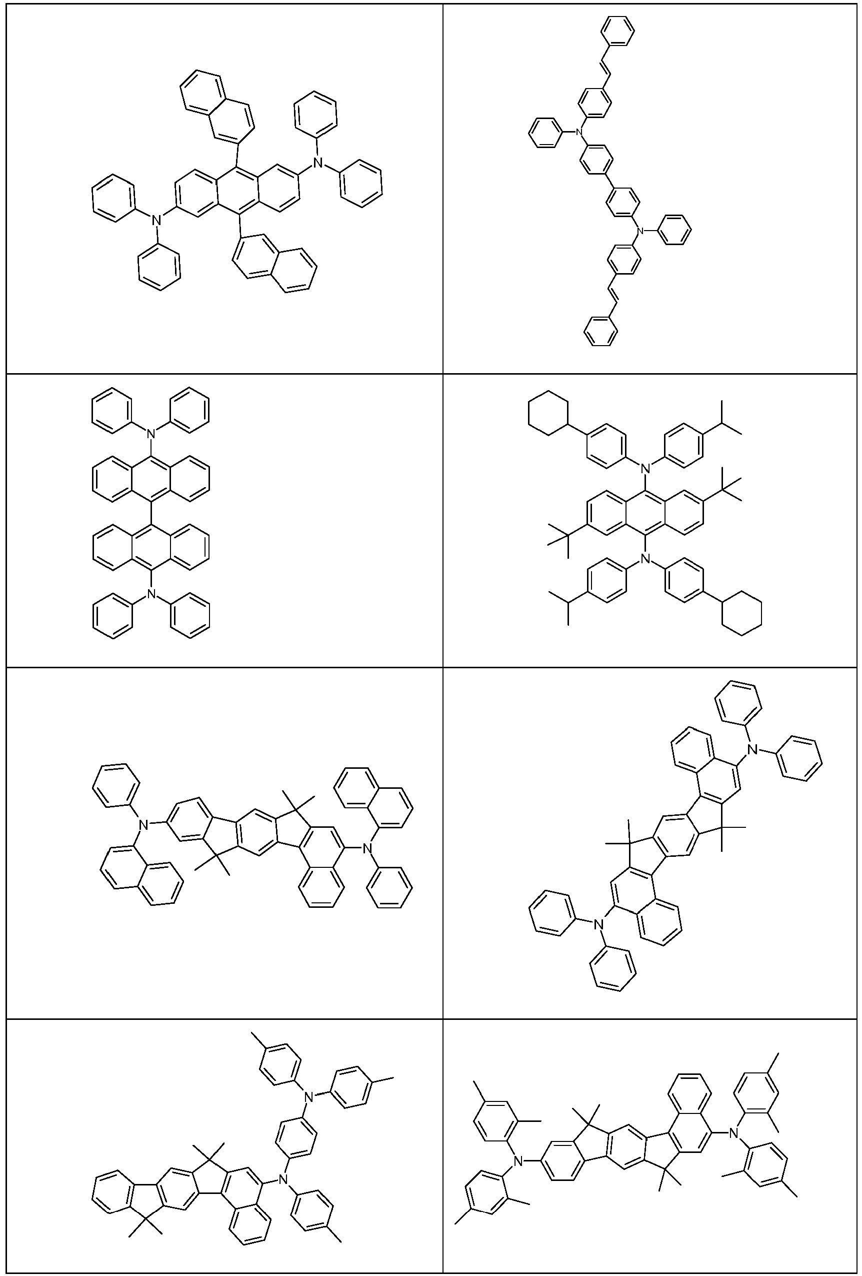

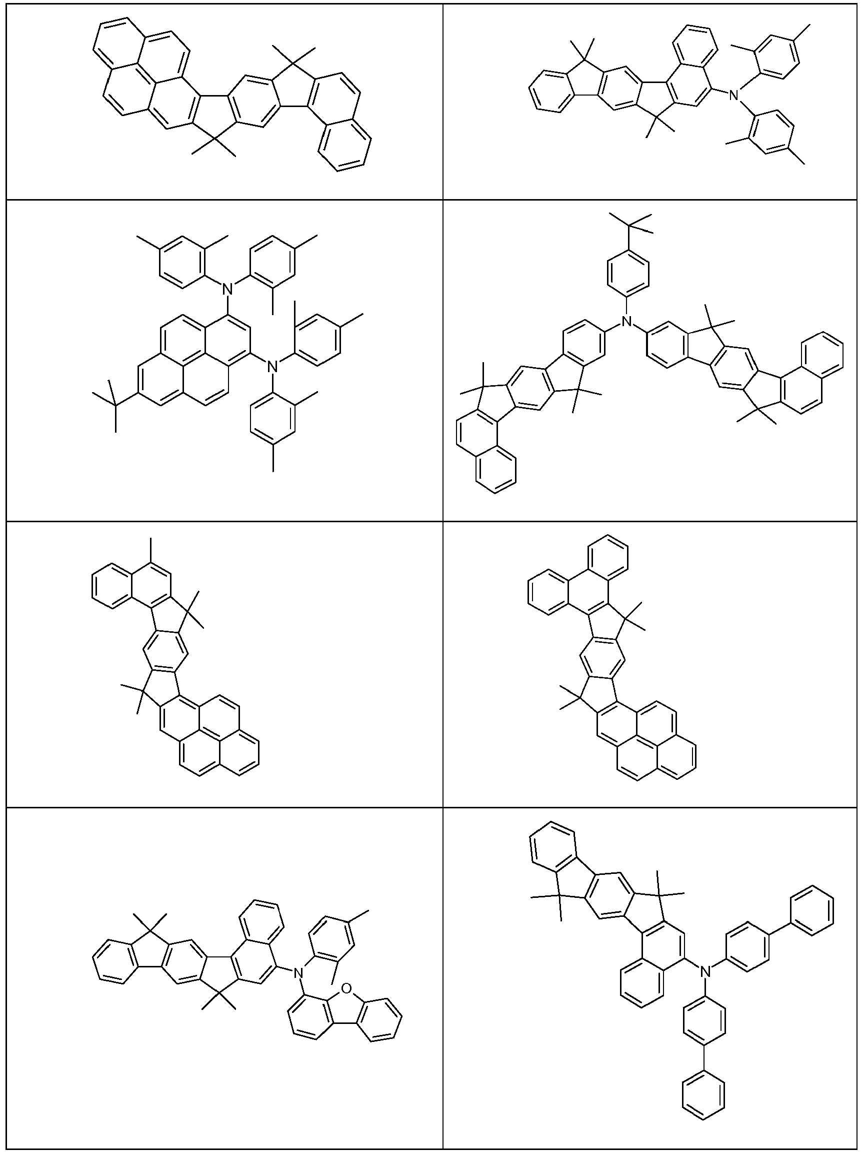

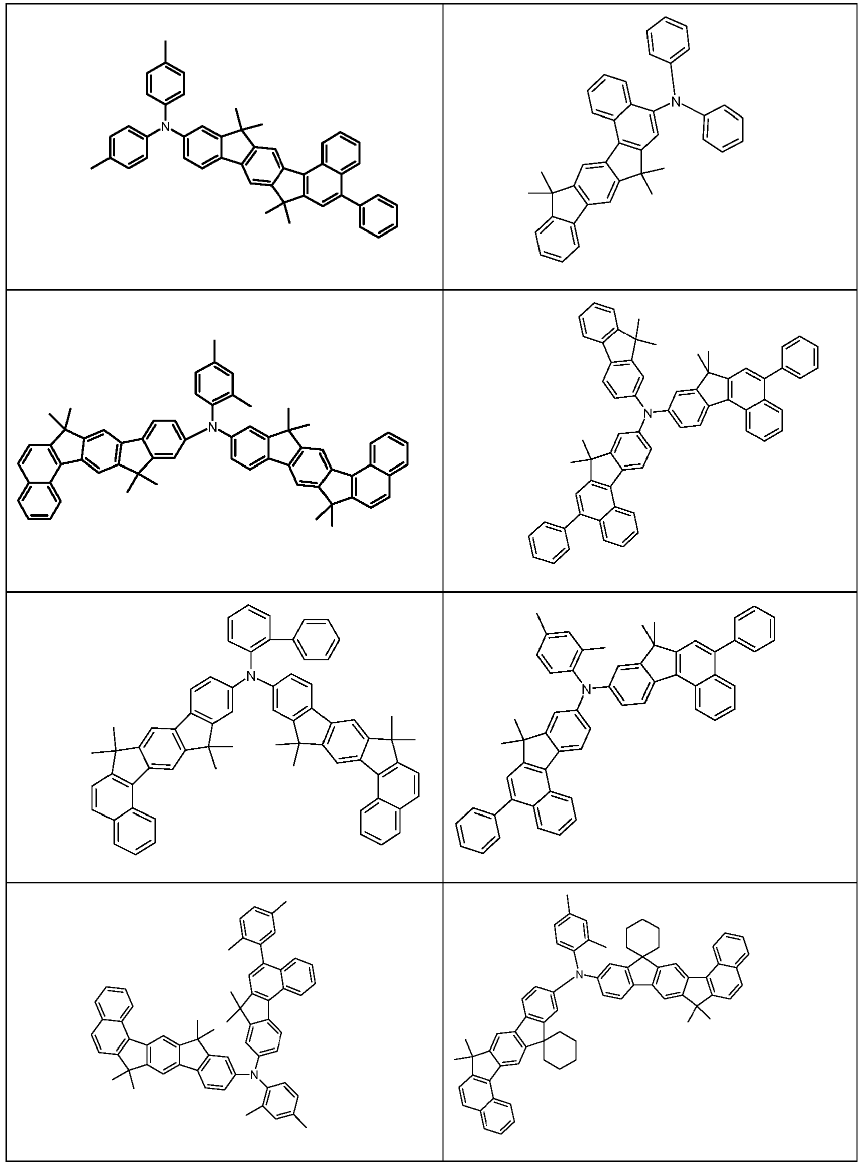

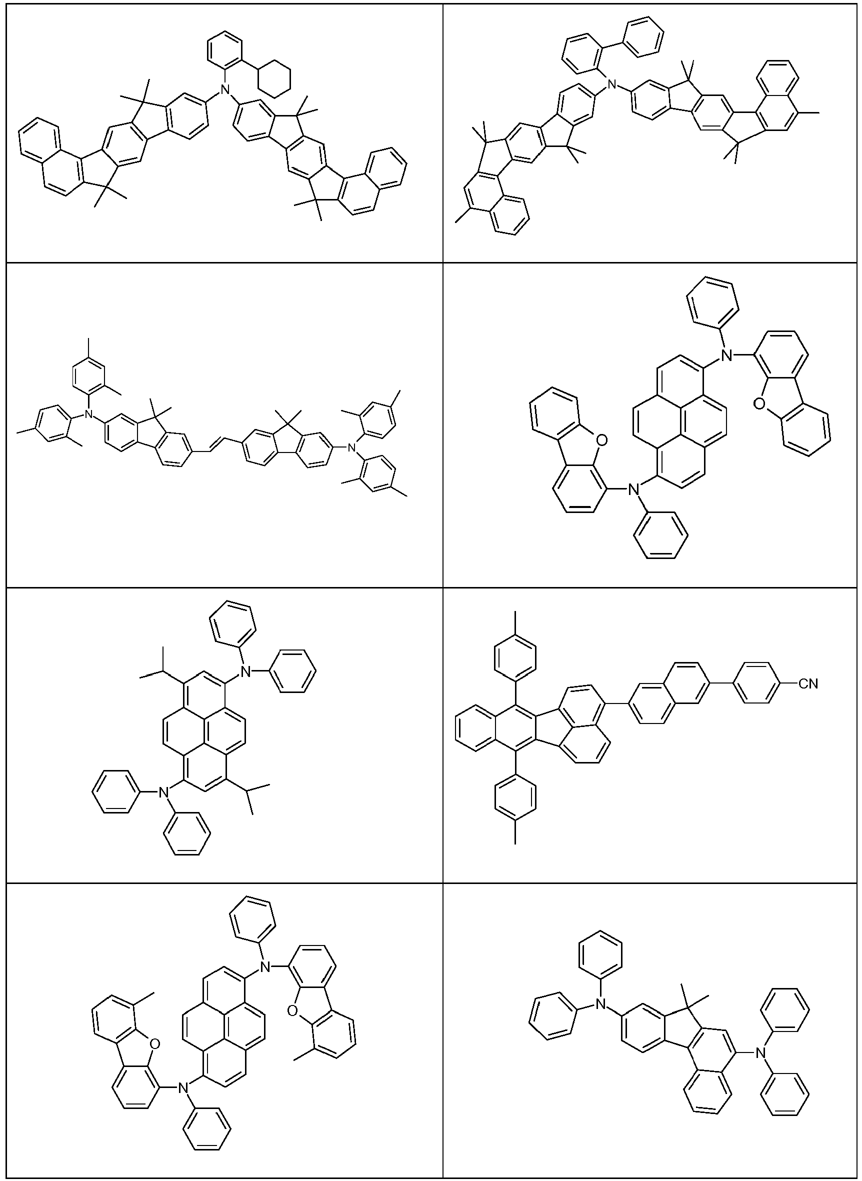

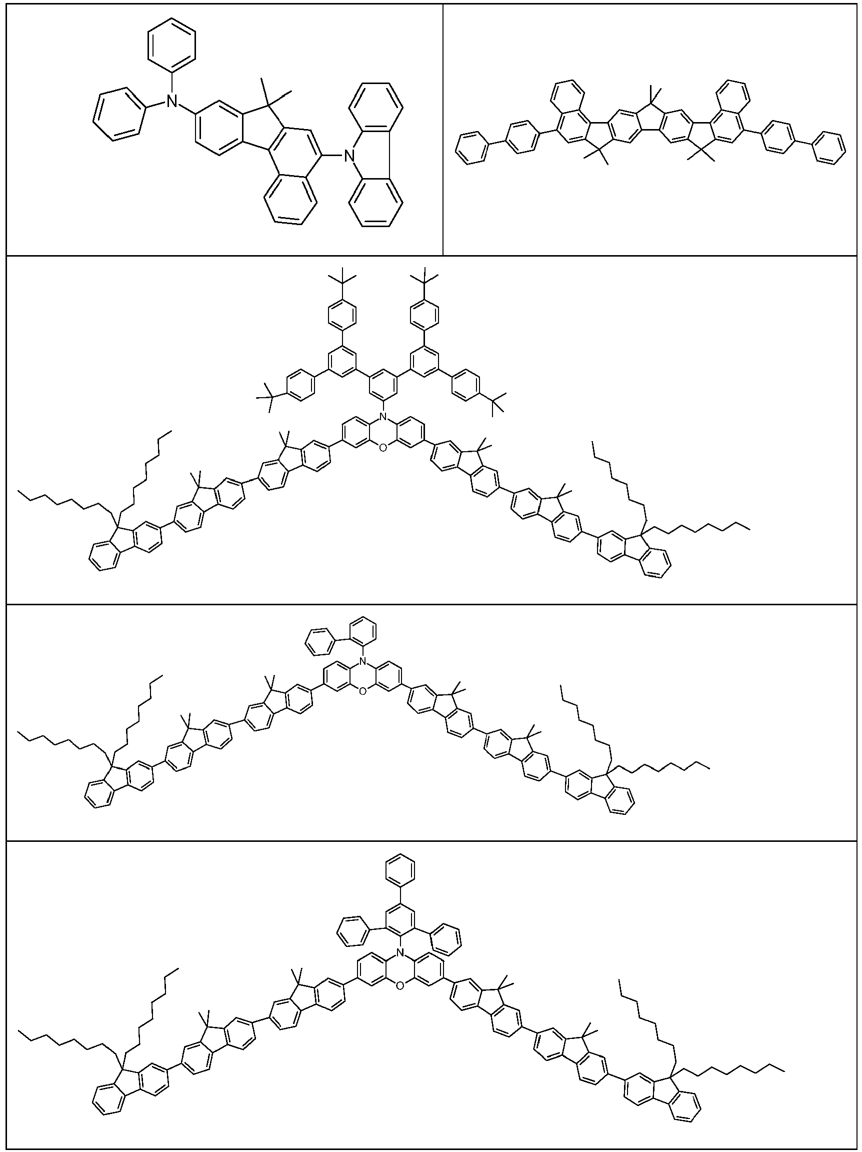

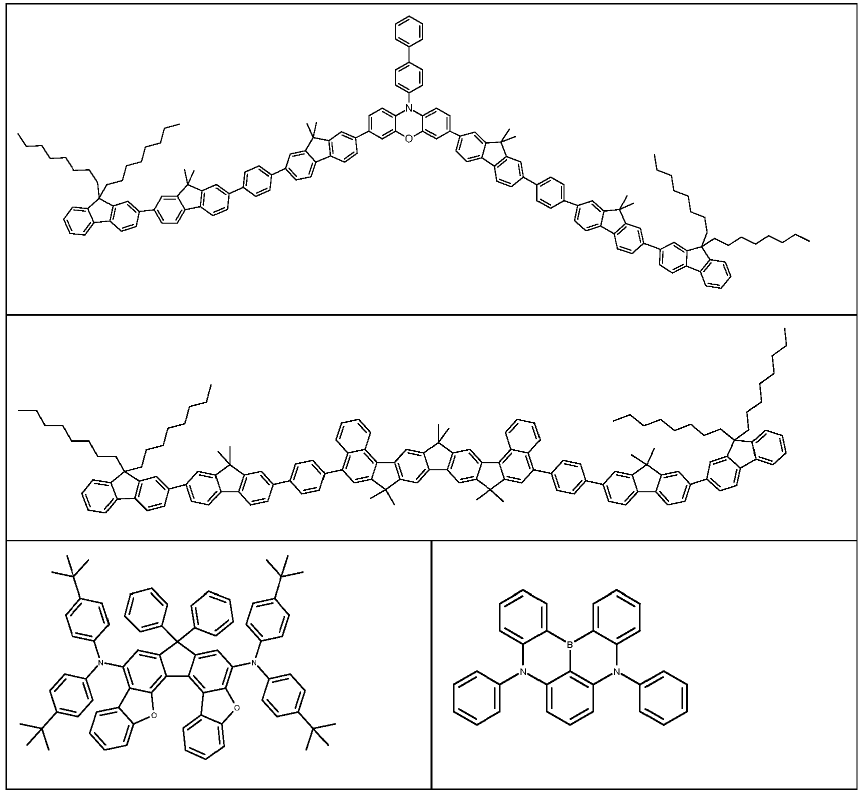

The compounds according to the invention can also be employed in other layers, for example as hole-transport materials in a hole-injection or hole-transport layer or electron-blocking layer or as matrix materials in an emitting layer, preferably as matrix materials for phosphorescent emitters.

If the compound of the formula (I) is employed as hole-transport material in a hole-transport layer, a hole-injection layer or an electron-blocking layer, the compound can be employed as pure material, i.e. in a proportion of 100%, in the hole-transport layer, or it can be employed in combination with one or more further compounds. According to a preferred embodiment, the organic layer comprising the compound of the formula (I) then additionally comprises one or more p-dopants. The p-dopants employed in accordance with the present invention are preferably organic electron-acceptor compounds which are able to oxidise one or more of the other compounds of the mixture.

Particularly preferred embodiments of p-dopants are the compounds disclosed in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 and WO 2012/095143.

If the compound of the formula (I) is employed as matrix material in combination with a phosphorescent emitter in an emitting layer, the phosphorescent emitter is preferably selected from the classes and embodiments of phosphorescent emitters indicated below. Furthermore, one or more further matrix materials are preferably present in the emitting layer in this case.

So-called mixed-matrix systems of this type preferably comprise two or three different matrix materials, particularly preferably two different matrix materials. It is preferred here for one of the two materials to be a material having hole-transporting properties and for the other material to be a material having electron-transporting properties. The compound of the formula (I) is preferably the material having hole-transporting properties.

However, the desired electron-transporting and hole-transporting properties of the mixed-matrix components may also be combined mainly or completely in a single mixed-matrix component, where the further mixed-matrix component or components satisfy other functions. The two different matrix materials may be present here in a ratio of 1:50 to 1:1, preferably 1:20 to 1:1, particularly preferably 1:10 to 1:1 and very particularly preferably 1:4 to 1:1. Mixed-matrix systems are preferably employed in phosphorescent organic electroluminescent devices. Further details on mixed-matrix systems are contained, inter alia, in the application WO 2010/108579.

Particularly suitable matrix materials which can be used as matrix components of a mixed-matrix system in combination with the compounds according to the invention are selected from the preferred matrix materials for phosphorescent emitters indicated below or the preferred matrix materials for fluorescent emitters, depending on what type of emitter compound is employed in the mixed-matrix system.

Generally preferred classes of material for use as corresponding functional materials in the organic electroluminescent devices according to the invention are indicated below.

Suitable phosphorescent emitters are, in particular, compounds which emit light, preferably in the visible region, on suitable excitation and in addition contain at least one atom having an atomic number greater than 20, preferably greater than 38 and less than 84, particularly preferably greater than 56 and less than 80. The phosphorescent emitters used are preferably compounds which contain copper, molybdenum, tungsten, rhenium, ruthenium, osmium, rhodium, iridium, palladium, platinum, silver, gold or europium, in particular compounds which contain iridium, platinum or copper.

For the purposes of the present invention, all luminescent iridium, platinum or copper complexes are regarded as phosphorescent compounds.

Examples of the phosphorescent emitters described above are revealed by the applications WO 2000/70655, WO 2001/41512, WO 2002/02714, WO 2002/15645, EP 1191613, EP 1191612, EP 1191614, WO 2005/033244, WO 2005/019373 and US 2005/0258742. In general, all phosphorescent complexes as used in accordance with the prior art for phosphorescent OLEDs and as are known to the person skilled in the art in the area of organic electroluminescent devices are suitable for use in the devices according to the invention. The person skilled in the art will also be able to employ further phosphorescent complexes without inventive step in combination with the compounds according to the invention in OLEDs.

Preferred matrix materials for phosphorescent emitters are aromatic ketones, aromatic phosphine oxides or aromatic sulfoxides or sulfones, for example in accordance with WO 2004/013080, WO 2004/093207, WO 2006/005627 or WO 2010/006680, triarylamines, carbazole derivatives, for example CBP (N,N-biscarbazolylbiphenyl) or the carbazole derivatives disclosed in WO 2005/039246, US 2005/0069729, JP 2004/288381, EP 1205527 or WO 2008/086851, indolocarbazole derivatives, for example in accordance with WO 2007/063754 or WO 2008/056746, indenocarbazole derivatives, for example in accordance with WO 2010/136109, WO 2011/000455 or WO 2013/041176, azacarbazole derivatives, for example in accordance with EP 1617710, EP 1617711, EP 1731584, JP 2005/347160, bipolar matrix materials, for example in accordance with WO 2007/137725, silanes, for example in accordance with WO 2005/111172, azaboroles or boronic esters, for example in accordance with WO 2006/117052, triazine derivatives, for example in accordance with WO 2010/015306, WO 2007/063754 or WO 2008/056746, zinc complexes, for example in accordance with EP 652273 or WO 2009/062578, diazasilole or tetraazasilole derivatives, for example in accordance with WO 2010/054729, diazaphosphole derivatives, for example in accordance with WO 2010/054730, bridged carbazole derivatives, for example in accordance with US 2009/0136779, WO 2010/050778, WO 2011/042107, WO 2011/088877 or WO 2012/143080, triphenylene derivatives, for example in accordance with WO 2012/048781, or lactams, for example in accordance with WO 2011/116865 or WO 2011/137951.

Besides the compounds according to the invention, suitable charge-transport materials, as can be used in the hole-injection or hole-transport layer or electron-blocking layer or in the electron-transport layer of the electronic device according to the invention, are, for example, the compounds disclosed in Y. Shirota et al., Chem. Rev. 2007, 107(4), 953-1010, or other materials as are employed in these layers in accordance with the prior art.

Materials which can be used for the electron-transport layer are all materials as are used in accordance with the prior art as electron-transport materials in the electron-transport layer. Particularly suitable are aluminium complexes, for example Alq₃, zirconium complexes, for example Zrq₄, lithium complexes, for example Liq, benzimidazole derivatives, triazine derivatives, pyrimidine derivatives, pyridine derivatives, pyrazine derivatives, quinoxaline derivatives, quinoline derivatives, oxadiazole derivatives, aromatic ketones, lactams, boranes, diazaphosphole derivatives and phosphine oxide derivatives. Furthermore suitable materials are derivatives of the above-mentioned compounds, as disclosed in JP 2000/053957, WO 2003/060956, WO 2004/028217, WO 2004/080975 and WO 2010/072300.

Preferred hole-transport materials which can be used in a hole-transport, hole-injection or electron-blocking layer in the electroluminescent device according to the invention are indenofluorenamine derivatives (for example in accordance with WO 06/122630 or WO 06/100896), the amine derivatives disclosed in EP 1661888, hexaazatriphenylene derivatives (for example in accordance with WO 01/049806), amine derivatives containing condensed aromatic rings (for example in accordance with US 5,061,569), the amine derivatives disclosed in WO 95/09147, monobenzoindenofluorenamines (for example in accordance with WO 08/006449), dibenzoindenofluorenamines (for example in accordance with WO 07/140847), spirobifluorenamines (for example in accordance with WO 2012/034627 or WO 2013/120577), fluorenamines (for example in accordance with the as applications EP 2875092, EP 2875699 and EP 2875004), spirodibenzopyranamines (for example in accordance with WO 2013/083216) and dihydroacridine derivatives (for example in accordance with WO 2012/150001). The compounds according to the invention can also be used as hole-transport materials.

The cathode of the organic electroluminescent device preferably comprises metals having a low work function, metal alloys or multilayered structures comprising various metals, such as, for example, alkaline-earth metals, alkali metals, main-group metals or lanthanoids (for example Ca, Ba, Mg, Al, In, Mg, Yb, Sm, etc.). Also suitable are alloys comprising an alkali metal or alkaline-earth metal and silver, for example an alloy comprising magnesium and silver. In the case of multilayered structures, further metals which have a relatively high work function, such as, for example, Ag or Al, can also be used in addition to the said metals, in which case combinations of the metals, such as, for example, Ca/Ag, Mg/Ag or Ag/Ag, are generally used. It may also be preferred to introduce a thin interlayer of a material having a high dielectric constant between a metallic cathode and the organic semiconductor. Suitable for this purpose are, for example, alkali metal fluorides or alkaline-earth metal fluorides, but also the corresponding oxides or carbonates (for example LiF, Li₂O, BaF₂, MgO, NaF, CsF, Cs₂CO₃, etc.). Furthermore, lithium quinolinate (LiQ) can be used for this purpose. The layer thickness of this layer is preferably between 0.5 and 5 nm.

The anode preferably comprises materials having a high work function. The anode preferably has a work function of greater than 4.5 eV vs. vacuum. Suitable for this purpose are on the one hand metals having a high redox potential, such as, for example, Ag, Pt or Au. On the other hand, metal/metal oxide electrodes (for example Al/Ni/NiOₓ, Al/PtOₓ) may also be preferred. For some applications, at least one of the electrodes must be transparent or partially transparent in order to facilitate either irradiation of the organic material (organic solar cells) or the coupling-out of light (OLEDs, O-lasers). Preferred anode materials here are conductive mixed metal oxides. Particular preference is given to indium tin oxide (ITO) or indium zinc oxide (IZO). Preference is furthermore given to conductive, doped organic materials, in particular conductive doped polymers.

The device is appropriately (depending on the application) structured, provided with contacts and finally sealed, since the lifetime of the devices according to the invention is shortened in the presence of water and/or air.

In a preferred embodiment, the organic electroluminescent device according to the invention is characterised in that one or more layers are coated by means of a sublimation process, in which the materials are applied by vapour deposition in vacuum sublimation units at an initial pressure of less than 10⁻⁵ mbar, preferably less than 10⁻⁶ mbar. However, it is also possible here for the initial pressure to be even lower, for example less than 10⁻⁷ mbar.

Preference is likewise given to an organic electroluminescent device, characterised in that one or more layers are coated by means of the OVPD (organic vapour phase deposition) process or with the aid of carrier-gas sublimation, in which the materials are applied at a pressure of between 10⁻⁵ mbar and 1 bar. A special case of this process is the OVJP (organic vapour jet printing) process, in which the materials are applied directly through a nozzle and are thus structured (for example M. S. Arnold et al., Appl. Phys. Lett. 2008, 92, 053301).

Preference is furthermore given to an organic electroluminescent device, characterised in that one or more layers are produced from solution, such as, for example, by spin coating, or by means of any desired printing process, such as, for example, screen printing, flexographic printing, nozzle printing or offset printing, but particularly preferably LITI (light induced thermal imaging, thermal transfer printing) or ink-jet printing. Soluble compounds of the formula (I) are necessary for this purpose. High solubility can be achieved through suitable substitution of the compounds.

Also possible are hybrid processes, in which, for example, one or more layers are applied from solution and one or more further layers are applied by vapour deposition. Thus, it is possible, for example, to apply the emitting layer from solution and to apply the electron-transport layer by vapour deposition. These processes are generally known to the person skilled in the art and can be applied by him without inventive step to organic electroluminescent devices comprising the compounds according to the invention.

In accordance with the invention, the electronic devices comprising one or more compounds according to the invention can be employed in displays, as light sources in lighting applications and as light sources in medical and/or cosmetic applications (for example light therapy).

The invention will now be explained in greater detail by the following examples, without wishing to restrict it thereby.

### A) Syntheses Examples

### A-1) Part 1

### Synthesis building block BB-I:

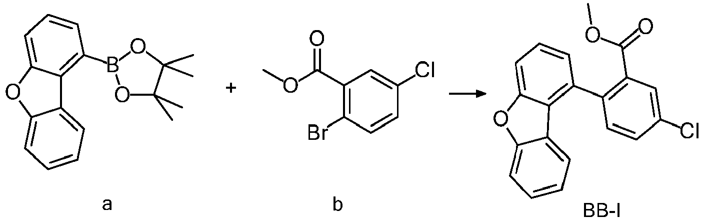

117.9 g (401 mmol) starting material a, 100 g (401 mmol) starting material b and 203.1 g (882 mmol) potassium phosphate monohydrate are mixed in 1.6 L toluene/water/dioxane (2:1:1) and degassed. To the mixture, palladium acetate (0.9 g, 4 mmol) and tri-*ortho*-tolylphosphine (2.44 g, 8 mmol) are added and the mixture is stirred at reflux for 16 h. After cooling the mixture to room temperature, the phases are separated. The aqueous phase is further extracted with ethyl acetate (2 x 300 mL). The combined organic phases are washed multiple times with water, dried over sodium sulfate and finally removed in vacuum. The crude is filtered over a plug of SiO₂/Al₂O₃ using ethyl acetate as solvent. After removing the solvent in vacuum, an oil is obtained in quantitative yield.

The following compounds can be synthesized in an analogous manner:

| Compound | Starting material | Starting material | Product |
|---|---|---|---|
| BB-I.a | 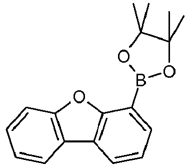 CAS 912824-85-2 | 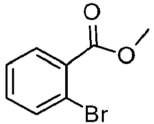 | 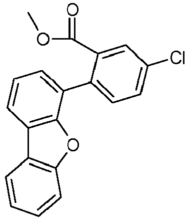 |

### Synthesis BB-II:

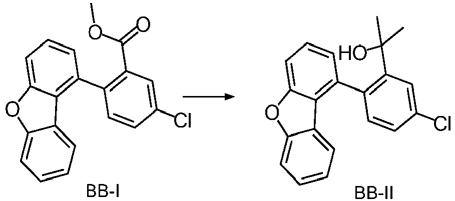

MeMgCl (461 mL, 3 M in THF, 1.38 mol) is added dropwise to a pre-cooled THF suspension (0 °C, 1.5 L) of compound BB-I (135 g, 0.4 mol) and CeCl₃ (199 g, 0.8 mol). After completion of the reaction, a saturated aqueous solution of NH₄Cl is added to quench the excess of MeMgCl, and the organic phase is extracted three times with ethyl acetate. The organic fractions are combined and washed with water and brine, successively. The volatiles were removed in vacuum to yield the desired product. 129 g (96 %).

The following compounds can be synthesized in an analogous manner:

| Compound | Starting material | Product |
|---|---|---|
| BB-ll.a | 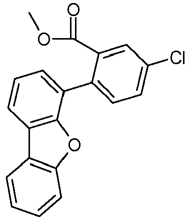 | 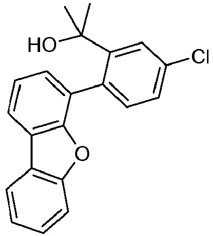 |

### Synthesis BB-III:

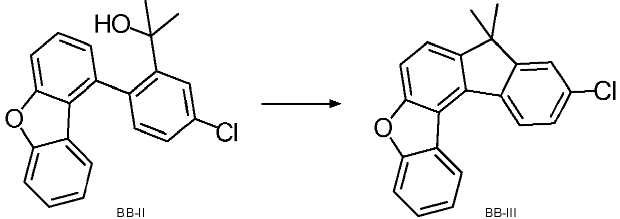

To a solution of compound BB-II (129 g, 383 mmol) in toluene (1 L), 50 g of Amberlyst-15 are added. The mixture is stirred at reflux overnight. The mixture is cooled down to room temperature and the Amberlyst-15 filtered off. The solvent is removed in vacuum and the crude product is purified by column chromatography (SiO₂, heptane). Yield: 106.2 g (87 %).

The following compounds can be synthesized in an analogous manner:

| Compound | Starting material | Product |
|---|---|---|
| BB-III.a | 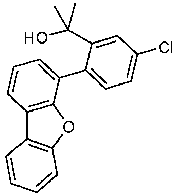 | 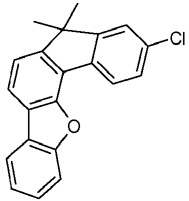 |

### Synthesis BB-IV:

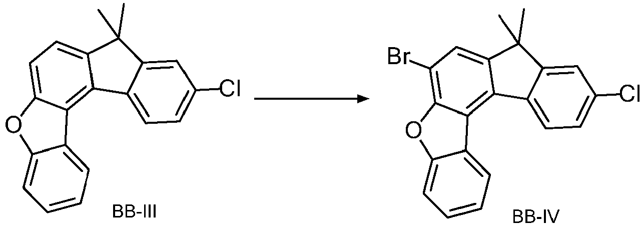

To a solution of compound BB-III (100 g, 314 mmol) in CH₂Cl₂ (1.2 L), N-bromosuccinimide (55.83 g, 314 mmol) and HBr (32 % solution in acetic acid, 0.5 mL) are added. The reaction is heated at 30 °C for 4 days. After completion of the reaction, Na₂S₂O₃ (300 mL, saturated aqueous solution) is added and the mixture is stirred vigorously for 30 minutes. The phases are separated and the organic phase is washed several times with water. The solvent is removed in vacuum and the crude product vigorously stirred with ethanol to yield a white solid. Yield: 119.8 g (96%).

The following compounds can be synthesized in an analogous manner:

| Compound | Starting material | Product |
|---|---|---|
| BB-IV.a | 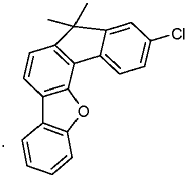 | 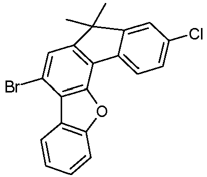 |

### Synthesis Intermediate BB-V:

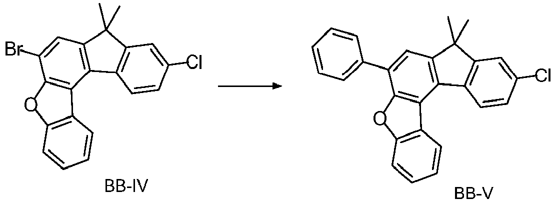

30.0 g (75.4 mmol) BB-IV, 9.2 g (75.4 mmol) phenylboronic acid and 16.0 g (151 mmol) sodium carbonate are mixed in 600 mL toluene/dioxane/water (2:1:2) and degassed. To the mixture, Tetrakis(triphenylphosphine)palladium (2.2 g, 1.9 mmol) is added and the mixture is stirred at reflux for 4 h. After cooling the mixture to room temperature, 400 mL of ethyl acetate is added and the phases are separated. The organic phase is washed multiple times with water and the solvent is removed in vacuum. Afterwards, the organic phase is filtrated over a plug of silica using ethyl acetate as solvent. The solvent is removed in vacuum and the crude product vigorously stirred with ethanol to yield a white solid. Yield: 28.3 g (95%).

The following compounds can be synthesized in an analogous manner:

| Comp. | Starting material | Starting material | Product |
|---|---|---|---|
| BB-V.b | BB-IV.a | 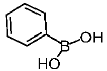 | 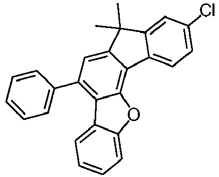 |
| BB-V.c | BB-IV | 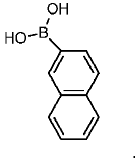 | 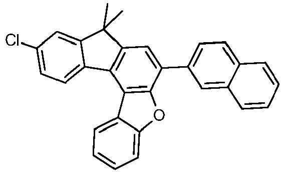 |
| BB-V.d | BB-IV.a | 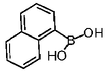 | 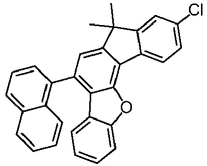 |
| BB-V.e | BB-IV | 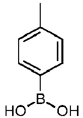 | 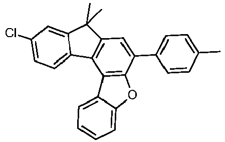 |
| BB-V.f | BB-IV | 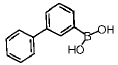 | 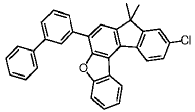 |
| BB-V.g | BB-IV | 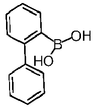 | 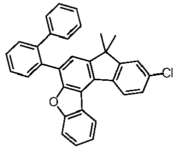 |
| BB-V.h | BB-IV | 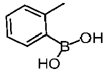 | 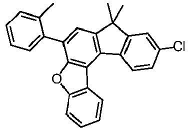 |
| BB-V.i | BB-IV | CAS 1010100-76-1 | 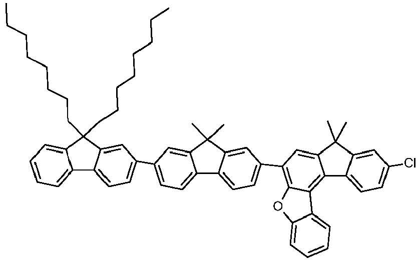 |
| BB-V.j | BB-IV.a | CAS 1010100-76-1 | 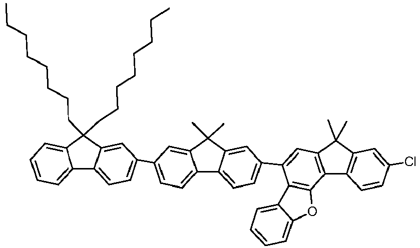 |

### A-2) Part 2:

### Scheme synthesis example Compound 1.1

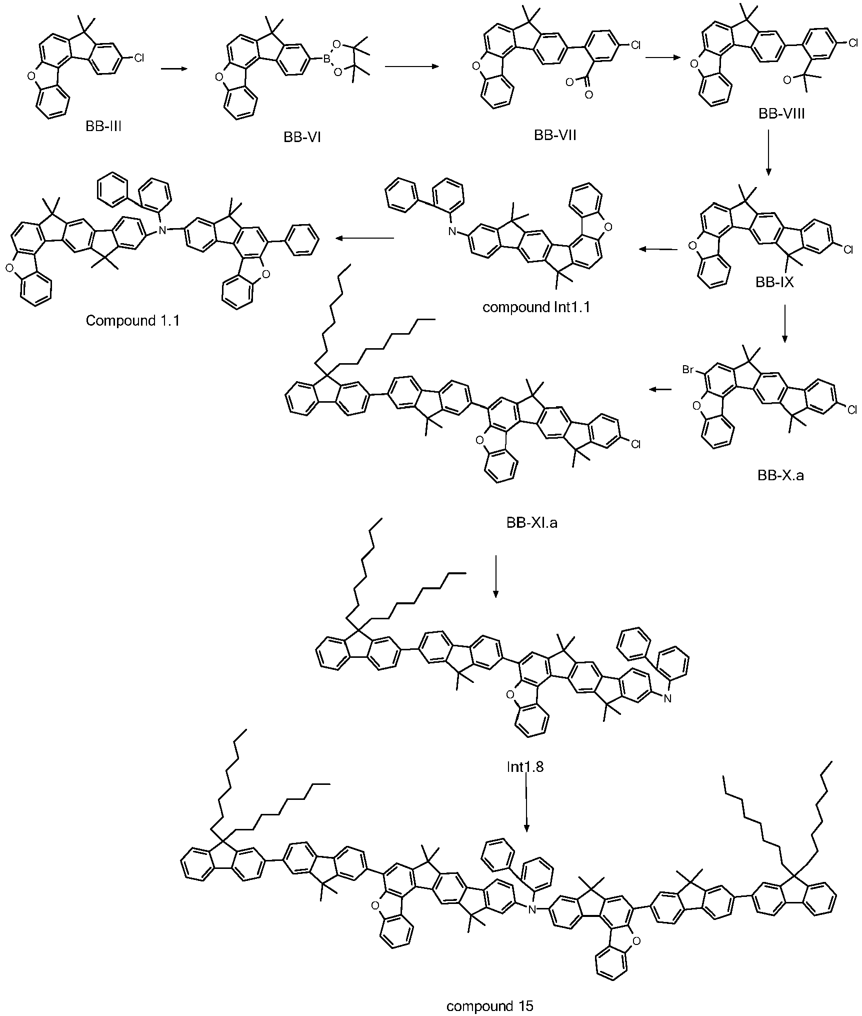

### Synthesis BB-VI:

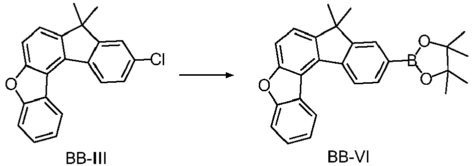

10-Chloro-8,8-dimethyl-8H-5-oxa-indeno[2,1-c]fluorene (30.00 g; 94.1 mmol), bis-(pinacolato)-diboron (28.68 g; 112.9 mmol) and potassium acetate (18.47 g; 188.2 mmol) are dissolved in 800 mL 1,4-dioxane. XPhos Palladacycle Gen 3 (CAS:1445085-55-1; 1.59 g; 1.882 mmol) and bis-(pinacolato)-diboron (28.68 g; 112.9 mmol) are added and the reaction mixture is stirred at 100 °C overnight. After complete conversion, the reaction mixture is cooled down to room temperature and water and toluene are added. The phases are separated and the organic phase is washed several times with water. The combined organic phases are filtrated over silica with toluene as eluent. The solvent is removed in vacuum and the crude product vigorously stirred with ethanol to yield a white solid.

Yield: 34.2 g (83.4 mmol; 88%)

The following compound can be synthesized in an analogous manner:

| Compound | Starting material | Product |
|---|---|---|
| BB-Vl.a | 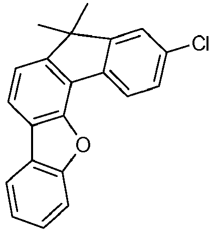 | 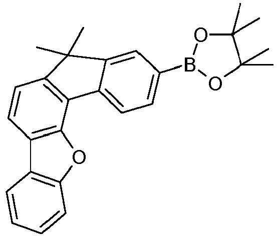 |

### Synthesis of BB-VII:

### Synthesis of BB-VII is done analog to BB-I:

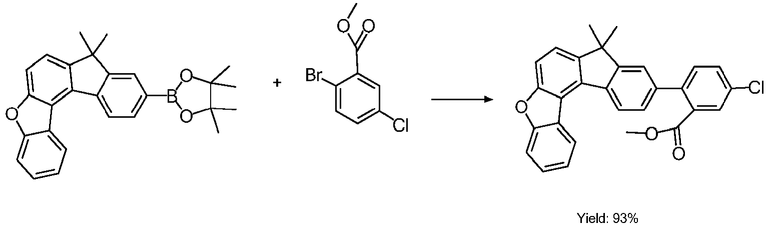

The following compound can be synthesized in an analogous manner:

| Compound | Starting material | Product |
|---|---|---|
| BB-Vll.a | 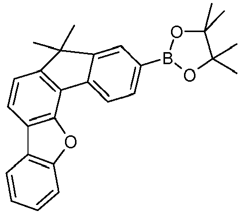 | 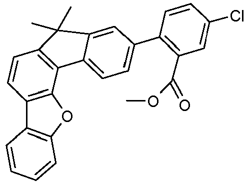 |

### Synthesis of BB-VIII:

### Synthesis of BB-VIII is done analog to BB-II:

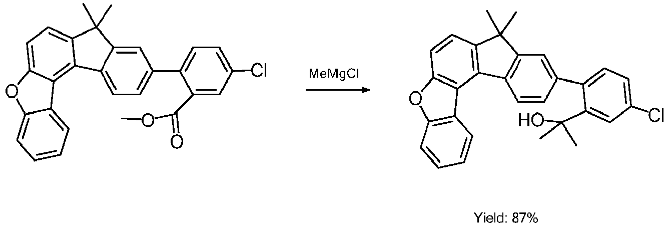

The following compound can be synthesized in an analogous manner:

| Compound | Starting material | Product |
|---|---|---|
| BB-VIII.a | 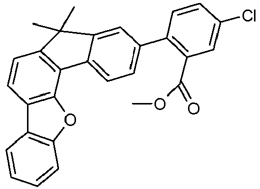 | 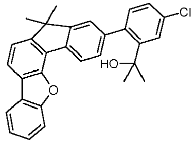 |

### Synthesis of BB-IX:

### Synthesis of BB-IX is done analog to BB-III:

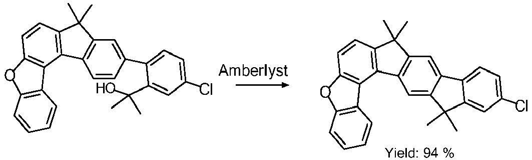

The following compound can be synthesized in an analogous manner:

| Compound | Starting material | Product |
|---|---|---|
| BB-IX.a | 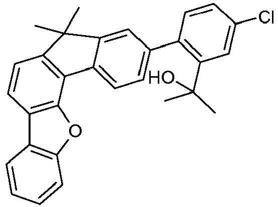 | 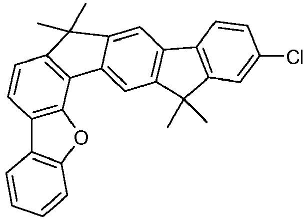 |

### Synthesis of Intermediate BB-X.a and BB-X.b

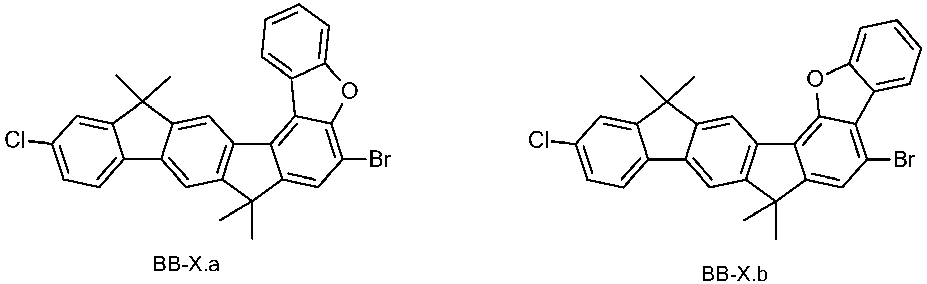

Intermediate BB-X.a and BB-X.b can be synthesized in analogous manner like BB-IV.

### Synthesis of Intermediate BB-XI.a BB-XI.b

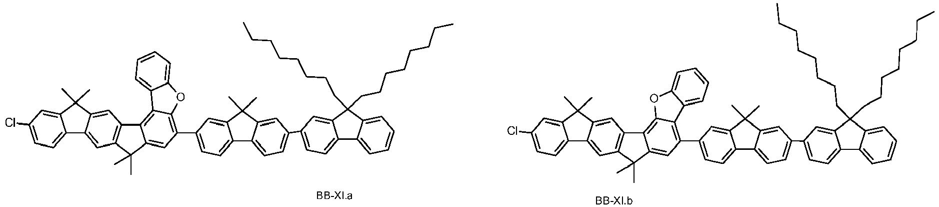

BB-XI.a and BB-XI.b can be synthesized in analogous manner like BB-V by using CAS 1010100-76-1 as boronate ester starting material.

### Synthesis of compound Int1.1:

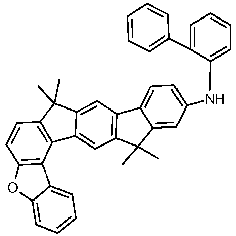

4.41 g (26.0 mmol) biphenyl-2-ylamine, 11.31 g (26.0 mmol) BB-IX and 6.82 g (70.9 mmol) sodium tertbutylate are mixed in 300 mL toluene and degassed. Afterwards, 563 mg (1.4 mmol) S-Phos and 151 mg (0.7 mmol) palladium acetate are added and the mixture is stirred at reflux for 16 h. After cooling the mixture at room temperature, 200 mL of water is added and the phases are separated. The crude product is filtrate over a plug of aluminium oxide using toluene as solvent. The product is further purified by recrystallizations from toluene/heptane. Yield: 13.1 g (89 %).

The following compounds can be synthesized in an analogous manner:

| Comp. | SM | SM | Product |
|---|---|---|---|
| Int1.2 | BB-IX | 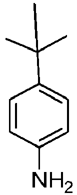 | 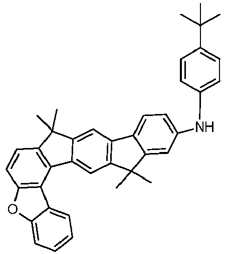 |
| Int1.3 | BB-IX | 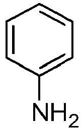 | 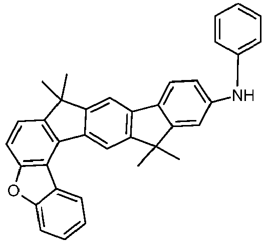 |
| Int1.4 | BB-IX | 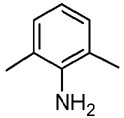 | 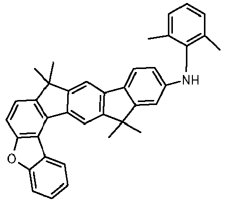 |
| Int1.5 | BB-IX.a | 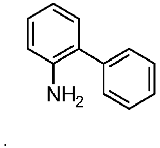 | 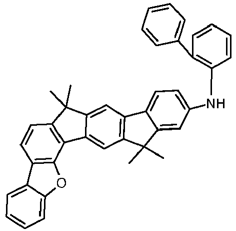 |
| Int1.6 | BB-IX | 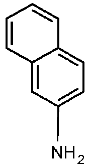 | 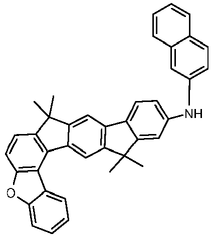 |

| | | | |
|---|---|---|---|
| Int1.7 | BB-IX | 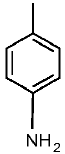 | 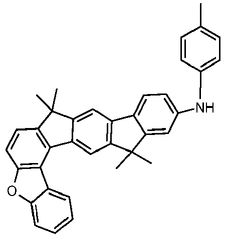 |
| Int1.8 | BB-XI.a | 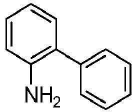 | 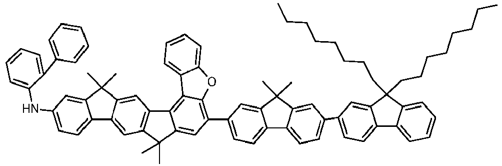 |
| Int1.9 | BB-XI.b | 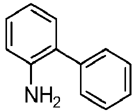 | 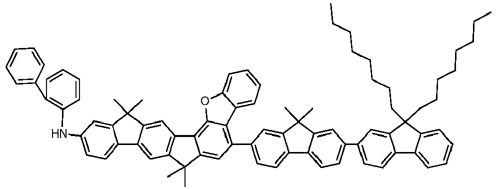 |

### A-3) Part 3

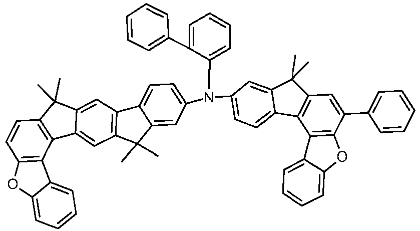

20.5 g (36.1 mmol) Int1.1, 14.3 g (36.1 mmol) BB-V and 20.9 g (217 mmol) sodium tertbutylate are mixed in 700 mL toluene and degassed. Afterwards, 1.7 g (4.1 mmol) S-Phos and 455 mg (2.0 mmol) palladium acetate are added and the mixture is stirred at reflux for 16 h. After cooling the mixture at room temperature, 200 mL of water is added and the phases are separated. The crude product is filtrate over a plug of aluminium oxide using toluene as solvent. The product is further purified by recrystallizations from toluene/heptane up to a purity of >99.9 % by HPLC. Yield: 12.4 g (37 %).

The following compounds can be synthesized in an analogous manner:

| **Comp.** | **Starting material** | **Amine** | **Product** |
|---|---|---|---|
| **1** | **BB-V** | **Int1.1** | 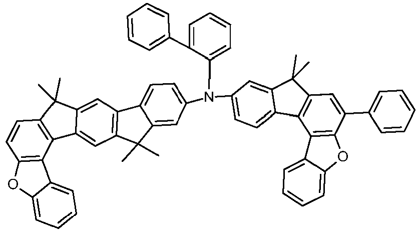 |
| **2** | **BB-V** | **Int1.2** | 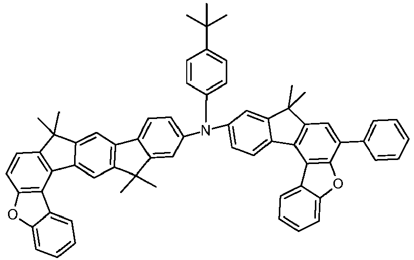 |
| **3** | **BB-V** | **Int1.3** | 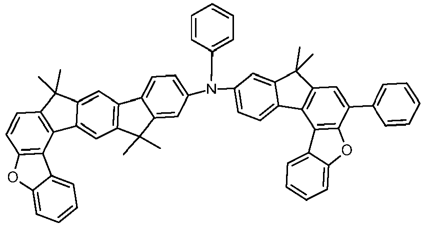 |
| **4** | **BB-V** | **Int1.4** | 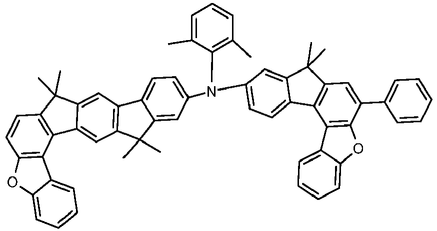 |
| **5** | **BB-V** | **Int1.5** | 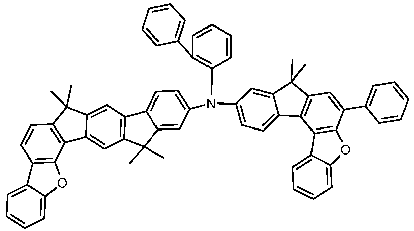 |
| **6** | **BB-V** | **Int1.6** | 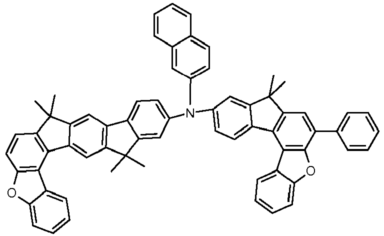 |
| **7** | **BB-V** | **Int1.7** | 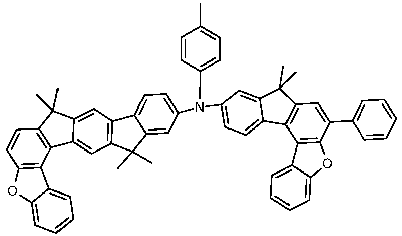 |
| **8** | **BB-V.b** | **Int1.1** | 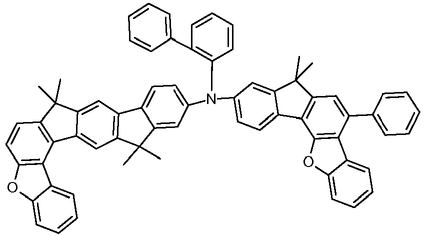 |
| **9** | **BB-V.c** | **Int1.1** | 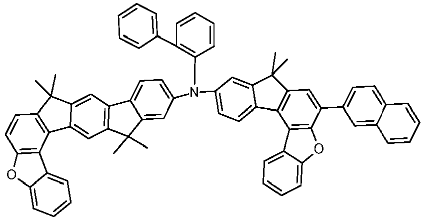 |
| **10** | **BB-V.d** | **Int1.1** | 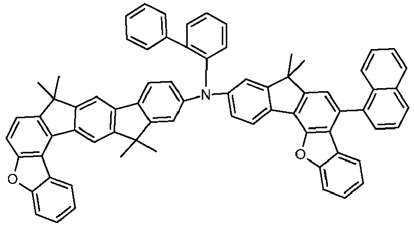 |
| **11** | **BB-V.e** | **Int1.1** | 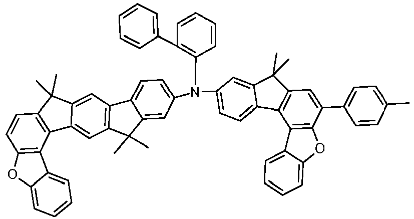 |
| **12** | **BB-V.f** | **Int1.1.** | 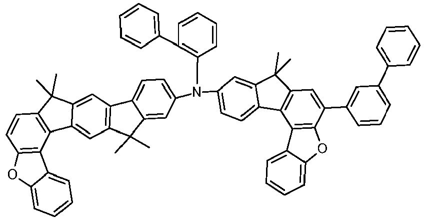 |
| **13** | **BB-V.g** | **Int1.1** | 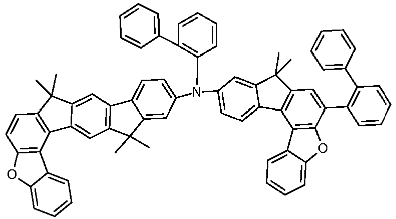 |
| **14** | **BB-V.h** | **Int1.1** | 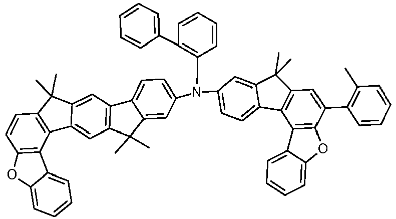 |
| **15** | **BB-V.j** | **Int1.8** | 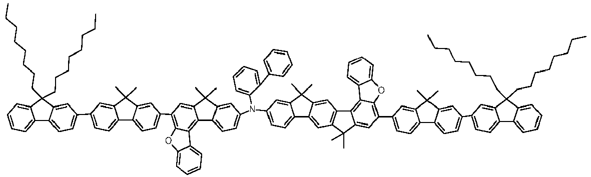 |
| **16** | **BB-V.j** | **Int1.9** | 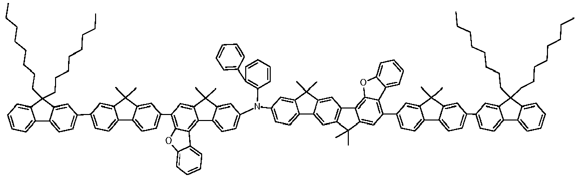 |

### B) Fabrication of OLEDs

The production of solution-based OLEDs has already been described many times in the literature, for example in WO 2004/037887 and WO 2010/097155. The process is adapted to the circumstances described below (layer-thickness variation, materials).

The inventive material combinations are used in the following layer sequence:
- substrate,
- ITO (50 nm),
- Buffer (40 nm),
- emission layer (EML) (40 nm),
- hole-blocking layer (HBL) (10 nm)
- electron-transport layer (ETL) (30 nm),
- cathode (Al) (100 nm).

Glass plates coated with structured ITO (indium tin oxide) in a thickness of 50 nm serve as substrate. These are coated with the buffer (PEDOT) Clevios P VP AI 4083 (Heraeus Clevios GmbH, Leverkusen). The spin coating of the buffer is carried out from water in air. The layer is subsequently dried by heating at 180°C for 10 minutes. The emission layers are applied to the glass plates coated in this way.

The emission layer (EML) is composed of the matrix material (host material) H and the emitting dopant (emitter) D. Both materials are present in the emission layer in a proportion of 97 % by weight H and 3 % by weight D. The mixture for the emission layer is dissolved in toluene. The solids content of such solutions is about 9 mg/ml if, as here, the layer thickness of 40 nm which is typical for a device is to be achieved by means of spin coating. The layers are applied by spin coating in an inert-gas atmosphere and dried by heating at 120°C for 10 minutes. The materials used in the present case are shown in Table A.

**Table A: Structural formulae of the solution processed materials in the EML**

| | |
|---|---|
| 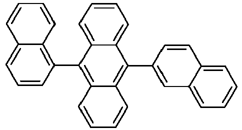 | 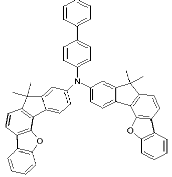 |
| H | SdT1 |
| 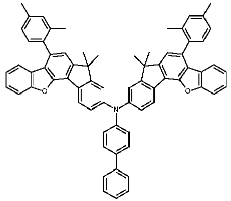 | 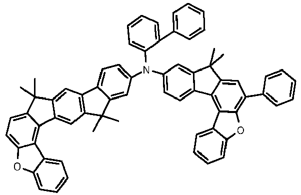 |
| SdT2 | D1 |
| 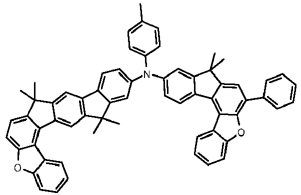 | 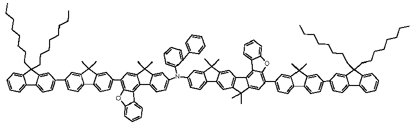 |
| D2 | D3 |

The materials for the hole-blocking layer and electron-transport layer are likewise applied by thermal vapour deposition in a vacuum chamber and are shown in table B. The hole-blocking layer (HBL) consists of ETM. The electron-transport layer (ETL) consists of the two materials ETM and LiQ, which are mixed with one another in a proportion by volume of 50% each by co-evaporation. The cathode is formed by the thermal evaporation of an aluminium layer with a thickness of 100 nm.

**Table B: Structural formulae of vapor processed OLED materials**

| | |
|---|---|
| 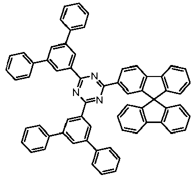 | 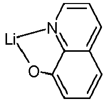 |
| ETM | LiQ |

The OLEDs are characterised by standard methods. For this purpose, the electroluminescence spectra are recorded, the current efficiency (measured in cd/A) and the external quantum efficiency (EQE, measured in percent) as a function of the luminous density assuming Lambert emission characteristics are calculated from current/voltage/luminous density characteristic lines (IUL characteristic lines). The electroluminescence spectra are recorded at a luminous density of 1000 cd/m², and the CIE 1931 x and y colour coordinates are calculated from this data. The term EQE1000 denotes the external quantum efficiency at an operating luminous density of 1000 cd/m².

The properties of the various OLEDs are summarised in table C. Example V1 and V2 are the comparative examples, whereas E3, E4 and E5 show properties of OLEDs containing materials of the present invention.

**Table C: Device data of solution processed OLEDs**

| Example | EML host | EML dopant | EQE1000 [%] | CIE x/y |
|---|---|---|---|---|
| V1 | H | SdT1 | 1.9 | 0.15 / 0.05 |
| V2 | H | SdT2 | 2.6 | 0.16 / 0.08 |
| E3 | H | D1 | 4.0 | 0.14/0.14 |
| E4 | H | D2 | 4.3 | 0.14/0.14 |
| E5 | H | D3 | 5.1 | 0.14/0.13 |

Table C shows that use of materials (D1, D2 and D3) according to the present invention give rise to improvements over the prior art (SdT1 and SdT2) when used as fluorescent blue emitters, in particular with respect to efficiency.

## Claims

1. Compound of the formula (1), 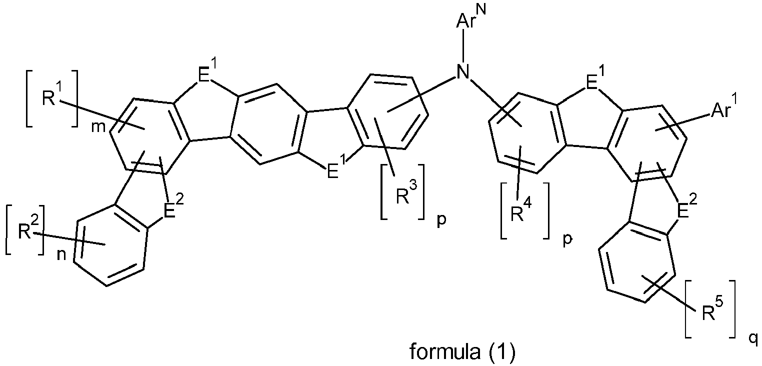 where the following applies to the symbols and indices used:
Ar¹, Ar^{N} are on each occurrence, identically or differently, selected from an aromatic or heteroaromatic ring system having 5 to 60 aromatic ring atoms, which may in each case be substituted by one or more radicals R;
E¹ is on each occurrence, identically or differently, selected from -BR⁰-, - C(R⁰)₂-, -C(R⁰)₂-C(R⁰)₂-, -C(R⁰)₂-O-, -C(R⁰)₂-S-, -R⁰C=CR⁰-, -R⁰C=N-, Si(R⁰)₂, -Si(R⁰)₂-Si(R⁰)₂-, -C(=O)-, -C(=NR⁰)-, -C(=C(R⁰)₂)-, -O-, -S-, - S(=O)-, -SO₂-, -N(R⁰)-, -P(R⁰)- and -P((=O)R⁰)-; or E¹ is a group of formula (E-1), 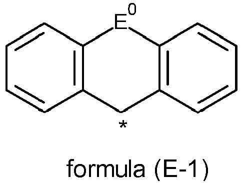 where the symbol * in formula (E-1) indicates the corresponding group E¹ in formula (1); and
E⁰ is identically or differently on each occurrence, selected from the group consisting of a single bond, -BR⁰-, -C(R⁰)₂-, -C(R⁰)₂-C(R⁰)₂-, - C(R⁰)₂-O-, -C(R⁰)₂-S-, -R⁰C=CR⁰-, -R⁰C=N-, Si(R⁰)₂, -Si(R⁰)₂-Si(R⁰)₂-, - C(=O)-, -C(=NR⁰)-, -C(=C(R⁰)₂)-, -O-, -S-, -S(=O)-, -SO₂-, -N(R⁰)-, -P(R⁰)- and -P((=O)R⁰)-;
E² is, identically or differently, on each occurrence, selected from the group consisting of -O-, -S-, -S(=O)- and -SO₂-;
R⁰ to R⁵ stand on each occurrence, identically or differently, for H, D, F, CI, Br, I, CHO, CN, N(Ar)₂, C(=O)Ar, P(=O)(Ar)₂, S(=O)Ar, S(=O)₂Ar, NO₂, Si(R)₃, B(OR)₂, OSO₂R, a straight-chain alkyl, alkoxy or thioalkyl group having 1 to 40 C atoms or branched or cyclic alkyl, alkoxy or thioalkyl group having 3 to 40 C atoms, each of which may be substituted by one or more radicals R, where in each case one or more non-adjacent CH₂ groups may be replaced by RC=CR, C≡C, Si(R)₂, Ge(R)₂, Sn(R)₂, C=O, C=S, C=Se, P(=O)(R), SO, SO₂, O, S or CONR and where one or more H atoms may be replaced by D, F, CI, Br, I, CN or NO₂, an aromatic or heteroaromatic ring system having 5 to 60 aromatic ring atoms, which may in each case be substituted by one or more radicals R, or an aryloxy group having 5 to 60 aromatic ring atoms, which may be substituted by one or more radicals R, where two adjacent substituents R⁰, and/or two adjacent substituents R¹, and/or two adjacent substituents R², and/or two adjacent substituents R³, and/or two adjacent substituents R⁴, and/or two adjacent substituents R⁵ may form a mono- or polycyclic, aliphatic ring system or aromatic ring system, which may be substituted by one or more radicals R;
m stands on each occurrence, identically or differently, for an integer selected from 0, 1 or 2;
n, q stand on each occurrence, identically or differently, for an integer selected from 0, 1, 2, 3 or 4;
p stands on each occurrence, identically or differently, for an integer selected from 0, 1, 2 or 3;
R stands on each occurrence, identically or differently, for H, D, F, Cl, Br, I, CHO, CN, N(Ar)₂, C(=O)Ar, P(=O)(Ar)₂, S(=O)Ar, S(=O)₂Ar, NO₂, Si(R')₃, B(OR')₂, OSO₂R', a straight-chain alkyl, alkoxy or thioalkyl group having 1 to 40 C atoms or branched or cyclic alkyl, alkoxy or thioalkyl group having 3 to 40 C atoms, each of which may be substituted by one or more radicals R', where in each case one or more non-adjacent CH₂ groups may be replaced by R'C=CR', C=C, Si(R')₂, Ge(R')₂, Sn(R')₂, C=O, C=S, C=Se, P(=O)(R'), SO, SO₂, O, S or CONR' and where one or more H atoms may be replaced by D, F, Cl, Br, I, CN or NO₂, an aromatic or heteroaromatic ring system having 5 to 60 aromatic ring atoms, which may in each case be substituted by one or more radicals R', or an aryloxy group having 5 to 60 aromatic ring atoms, which may be substituted by one or more radicals R', where two adjacent substituents R may form a mono- or polycyclic, aliphatic ring system or aromatic ring system, which may be substituted by one or more radicals R';
Ar is an aromatic or heteroaromatic ring system having 5 to 24 aromatic ring atoms, which may in each case also be substituted by one or more radicals R';
R' stands on each occurrence, identically or differently, for H, D, F, Cl, Br, I, CN, a straight-chain alkyl, alkoxy or thioalkyl group having 1 to 20 C atoms or branched or cyclic alkyl, alkoxy or thioalkyl group having 3 to 20 C atoms, where in each case one or more non-adjacent CH₂ groups may be replaced by SO, SO₂, O, S and where one or more H atoms may be replaced by D, F, Cl, Br or I, or an aromatic or heteroaromatic ring system having 5 to 24 C atoms.

2. Compound according to claim 1, **characterized in that** Ar^{N} stands for phenyl, biphenyl, fluorene, spirobifluorene, naphthalene, phenanthrene, dibenzofuran, dibenzothiophene, carbazole, pyridine, pyrimidine, pyrazine, pyridazine, triazine, benzopyridine, benzopyridazine, benzopyrimidine or quinazoline, or for a combination of two to six of these groups, each of which may be substituted by one or more radicals R.

3. Compound according to claim 1 or 2, **characterized in that** Ar^{N} stands for phenyl, biphenyl, fluorene, naphthalene, phenanthrene, dibenzofuran, dibenzothiophene, carbazole, or for a combination of two to six of these groups, each of which may be substituted by one or more radicals R.

4. Compound according to one or more of the preceding claims, **characterized in that** it is selected from selected from compounds of formula (2), 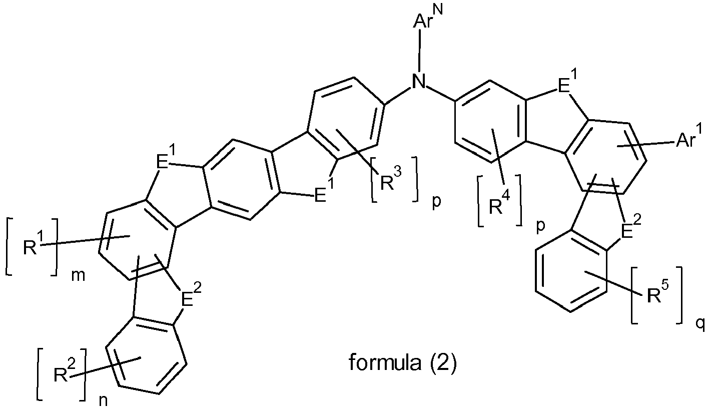 where the symbols Ar^{N}, E¹, E², Ar¹, R¹ to R⁵ and the indices m, n, p and q have the same meaning as in claim 1.

5. Compound according to one or more of the preceding claims, selected from compounds of formula (3), 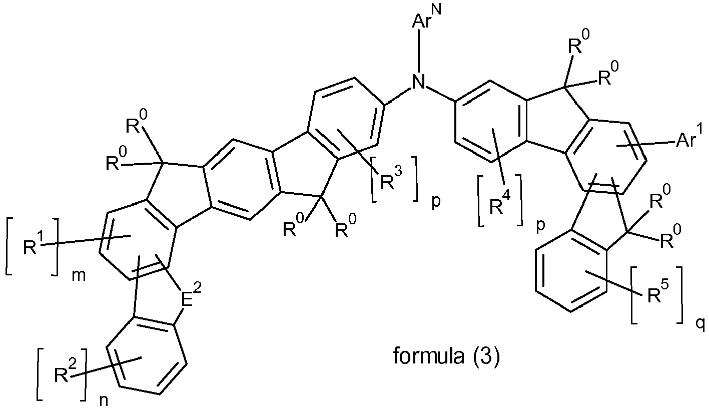 where the symbols Ar^{N}, E², Ar¹, R⁰, R¹ to R⁵ and the indices m, n, p and q have the same meaning as in claim 1.

6. Compound according to one or more of the preceding claims, selected from the compounds of formula (4), 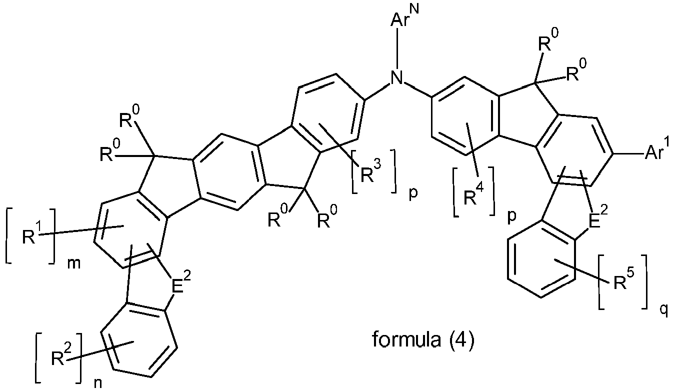 where the symbols Ar^{N}, E², Ar¹, R⁰, R¹ to R⁵ and the indices m, n, p and q have the same meaning as in claim 1.

7. Compound according to one or more of the preceding claims, **characterized in that** it is selected from the compounds of formula (4-1) to (4-4), 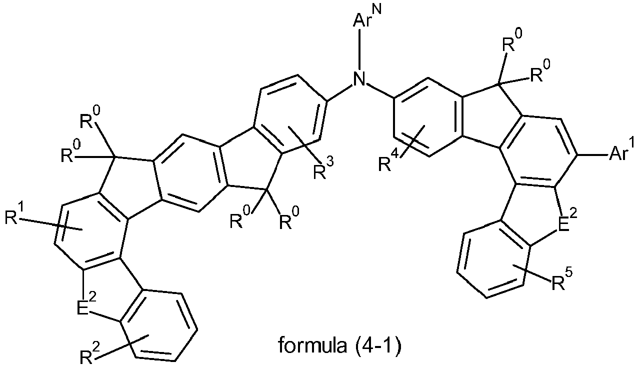 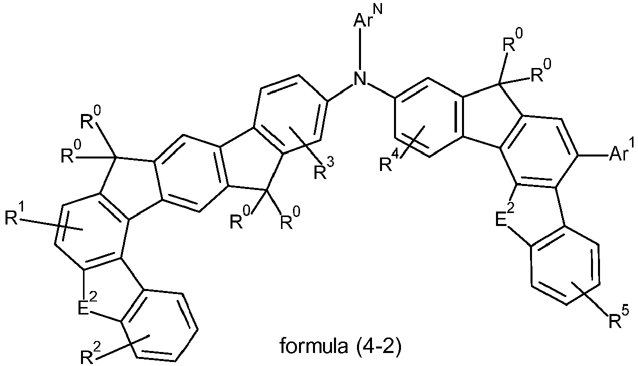 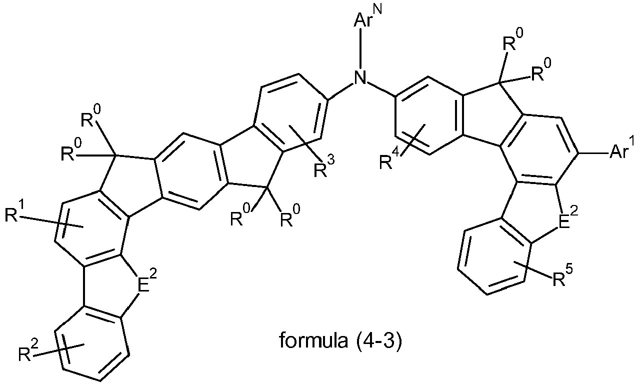 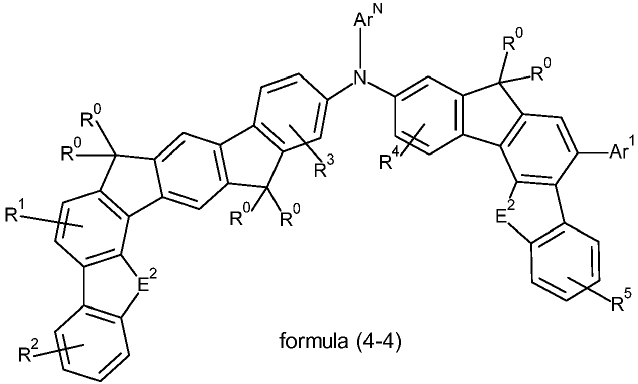 where the symbols Ar^{N}, E², Ar¹, R⁰, R¹ to R⁵ have the same meaning as in claim 1.

8. Compound according to one or more of the preceding claims, **characterized in that** it is selected from formulae (4-1a) to (4-4a), 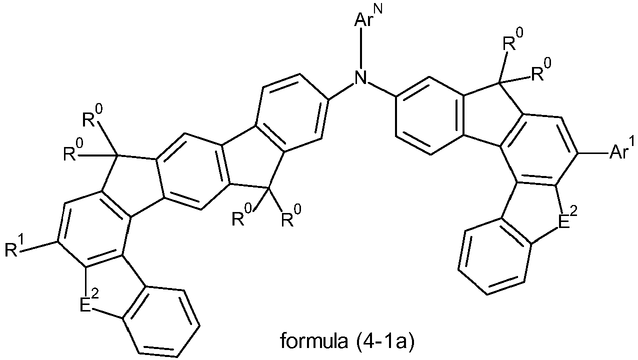 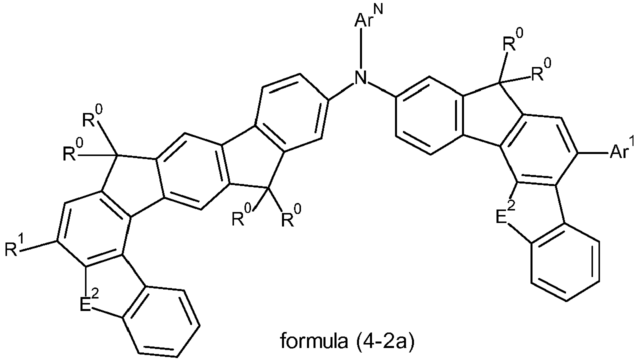 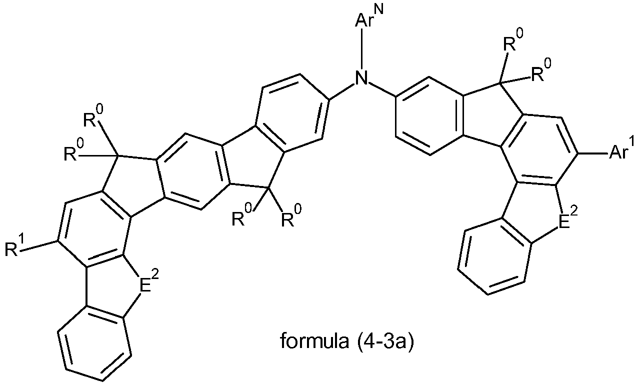 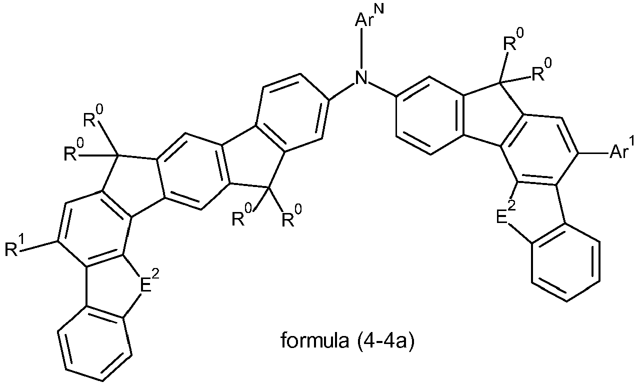 where the symbols Ar^{N}, E², Ar¹, R⁰ and R¹ have the same meaning as in claim 1.

9. Compound according to one or more of the preceding claims, **characterized in that** it is selected from formulae (4-1b) to (4-4b), 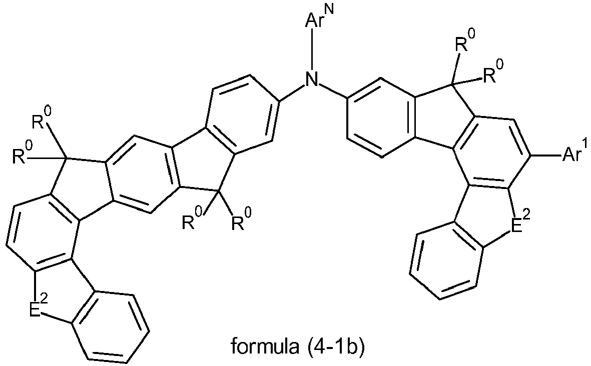 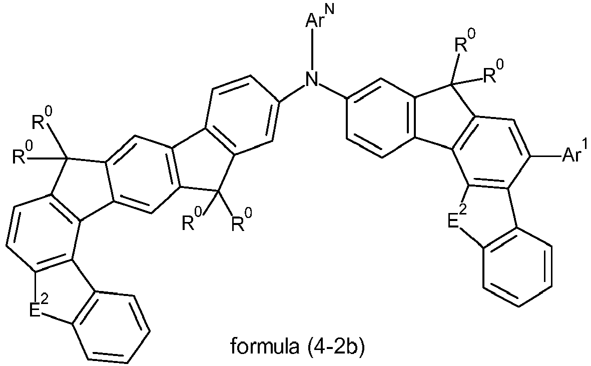 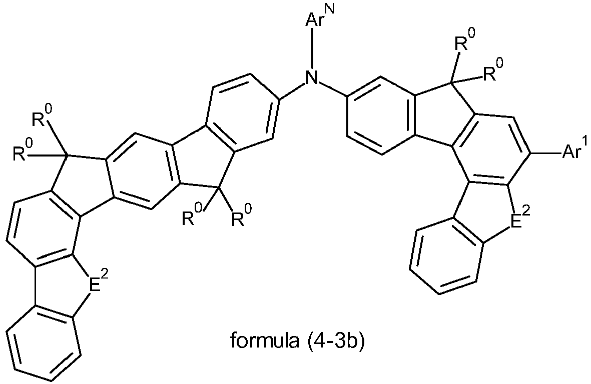 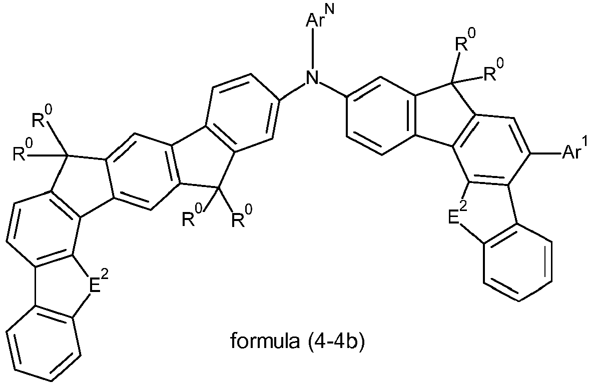 where the symbols Ar^{N}, E², Ar¹ and R⁰ have the same meaning as in claim 1.

10. Compound according to one or more of the preceding claims, **characterized in that** E² stands for O.

11. Compound according to one or more of the preceding claims, **characterized in that** Ar¹ stands on each occurrence, identically or differently, for phenyl, biphenyl, fluorene, spirobifluorene, naphthalene, phenanthrene, dibenzofuran, dibenzothiophene, carbazole, pyridine, pyrimidine, pyrazine, pyridazine, triazine, benzopyridine, benzopyridazine, benzopyrimidine, quinazoline, or for a combination of two to six of these groups, each of which may be substituted by one or more radicals R.

12. Compound according to one or more of the preceding claims, **characterized in that** Ar¹ stands on each occurrence, identically or differently, for an aromatic or heteroaromatic ring system selected from phenyl, biphenyl, fluorene or naphthalene, or for a combination of two to six of these groups, each of which may be substituted by one or more radicals R.

13. Compound according to one or more of the preceding claims, **characterized in that** Ar¹ is selected from the groups of formulae (Ar1-1) to (Ar1-22), 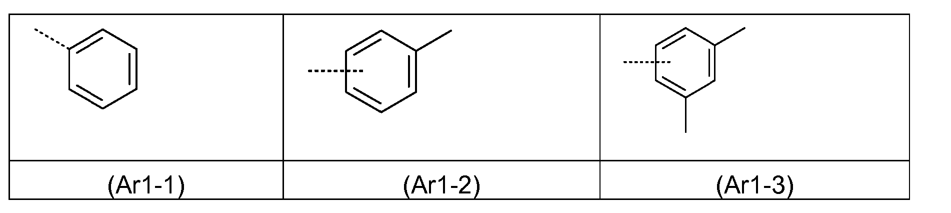 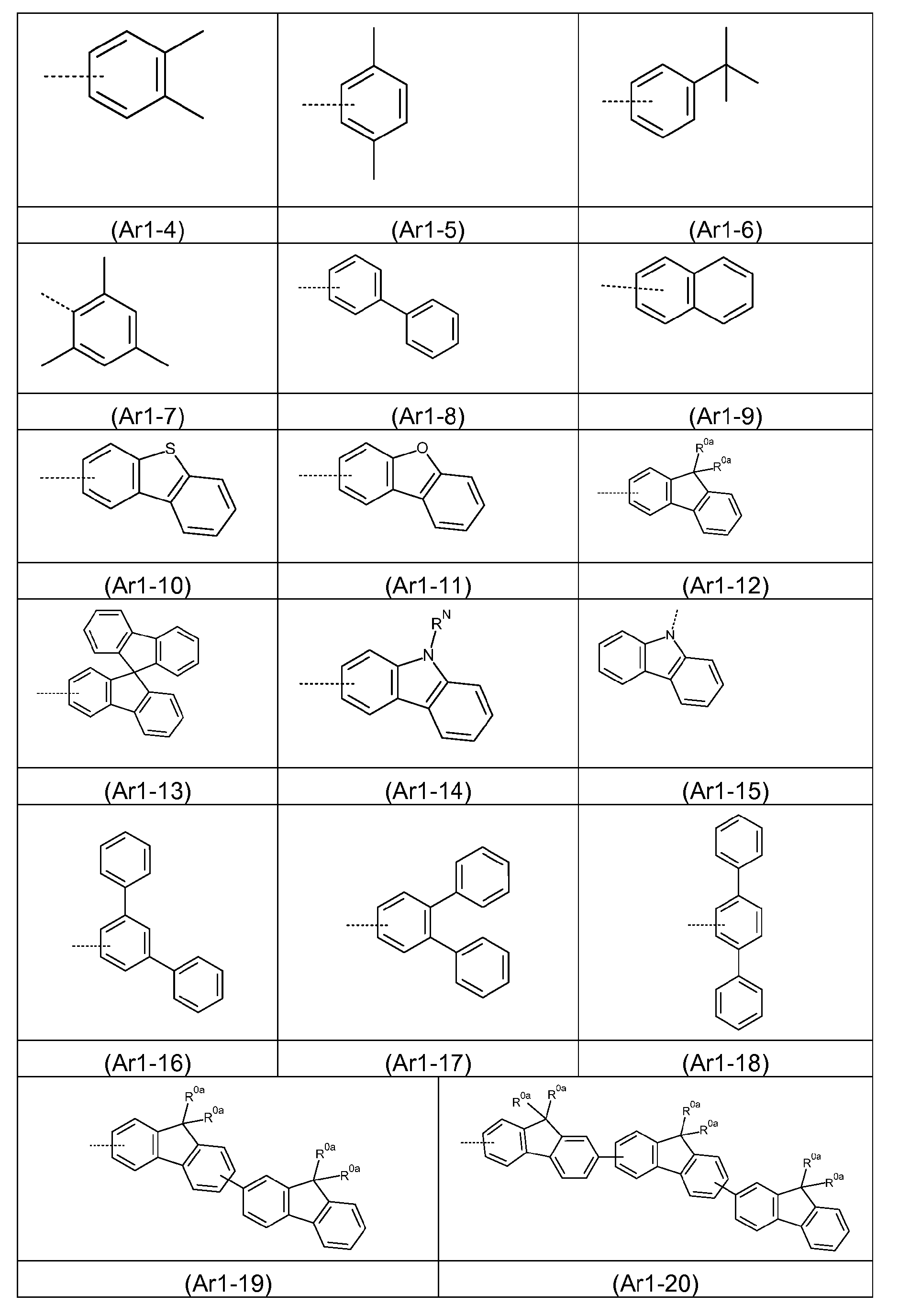 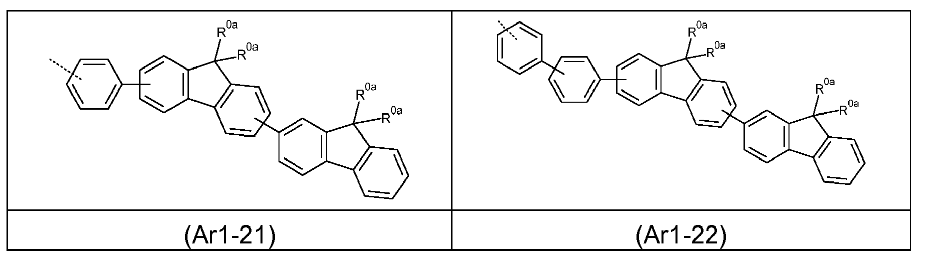 where in formulae (Ar1-1) to (Ar1-22):
- the dashed bond indicates the bonding to the structure of formula (1);
- the group R^{N} in formula (Ar1-14) stands on each occurrence, identically or differently, for H, D, a straight-chain alkyl group having 1 to 40, preferably 1 to 20, more preferably 1 to 10 C atoms or branched or cyclic alkyl group having 3 to 40, preferably 3 to 20, more preferably 3 to 10 C atoms, each of which may be substituted by one or more radicals R, where in each case one or more non-adjacent CH₂ groups may be replaced by RC=CR, C=C, C=O, C=S, SO, SO₂, O or S, and where one or more H atoms may be replaced by D, F or CN, an aromatic or heteroaromatic ring system having 5 to 60, preferably 5 to 40, more preferably 5 to 30, particularly preferably 5 to 18 aromatic ring atoms, which may in each case be substituted by one or more radicals R, where two adjacent substituents R^{N} may form a mono- or polycyclic, aliphatic ring system or aromatic ring system, which may be substituted by one or more radicals R, where R has the same meaning as in claim 1;
- the group R^{0a} in formulae (Ar1-12) and (Ar1-19) to (Ar1-22) has the same definition as the group R⁰ as defined in claim 1; and
- the groups of formulae (Ar1-1) to (Ar1-22) may be substituted at each free position by a group R, which has the same as in claim 1.

14. Polymer, oligomer or dendrimer containing one or more compounds according to claim 1, where the bond(s) to the polymer, oligomer or dendrimer may be localised at any positions in formula (1) which is substituted by R⁰, R¹, R², R³, R⁴ or R⁵.

15. Formulation comprising at least one compound according to one or more of the claims 1 to 13 or at least one polymer, oligomer or dendrimer according to claim 14 and at least one solvent.

16. Electronic device comprising at least one compound according to one or more of claims 1 to 13 or at least one polymer, oligomer or dendrimer according to claim 14, selected from the group consisting of organic electroluminescent devices, organic integrated circuits, organic field-effect transistors, organic thin-film transistors, organic light-emitting transistors, organic solar cells, dye-sensitised organic solar cells, organic optical detectors, organic photoreceptors, organic field-quench devices, light-emitting electrochemical cells, organic laser diodes and organic plasmon emitting devices.

17. Electronic device according to claim 16, which is an organic electroluminescent device, **characterised in that** the compound according to one or more of claims 1 to 13 or the polymer, oligomer or dendrimer according to claim 14 is employed as a fluorescent emitter or as a matrix material for fluorescent emitters.

## Patentansprüche

1. Verbindung der Formel (1), 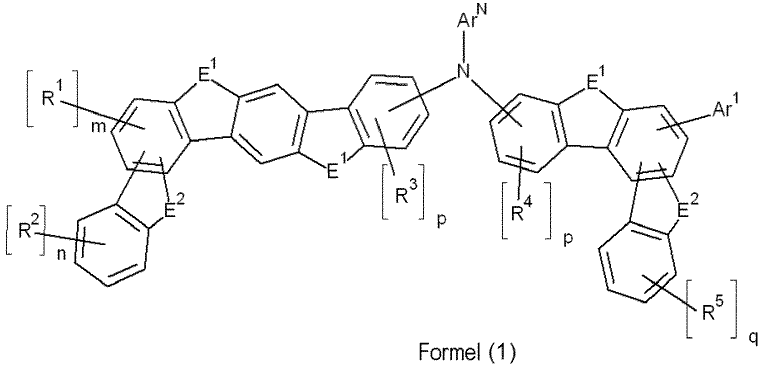 wobei für die verwendeten Symbole und Indizes Folgendes gilt:
Ar¹, Ar^{N} sind bei jedem Auftreten gleich oder verschieden aus einem aromatischen oder heteroaromatischen Ringsystem mit 5 bis 60 aromatischen Ringatomen, das jeweils durch einen oder mehrere Reste R substituiert sein kann, ausgewählt;
E¹ ist bei jedem Auftreten gleich oder verschieden aus - BR⁰-, -C(R⁰)₂-, -C(R⁰)₂-C(R⁰)₂-, -C(R⁰)₂-O-, -C(R⁰)₂-S-, -R⁰C=CR⁰-, -R⁰C=N-, Si(R⁰)₂, -Si(R⁰)₂-Si(R⁰)₂-, -C(=O)-, -C(=NR⁰)-, -C(=C(R⁰)₂)-, -O-, -S-, -S(=O)-, -SO₂-, -N(R⁰)-, -P(R⁰)- und -P((=O)R⁰)- ausgewählt; oder E¹ ist eine Gruppe der Formel (E-1), 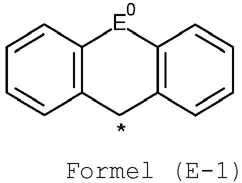 wobei das Symbol * in Formel (E-1) die entsprechende Gruppe E¹ in Formel (1) anzeigt; und
E⁰ ist bei jedem Auftreten gleich oder verschieden aus der Gruppe bestehend aus einer Einfachbindung, -BR⁰- , -C(R⁰)₂-, -C(R⁰)₂-C(R⁰)₂-, -C(R⁰)₂-O-, -C(R⁰)₂-S-, -R⁰C=CR⁰-, -R⁰C=N-, Si(R⁰)₂, -Si(R⁰)₂-Si(R⁰)₂-, -C(=O)-, -C(=NR⁰)-, -C(=C(R⁰)₂)-, -O-, -S-, -S(=O)-, -SO₂-, -N(R⁰)-, -P(R⁰)- und -P((=O)R⁰)- ausgewählt;
E² ist bei jedem Auftreten gleich oder verschieden aus der Gruppe bestehend aus -O-, -S-, -S(=O)- und -SO₂-ausgewählt;
R⁰ bis R⁵ stehen bei jedem Auftreten gleich oder verschieden für H, D, F, Cl, Br, I, CHO, CN, N(Ar)₂, C(=O)Ar, P(=O)(Ar)₂, S(=O)Ar, S(=O)₂Ar, NO₂, Si(R)₃, B(OR)₂, OSO₂R, eine geradkettige Alkyl-, Alkoxy- oder Thioalkylgruppe mit 1 bis 40 C-Atomen oder verzweigte oder cyclische Alkyl-, Alkoxy- oder Thioalkylgruppe mit 3 bis 40 C-Atomen, wobei jede dieser Gruppen durch einen oder mehrere Reste R substituiert sein kann, wobei jeweils eine oder mehrere nicht benachbarte CH₂-Gruppen durch RC=CR, C=C, Si(R)₂, Ge(R)₂, Sn(R)₂, C=O, C=S, C=Se, P(=O) (R), SO, SO₂, O, S oder CONR ersetzt sein können und wobei ein oder mehrere H-Atome durch D, F, Cl, Br, I, CN oder NO₂ ersetzt sein können, ein aromatisches oder heteroaromatisches Ringsystem mit 5 bis 60 aromatischen Ringatomen, das jeweils durch einen oder mehrere Reste R substituiert sein kann, oder eine Aryloxygruppe mit 5 bis 60 aromatischen Ringatomen, die durch einen oder mehrere Reste R substituiert sein kann, wobei zwei benachbarte Substituenten R⁰ und/oder zwei benachbarte Substituenten R¹ und/oder zwei benachbarte Substituenten R² und/oder zwei benachbarte Substituenten R³ und/oder zwei benachbarte Substituenten R⁴ und/oder zwei benachbarte Substituenten R⁵ ein mono- oder polycyclisches, aliphatisches Ringsystem oder aromatisches Ringsystem, das durch einen oder mehrere Reste R substituiert sein kann, bilden können; m steht bei jedem Auftreten gleich oder verschieden für eine ganze Zahl, die aus 0, 1 oder 2 ausgewählt ist;
n, q stehen bei jedem Auftreten gleich oder verschieden für eine ganze Zahl, die aus 0, 1, 2, 3 oder 4 ausgewählt ist;
p steht bei jedem Auftreten gleich oder verschieden für eine ganze Zahl, die aus 0, 1, 2 oder 3 ausgewählt ist;
R steht bei jedem Auftreten gleich oder verschieden für H, D, F, Cl, Br, I, CHO, CN, N(Ar)₂, C(=O)Ar, P(=O)(Ar)₂, S(=O)Ar, S(=O)₂Ar, NO₂, Si(R')₃, B(OR')₂, OSO₂R', eine geradkettige Alkyl-, Alkoxy- oder Thioalkylgruppe mit 1 bis 40 C-Atomen oder verzweigte oder cyclische Alkyl-, Alkoxy- oder Thioalkylgruppe mit 3 bis 40 C-Atomen, wobei jede dieser Gruppen durch einen oder mehrere Reste R' substituiert sein kann, wobei jeweils eine oder mehrere nicht benachbarte CH₂-Gruppen durch R'C=CR', C=C, Si(R')₂, Ge(R')₂, Sn(R')₂, C=O, C=S, C=Se, P(=O)(R'), SO, SO₂, O, S oder CONR' ersetzt sein können und wobei ein oder mehrere H-Atome durch D, F, Cl, Br, I, CN oder NO₂ ersetzt sein können, ein aromatisches oder heteroaromatisches Ringsystem mit 5 bis 60 aromatischen Ringatomen, das jeweils durch einen oder mehrere Reste R' substituiert sein kann, oder eine Aryloxygruppe mit 5 bis 60 aromatischen Ringatomen, die durch einen oder mehrere Reste R' substituiert sein kann, wobei zwei benachbarte Substituenten R ein mono- oder polycyclisches, aliphatisches Ringsystem oder aromatisches Ringsystem, das durch einen oder mehrere Reste R' substituiert sein kann, bilden können;
Ar ist ein aromatisches oder heteroaromatisches Ringsystem mit 5 bis 24 aromatischen Ringatomen, das jeweils auch durch einen oder mehrere Reste R' substituiert sein kann; R' steht bei jedem Auftreten gleich oder verschieden für H, D, F, Cl, Br, I, CN, eine geradkettige Alkyl-, Alkoxy- oder Thioalkylgruppe mit 1 bis 20 C-Atomen oder verzweigte oder cyclische Alkyl-, Alkoxy- oder Thioalkylgruppe mit 3 bis 20 C-Atomen, wobei jeweils eine oder mehrere nicht benachbarte CH₂-Gruppen durch SO, SO₂, O, S ersetzt sein können und wobei ein oder mehrere H-Atome durch D, F, Cl, Br oder I ersetzt sein können, oder ein aromatisches oder heteroaromatisches Ringsystem mit 5 bis 24 C-Atomen.

2. Verbindung nach Anspruch 1, **dadurch gekennzeichnet, dass** Ar^{N} für Phenyl, Biphenyl, Fluoren, Spirobifluoren, Naphthalin, Phenanthren, Dibenzofuran, Dibenzothiophen, Carbazol, Pyridin, Pyrimidin, Pyrazin, Pyridazin, Triazin, Benzopyridin, Benzopyridazin, Benzopyrimidin oder Chinazolin oder für eine Kombination von zwei bis sechs dieser Gruppen, die jeweils durch einen oder mehrere Reste R substituiert sein können, steht.

3. Verbindung nach Anspruch 1 oder 2, **dadurch gekennzeichnet, dass** Ar^{N} für Phenyl, Biphenyl, Fluoren, Naphthalin, Phenanthren, Dibenzofuran, Dibenzothiophenyl oder Carbazol oder für eine Kombination von zwei bis sechs dieser Gruppen, die jeweils durch einen oder mehrere Reste R substituiert sein können, steht.

4. Verbindung nach einem oder mehreren der vorhergehenden Ansprüche, **dadurch gekennzeichnet, dass** sie aus Verbindungen der Formel (2) ausgewählt ist, 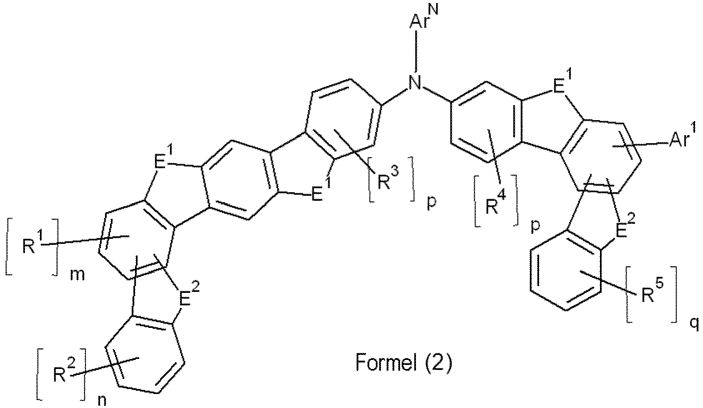 wobei die Symbole Ar^{N}, E¹, E², Ar¹, R¹ bis R⁵ und die Indizes m, n, p und q die gleiche Bedeutung wie in Anspruch 1 haben.

5. Verbindung nach einem oder mehreren der vorhergehenden Ansprüche, die aus Verbindungen der Formel (3) ausgewählt ist, 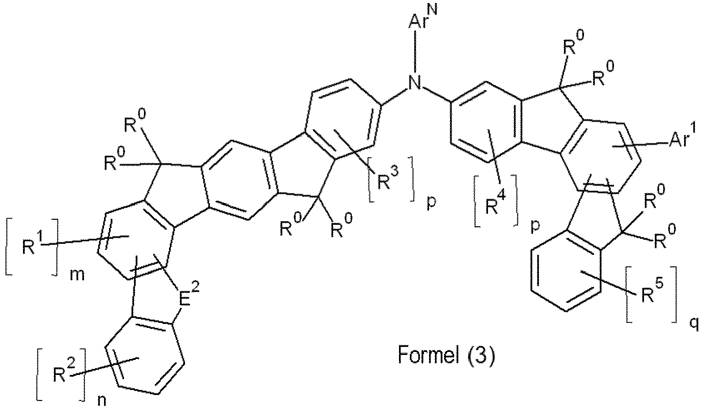 wobei die Symbole Ar^{N}, E², Ar¹, R⁰, R¹ bis R⁵ und die Indizes m, n, p und q die gleiche Bedeutung wie in Anspruch 1 haben.

6. Verbindung nach einem oder mehreren der vorhergehenden Ansprüche, die aus Verbindungen der Formel (4) ausgewählt ist, 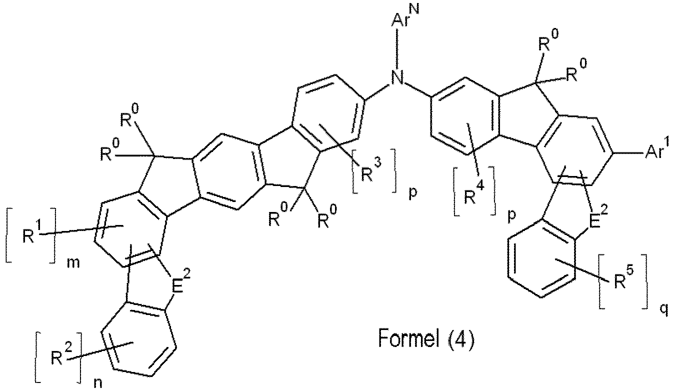 wobei die Symbole Ar^{N}, E², Ar¹, R⁰, R¹ bis R⁵ und die Indizes m, n, p und q die gleiche Bedeutung wie in Anspruch 1 haben.

7. Verbindung nach einem oder mehreren der vorhergehenden Ansprüche, **dadurch gekennzeichnet, dass** sie aus den Verbindungen der Formel (4-1) bis (4-4) ausgewählt ist, 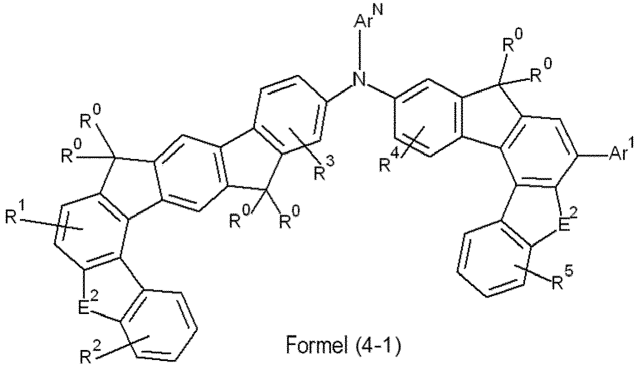 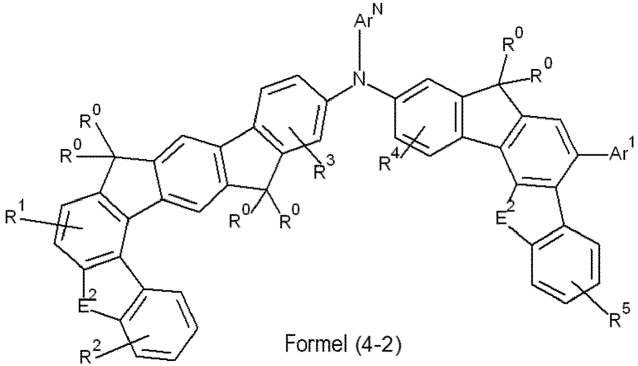 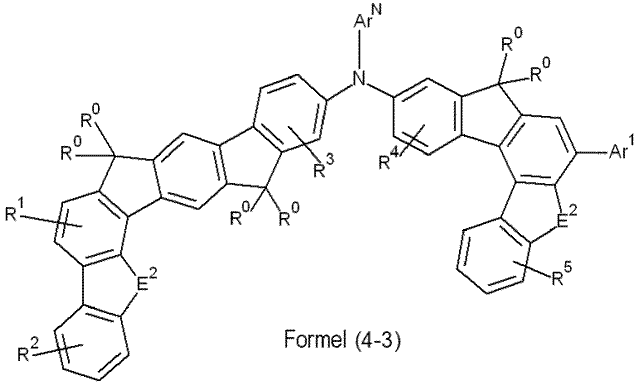 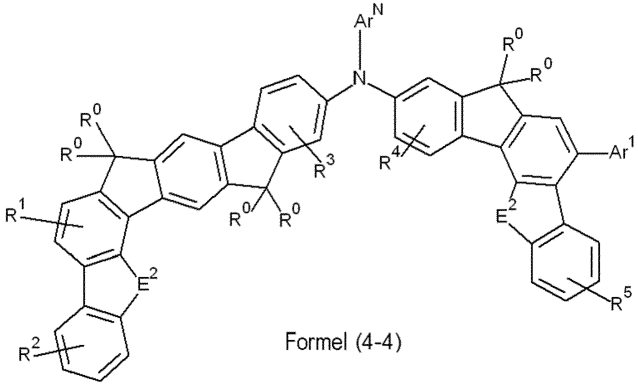 wobei die Symbole Ar^{N}, E², Ar¹, R⁰, R¹ bis R⁵ die gleiche Bedeutung wie in Anspruch 1 haben.

8. Verbindung nach einem oder mehreren der vorhergehenden Ansprüche, **dadurch gekennzeichnet, dass** sie aus den Verbindungen der Formel (4-1a) bis (4-4a) ausgewählt ist, 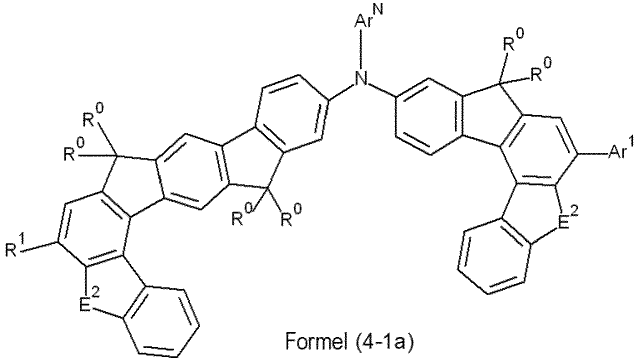 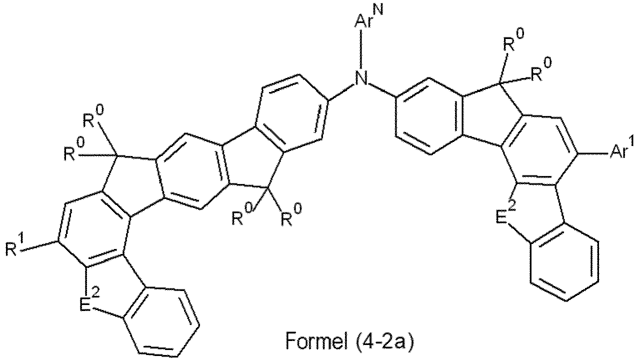 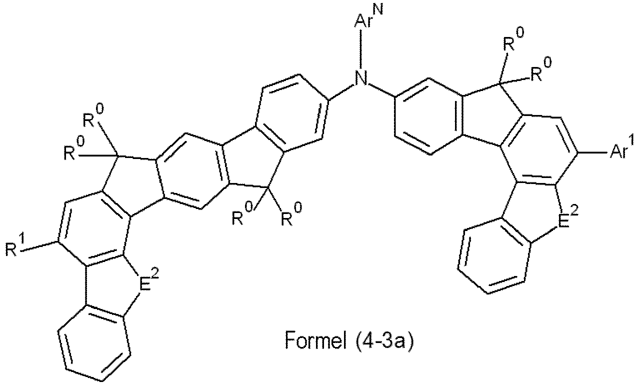 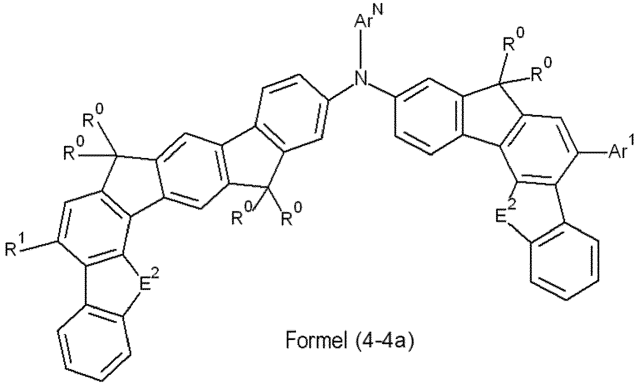 wobei die Symbole Ar^{N}, E², Ar¹, R⁰ und R¹ die gleiche Bedeutung wie in Anspruch 1 haben.

9. Verbindung nach einem oder mehreren der vorhergehenden Ansprüche, **dadurch gekennzeichnet, dass** sie aus den Verbindungen der Formel (4-1b) bis (4-4b) ausgewählt ist, 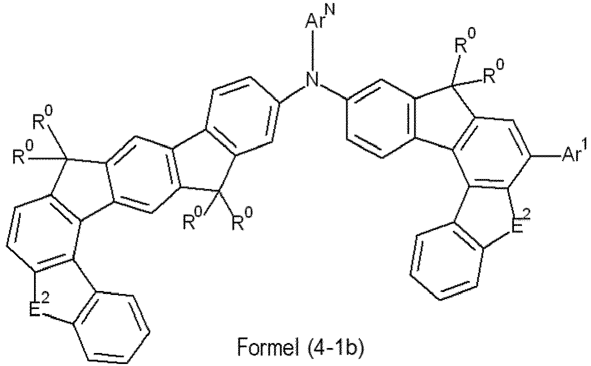 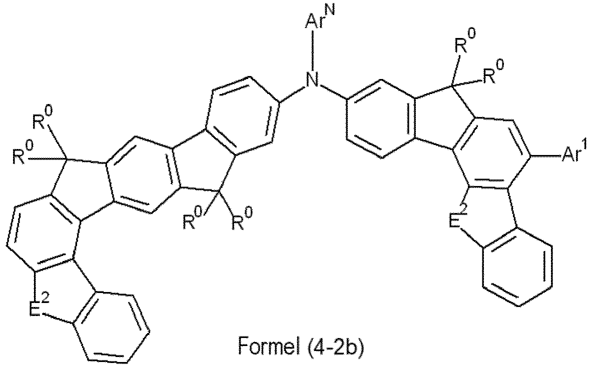 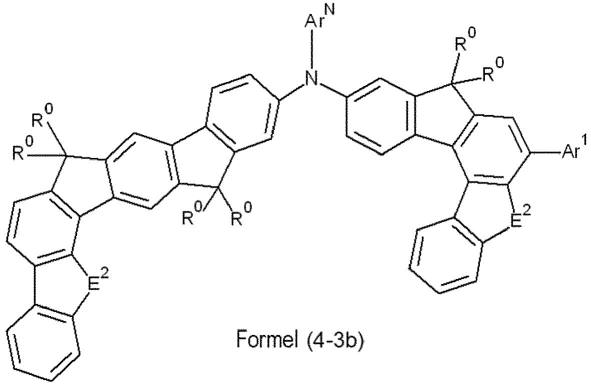 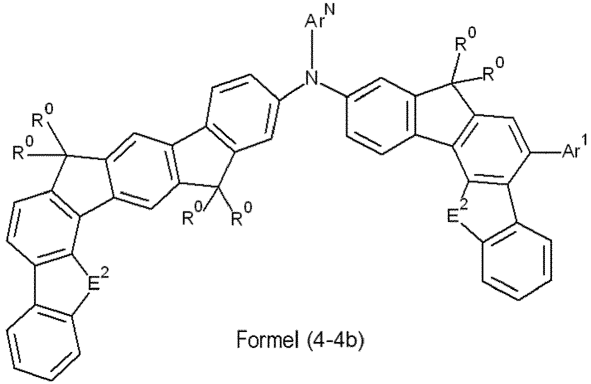 wobei die Symbole Ar^{N}, E², Ar¹ und R⁰ die gleiche Bedeutung wie in Anspruch 1 haben.

10. Verbindung nach einem oder mehreren der vorhergehenden Ansprüche, **dadurch gekennzeichnet, dass** E² für O steht.

11. Verbindung nach einem oder mehreren der vorhergehenden Ansprüche, **dadurch gekennzeichnet, dass** Ar¹ bei jedem Auftreten gleich oder verschieden für Phenyl, Biphenyl, Fluoren, Spirobifluoren, Naphthalin, Phenanthren, Dibenzofuran, Dibenzothiophen, Carbazol, Pyridin, Pyrimidin, Pyrazin, Pyridazin, Triazin, Benzopyridin, Benzopyridazin, Benzopyrimidin oder Chinazolin oder für eine Kombination von zwei bis sechs dieser Gruppen, die jeweils durch einen oder mehrere Reste R substituiert sein können, steht.

12. Verbindung nach einem oder mehreren der vorhergehenden Ansprüche, **dadurch gekennzeichnet, dass** Ar¹ bei jedem Auftreten gleich oder verschieden für Phenyl, Biphenyl, Fluoren oder Naphthalin oder für eine Kombination von zwei bis sechs dieser Gruppen, die jeweils durch einen oder mehrere Reste R substituiert sein können, steht.

13. Verbindung nach einem oder mehreren der vorhergehenden Ansprüche, **dadurch gekennzeichnet, dass** Ar¹ aus den Gruppen der Formeln (Ar1-1) bis (Ar1-22) ausgewählt ist, 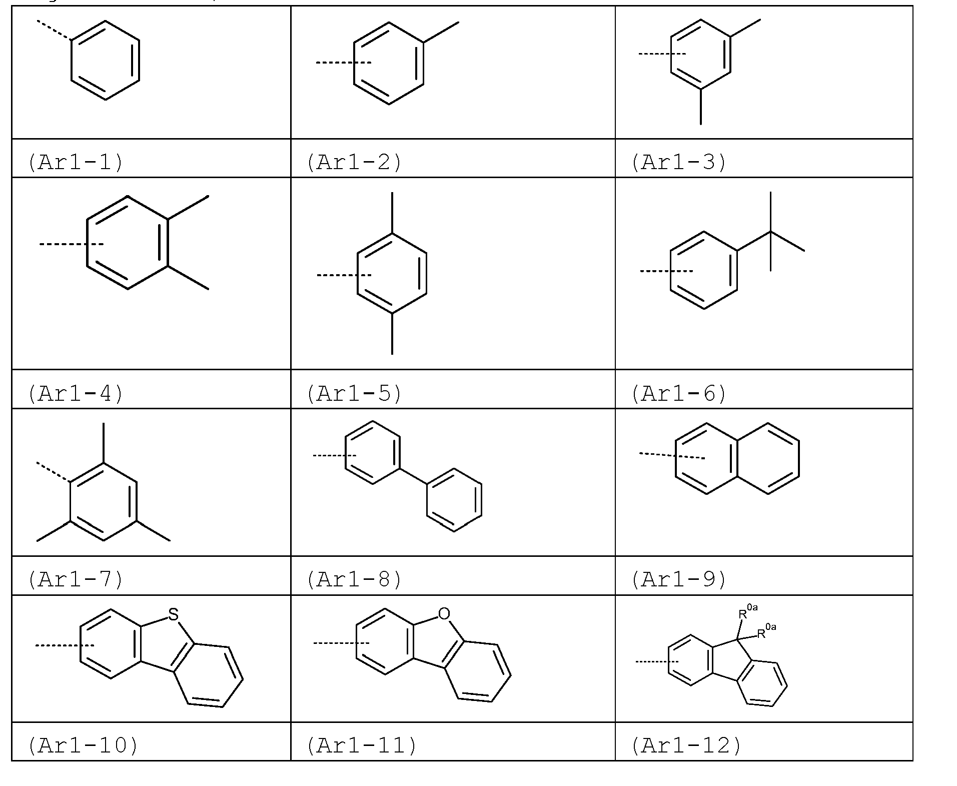 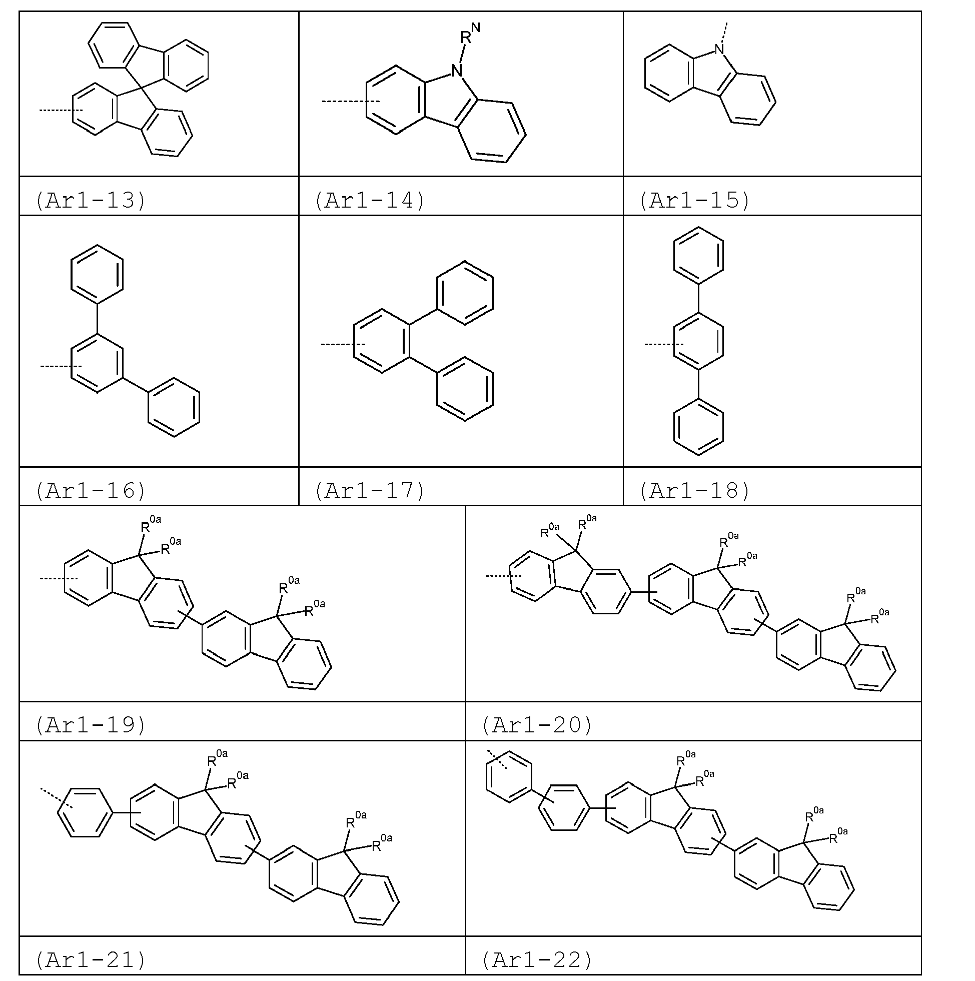 wobei in den Formeln (Ar1-1) bis (Ar1-22) Folgendes gilt:
- die gestrichelte Bindung zeigt die Anbindung an die Struktur der Formel (1) an;
- die Gruppe R^{N} in Formel (Ar1-14) steht bei jedem Auftreten gleich oder verschieden für H, D, eine geradkettige Alkylgruppe mit 1 bis 40, bevorzugt 1 bis 20 und weiter bevorzugt 1 bis 10 C-Atomen oder verzweigte oder cyclische Alkylgruppe mit 3 bis 40, bevorzugt 3 bis 20 und weiter bevorzugt 3 bis 10 C-Atomen, wobei jede dieser Gruppen durch einen oder mehrere Reste R substituiert sein kann, wobei jeweils eine oder mehrere nicht benachbarte CH₂-Gruppen durch RC=CR, C=C, C=O, C=S, SO, SO₂, O oder S ersetzt sein können und wobei ein oder mehrere H-Atome durch D, F oder CN ersetzt sein können, ein aromatisches oder heteroaromatisches Ringsystem mit 5 bis 60, bevorzugt 5 bis 40, weiter bevorzugt 5 bis 30 und besonders bevorzugt 5 bis 18 aromatischen Ringatomen, das jeweils durch einen oder mehrere Reste R substituiert sein kann, wobei zwei benachbarte Substituenten R^{N} ein mono- oder polycyclisches, aliphatisches Ringsystem oder aromatisches Ringsystem, das durch einen oder mehrere Reste R substituiert sein kann, bilden können, wobei R die gleiche Bedeutung wie in Anspruch 1 hat;
- die Gruppe R^{0a} in den Formeln (Ar1-12) und (Ar1-19) bis (Ar1-22) hat die gleiche Definition wie die wie in Anspruch 1 definierte Gruppe R⁰; und
- die Gruppen der Formeln (Ar1-1) bis (Ar1-22) können an jeder freien Position durch eine Gruppe R, die die gleiche Bedeutung wie in Anspruch 1 hat, substituiert sein.

14. Polymer, Oligomer oder Dendrimer, enthaltend eine oder mehrere Verbindungen nach Anspruch 1, wobei die Bindung bzw. die Bindungen zum Polymer, Oligomer oder Dendrimer in beliebigen Positionen in Formel (1), die durch R⁰, R¹, R², R³, R⁴ oder R⁵ substituiert ist, lokalisiert sein kann bzw. können.

15. Formulierung, umfassend mindestens eine Verbindung nach einem oder mehreren der Ansprüche 1 bis 13 oder mindestens ein Polymer, Oligomer oder Dendrimer nach Anspruch 14 und mindestens ein Lösungsmittel.

16. Elektronische Vorrichtung, umfassend mindestens eine Verbindung nach einem oder mehreren der Ansprüche 1 bis 13 oder mindestens ein Polymer, Oligomer oder Dendrimer nach Anspruch 14, ausgewählt aus der Gruppe bestehend aus organischen Elektrolumineszenzvorrichtungen, organischen integrierten Schaltungen, organischen Feldeffekttransistoren, organischen Dünnfilmtransistoren, organischen lichtemittierenden Transistoren, organischen Solarzellen, farbstoffsensibilisierten organischen Solarzellen, organischen optischen Detektoren, organischen Photorezeptoren, organischen Feld-Quench-Devices, lichtemittierenden elektrochemischen Zellen, organischen Laserdioden und "Organic Plasmon Emitting Devices".

17. Elektronische Vorrichtung nach Anspruch 16, bei der es sich um eine organische Elektrolumineszenzvorrichtung handelt, **dadurch gekennzeichnet, dass** die Verbindung nach einem oder mehreren der Ansprüche 1 bis 13 oder das Polymer, Oligomer oder Dendrimer nach Anspruch 14 als fluoreszierender Emitter oder als Matrixmaterial für fluoreszierende Emitter eingesetzt wird.

## Revendications

1. Composé de la formule (1), 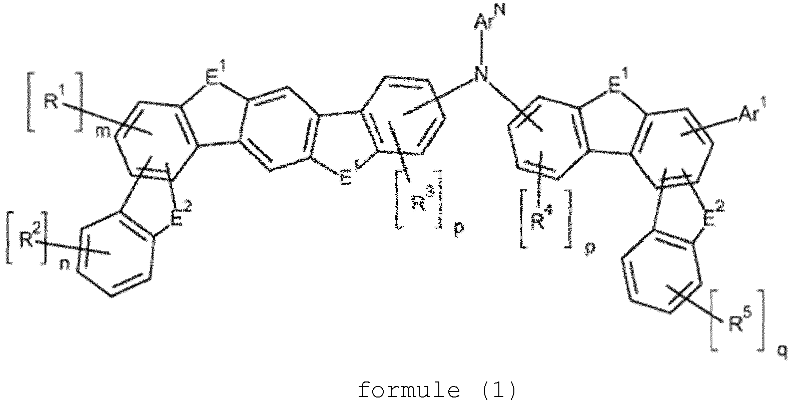 où ce qui suit s'applique aux symboles et indices utilisés :
Ar¹, Ar^{N} sont en chaque occurrence, de manière identique ou différente, choisis parmi un système cyclique aromatique ou hétéroaromatique possédant 5 à 60 atomes de cycle aromatique, qui peut en chaque fois être substitué par un ou plusieurs radicaux R ;
E¹ est en chaque occurrence, de manière identique ou différente, choisi parmi -BR⁰-, -C(R⁰)₂-, -C(R⁰)₂-C(R⁰)₂-, -C(R⁰)₂-O-, -C(R⁰)₂-S-, -R⁰C=CR⁰-, -R⁰C=N-, Si(R⁰)₂, -Si(R⁰)₂-Si(R⁰)₂-, -C(=O)-, -C(=NR⁰)-, - C(=C(R⁰)₂)-, -O-, -S-, -S(=O)-, -SO₂-, -N(R⁰)-, - P(R⁰)- et -P((=O)R⁰)- ; ou E¹ est un groupe de formule (E-1), 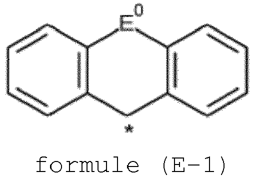 où le symbole * dans la formule (E-1) indique le groupe E¹ correspondant dans la formule (1) ; et
E⁰ est de manière identique ou différente en chaque occurrence, choisi dans le groupe constitué par une simple liaison, , -BR⁰-, -C(R⁰)₂-, -C(R⁰)₂-C(R⁰)₂-, - C(R⁰)₂-O-, -C(R⁰)₂-S-, -R⁰C=CR⁰-, -R⁰C=N-, Si(R⁰)₂, - Si(R⁰)₂-Si(R⁰)₂-, -C(=O)-, -C(=NR⁰)-, -C(=C(R⁰)₂)-, - O-, -S-, -S(=O)-, -SO₂-, -N(R⁰)-, -P(R⁰)- et - P((=O)R⁰)- ;
E² est, de manière identique ou différente, en chaque occurrence, choisi dans le groupe constitué par -O-, -S-, -S(=O)- et -SO₂- ;
R⁰ à R⁵ représentent en chaque occurrence, de manière identique ou différente, H, D, F, Cl, Br, I, CHO, CN, N(Ar)₂, C(=O)Ar, P(=O)(Ar)₂, S(=O)Ar, S(=O)₂Ar, NO₂, Si(R)₃, B(OR)₂, OSO₂R, un groupe alkyle, alcoxy ou thioalkyle linéaire possédant 1 à 40 atomes de C ou un groupe alkyle, alcoxy ou thioalkyle ramifié ou cyclique possédant 3 à 40 atomes de C, chacun desquels pouvant être substitué par un ou plusieurs radicaux R, où en chaque cas un ou plusieurs groupes CH₂ non adjacents peuvent être remplacés par RC=CR, C≡C, Si(R)₂, Ge(R)₂, Sn(R)₂, C=O, C=S, C=Se, P(=O)(R), SO, SO₂, O, S ou CONR et où un ou plusieurs atomes de H peuvent être remplacés par D, F, Cl, Br, I, CN ou NO₂, un système cyclique aromatique ou hétéroaromatique possédant 5 à 60 atomes de cycle aromatique, qui peut en chaque cas être substitué par un ou plusieurs radicaux R, ou un groupe aryloxy possédant 5 à 60 atomes de cycle aromatique, qui peut être substitué par un ou plusieurs radicaux R, où deux substituants R⁰ adjacents, et/ou deux substituants R¹ adjacents, et/ou deux substituants R² adjacents, et/ou deux substituants R³ adjacents, et/ou deux substituants R⁴ adjacents, et/ou deux substituants R⁵ adjacents peuvent former un système cyclique aliphatique ou un système cyclique aromatique, monocyclique ou polycyclique, qui peut être substitué par un ou plusieurs radicaux R ;
m représente en chaque occurrence, de manière identique ou différente, un entier choisi parmi 0, 1 et 2 ;
n, q représentent en chaque occurrence, de manière identique ou différente, un entier choisi parmi 0, 1, 2, 3 et 4 ;
p représente en chaque occurrence, de manière identique ou différente, un entier choisi parmi 0, 1, 2 et 3 ;
R représente en chaque occurrence, de manière identique ou différente, H, D, F, Cl, Br, I, CHO, CN, N(Ar)₂, C(=O)Ar, P(=O)(Ar)₂, S(=O)Ar, S(=O)₂Ar, NO₂, Si(R')₃, B(OR')₂, OSO₂R', un groupe alkyle, alcoxy ou thioalkyle linéaire possédant 1 à 40 atomes de C ou un groupe alkyle, alcoxy ou thioalkyle ramifié ou cyclique possédant 3 à 40 atomes de C, chacun desquels pouvant être substitué par un ou plusieurs radicaux R', où en chaque cas un ou plusieurs groupes CH₂ non adjacents peuvent être remplacés par R'C=CR', C≡C, Si(R')₂, Ge(R')₂, Sn(R')₂, C=O, C=S, C=Se, P(=O)(R'), SO, SO₂, O, S ou CONR' et où un ou plusieurs atomes de H peuvent être remplacés par D, F, Cl, Br, I, CN ou NO₂, un système cyclique aromatique ou hétéroaromatique possédant 5 à 60 atomes de cycle aromatique, qui peut en chaque cas être substitué par un ou plusieurs radicaux R', ou un groupe aryloxy possédant 5 à 60 atomes de cycle aromatique, qui peut être substitué par un ou plusieurs radicaux R', où deux substituants R adjacents peuvent former un système cyclique aliphatique ou un système cyclique aromatique, monocyclique ou polycyclique, qui peut être substitué par un ou plusieurs radicaux R' ;
Ar est un système cyclique aromatique ou hétéroaromatique possédant 5 à 24 atomes de cycle aromatique, qui peut également être en chaque cas substitué par un ou plusieurs radicaux R' ;
R' représente en chaque occurrence, de manière identique ou différente, H, D, F, Cl, Br, I, CN, un groupe alkyle, alcoxy ou thioalkyle linéaire possédant 1 à 20 atomes de C ou un groupe alkyle, alcoxy ou thioalkyle ramifié ou cyclique possédant 3 à 20 atomes de C, où en chaque cas un ou plusieurs groupes CH₂ non adjacents peuvent être remplacés par SO, SO₂, O, S et où un ou plusieurs atomes de H peuvent être remplacés par D, F, Cl, Br ou I, ou un système cyclique aromatique ou hétéroaromatique possédant 5 à 24 atomes de C.

2. Composé selon la revendication 1, **caractérisé en ce que** Ar^{N} représente phényle, biphényle, fluorène, spirobifluorène, naphtalène, phénanthrène, dibenzofuranne, dibenzothiophène, carbazole, pyridine, pyrimidine, pyrazine, pyridazine, triazine, benzopyridine, benzopyridazine, benzopyrimidine ou quinazoline, ou représente une combinaison de deux à six de ces groupes, chacun desquels pouvant être substitué par un ou plusieurs radicaux R.

3. Composé selon la revendication 1 ou 2, **caractérisé en ce que** Ar^{N} représente phényle, biphényle, fluorène, naphtalène, phénanthrène, dibenzofuranne, dibenzothiophène, carbazole, ou représente une combinaison de deux à six de ces groupes, chacun desquels pouvant être substitué par un ou plusieurs radicaux R.

4. Composé selon l'une ou plusieurs des revendications précédentes, **caractérisé en ce qu'**il est choisi parmi des composés de formule (2), 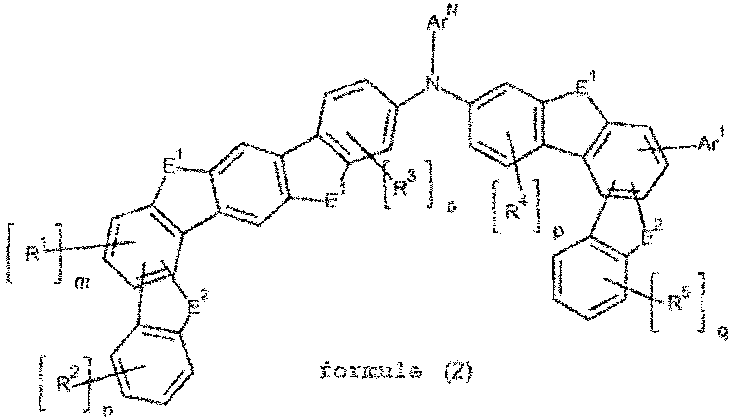 où les symboles Ar^{N}, E¹, E², Ar¹, R¹ à R⁵ et les indices m, n, p et q possèdent la même signification que dans la revendication 1.

5. Composé selon l'une ou plusieurs des revendications précédentes, choisi parmi des composés de formule (3) 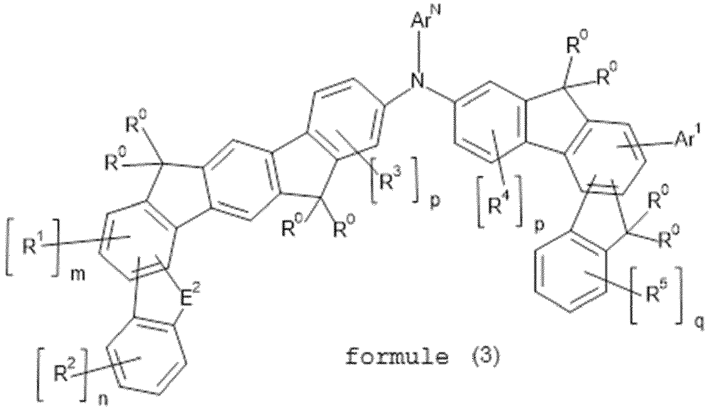 où les symboles Ar^{N}, E², Ar¹, R⁰, R¹ à R⁵ et les indices m, n, p et q possèdent la même signification que dans la revendication 1.

6. Composé selon l'une ou plusieurs des revendications précédentes, choisi parmi les composés de formule (4), 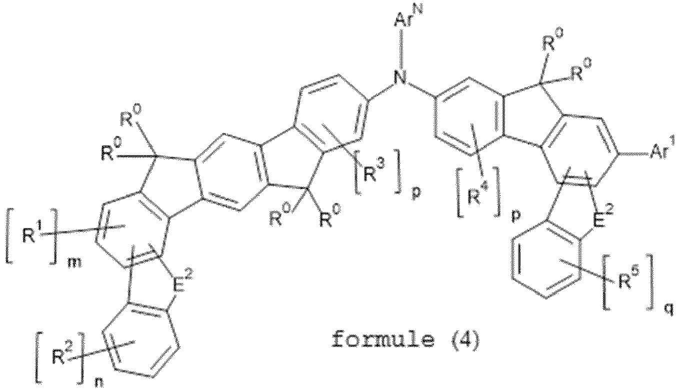 où les symboles Ar^{N}, E², Ar¹, R⁰, R¹ à R⁵ et les indices m, n, p et q possèdent la même signification que dans la revendication 1.

7. Composé selon l'une ou plusieurs des revendications précédentes, **caractérisé en ce qu'**il est choisi parmi les composés de formule (4-1) à (4-4), 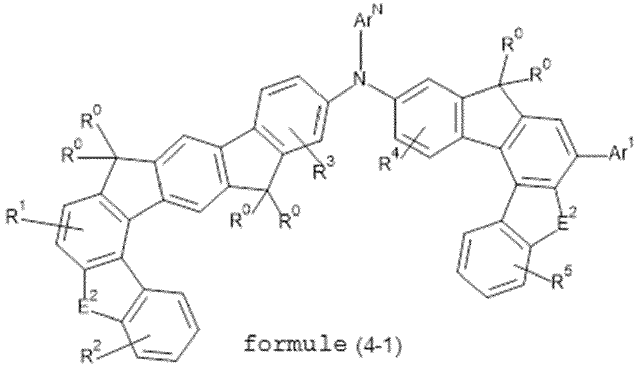 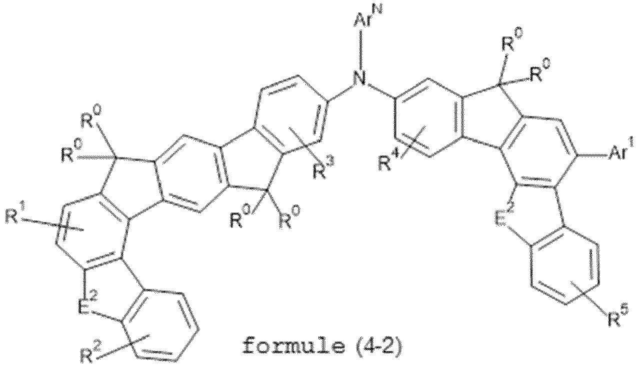 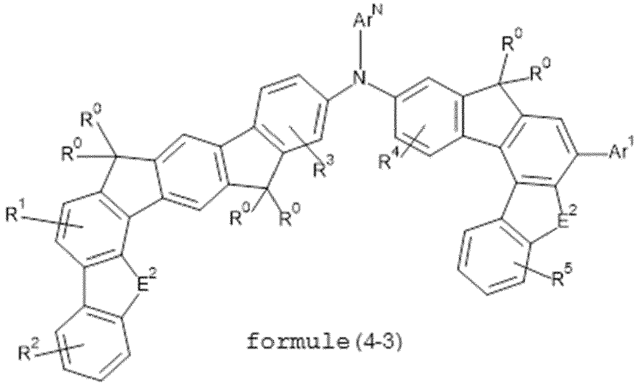 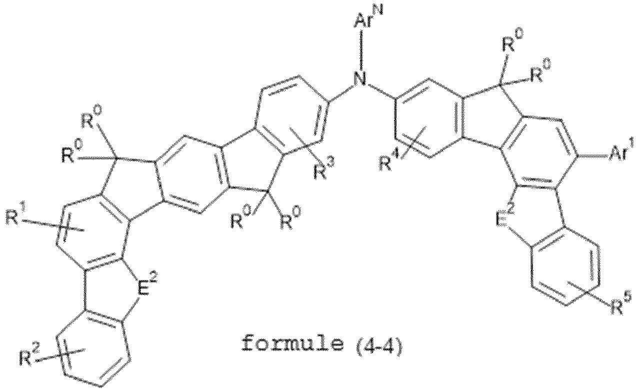 où les symboles Ar^{N}, E², Ar¹, R⁰, R¹ à R⁵ possèdent la même signification que dans la revendication 1.

8. Composé selon l'une ou plusieurs des revendications précédentes, **caractérisé en ce qu'**il est choisi parmi les composés de formules (4-1a) à (4-4a), 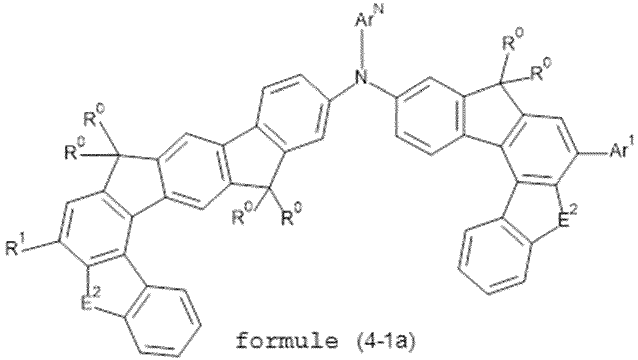 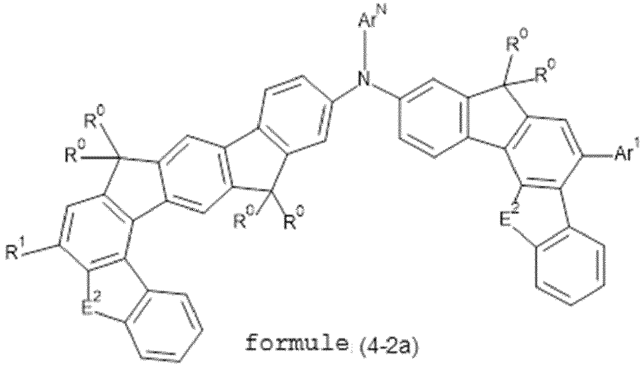 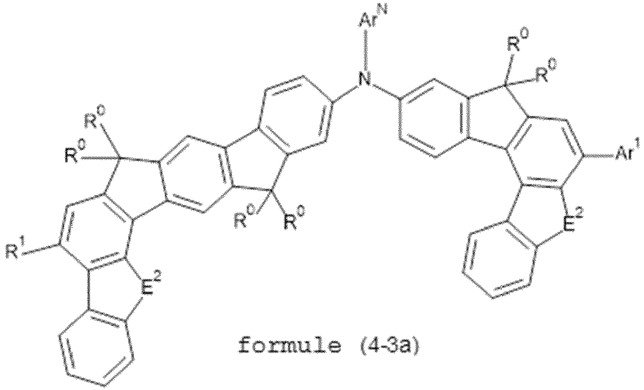 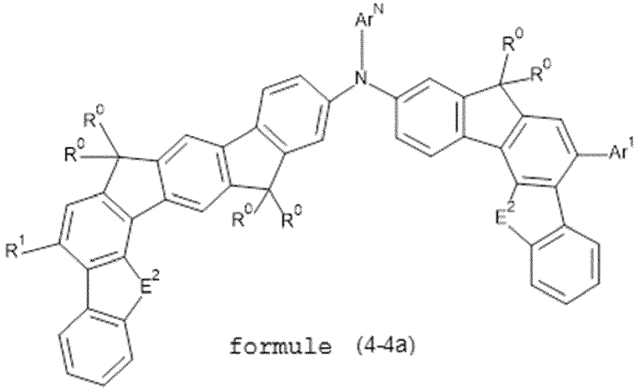 où les symboles Ar^{N}, E², Ar¹, R⁰ et R¹ possèdent la même signification que dans la revendication 1.

9. Composé selon l'une ou plusieurs des revendications précédentes, **caractérisé en ce qu'**il est choisi parmi les composés de formules (4-1b) à (4-4b), 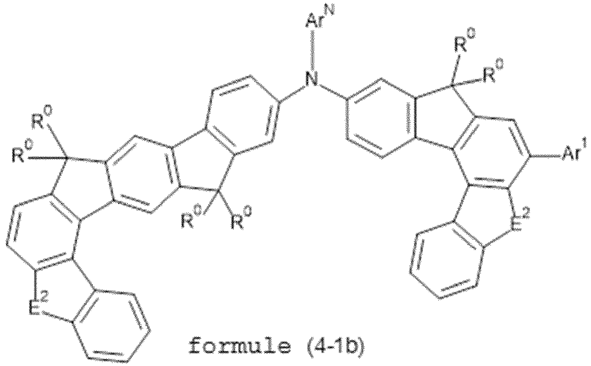 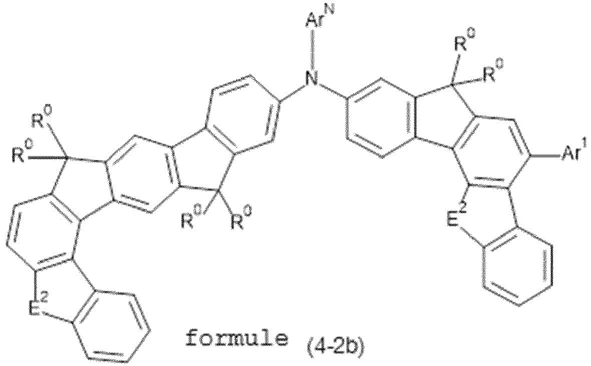 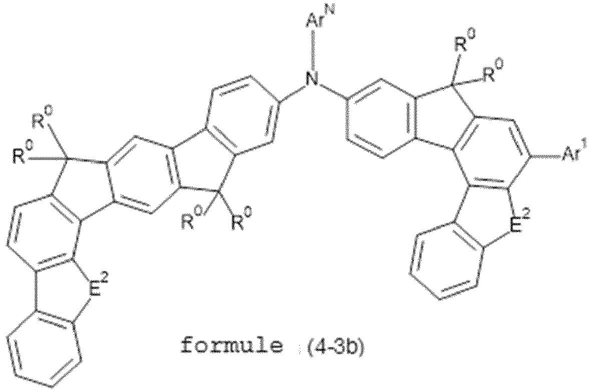 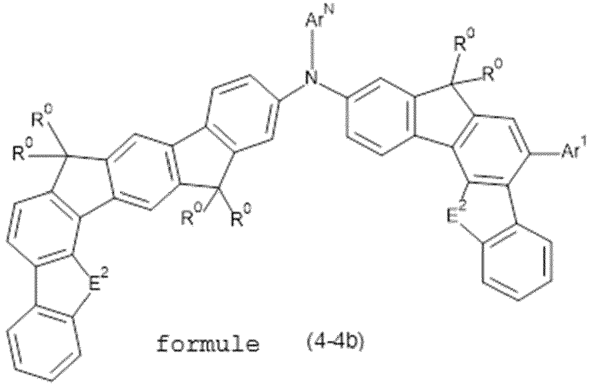 où les symboles Ar^{N}, E², Ar¹ et R⁰ possèdent la même signification que dans la revendication 1.

10. Composé selon l'une ou plusieurs des revendications précédentes, **caractérisé en ce que** E² représente O.

11. Composé selon l'une ou plusieurs des revendications précédentes, **caractérisé en ce que** Ar¹ représente en chaque occurrence, de manière identique ou différente, phényle, biphényle, fluorène, spirobifluorène, naphtalène, phénanthrène, dibenzofuranne, dibenzothiophène, carbazole, pyridine, pyrimidine, pyrazine, pyridazine, triazine, benzopyridine, benzopyridazine, benzopyrimidine ou quinazoline, ou représente une combinaison de deux à six de ces groupes, chacun desquels pouvant être substitué par un ou plusieurs radicaux R.

12. Composé selon l'une ou plusieurs des revendications précédentes, **caractérisé en ce que** Ar¹ représente en chaque occurrence, de manière identique ou différente, un système cyclique aromatique ou hétéroaromatique choisi parmi phényle, biphényle, fluorène et naphtalène, ou représente une combinaison de deux à six de ces groupes, chacun desquels pouvant être substitué par un ou plusieurs radicaux R.

13. Composé selon l'une ou plusieurs des revendications précédentes, **caractérisé en ce que** Ar¹ est choisi parmi les groupes de formules (Ar1-1) à (Ar1-22), 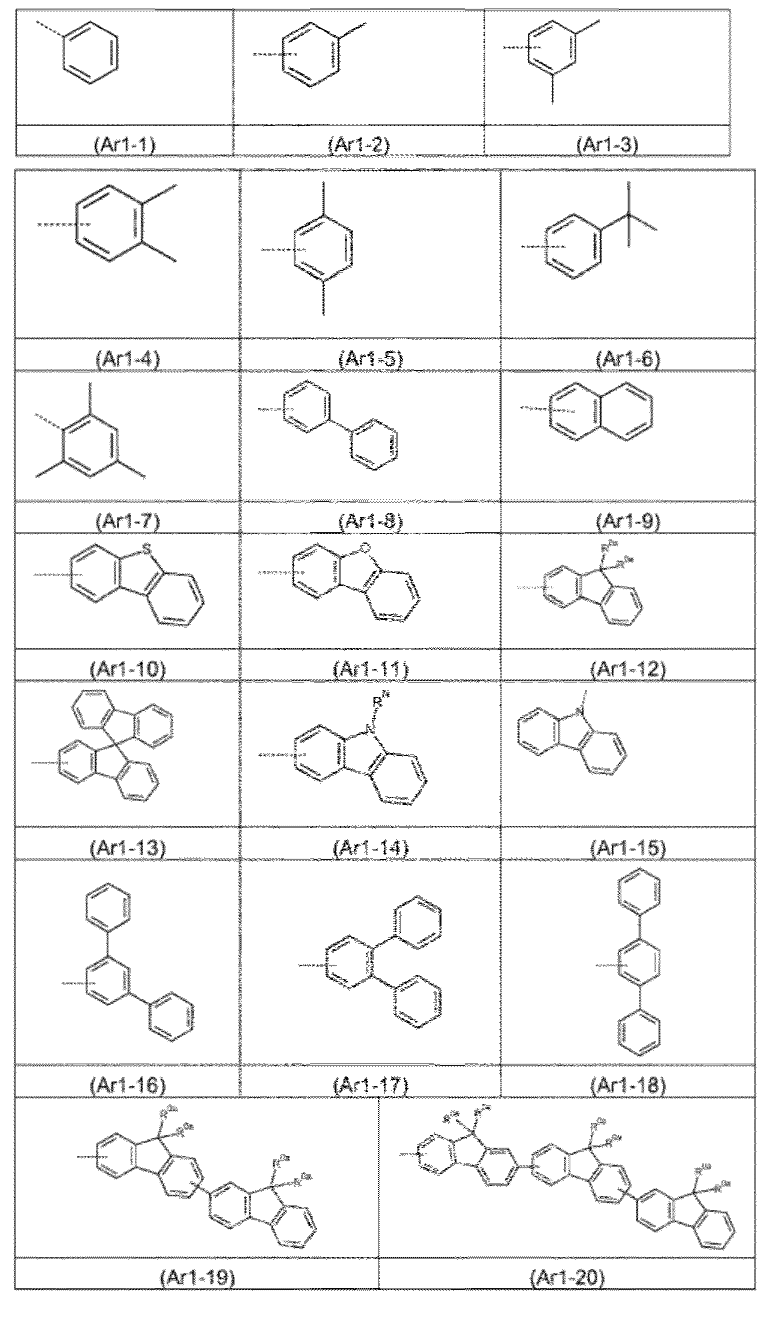 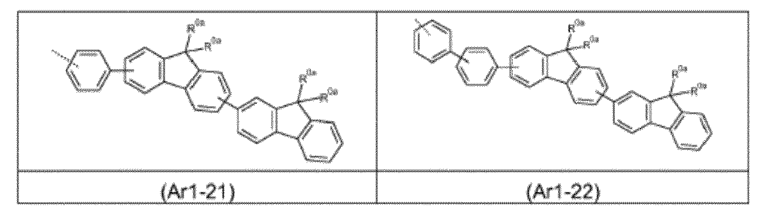 où dans les formules (Ar1-1) à (Ar1-22) :
- la liaison en pointillés indique la liaison à la structure de formule (1) ;
- le groupe R^{N} dans la formule (Ar1-14) représente en chaque occurrence, de manière identique ou différente, H, D, un groupe alkyle linéaire possédant 1 à 40, préférablement 1 à 20, plus préférablement 1 à 10 atomes de carbone ou un groupe alkyle ramifié ou cyclique possédant 3 à 40, préférablement 3 à 20, plus préférablement 3 à 10 atomes de carbone, chacun desquels pouvant être substitué par un ou plusieurs radicaux R, où en chaque cas un ou plusieurs groupes CH₂ non adjacents peuvent être remplacés par RC=CR, C≡C, C=O, C=S, SO, SO₂, O ou S, et où un ou plusieurs atomes de H peuvent être remplacés par D, F ou CN, un système cyclique aromatique ou hétéroaromatique possédant 5 à 60, préférablement 5 à 40, plus préférablement 5 à 30, particulièrement préférablement 5 à 18 atomes de cycle aromatique, qui peut en chaque cas être substitué par un ou plusieurs radicaux R, où deux substituants R^{N} adjacents peuvent former un système cyclique aliphatique ou un système cyclique aromatique, monocyclique ou polycyclique, qui peut être substitué par un ou plusieurs radicaux R, où R possède la même signification que dans la revendication 1 ;
- le groupe R^{0a} dans les formules (Ar1-12) et (Ar1-19) à (Ar1-22) possède la même signification que le groupe R⁰ telle que définie dans la revendication 1 ; et
- les groupes de formules (Ar1-1) à (Ar1-22) peuvent être substitués au niveau de chaque position libre par un groupe R, qui possède la même signification que dans la revendication 1.

14. Polymère, oligomère ou dendrimère contenant un ou plusieurs composés selon la revendication 1, où la ou les liaisons au polymère, à l'oligomère ou au dendrimère peuvent être localisées au niveau de quelconques positions dans la formule (1) qui est substituée par R⁰, R¹, R², R³, R⁴ ou R⁵.

15. Formulation comprenant au moins un composé selon l'une ou plusieurs des revendications 1 à 13 ou au moins un polymère, oligomère ou dendrimère selon la revendication 14 et au moins un solvant.

16. Dispositif électronique comprenant au moins un composé selon l'une ou plusieurs des revendications 1 à 13 ou au moins un polymère, oligomère ou dendrimère selon la revendication 14, choisi dans le groupe constitué par des dispositifs électroluminescents organiques, des circuits intégrés organiques, des transistors organiques à effet de champ, des transistors organiques en film mince, des transistors luminescents organiques, des cellules solaires organiques, des cellules solaires organiques sensibilisées par un colorant, des détecteurs optiques organiques, des photorécepteurs organiques, des dispositifs organiques d'extinction de champ, des cellules électrochimiques luminescentes, des diodes laser organiques et des dispositifs organiques d'émission de plasmon.

17. Dispositif électronique selon la revendication 16, qui est un dispositif électroluminescent organique, **caractérisé en ce que** le composé selon l'une ou plusieurs des revendications 1 à 13 ou le polymère, l'oligomère ou le dendrimère selon la revendication 14 est employé en tant qu'émetteur fluorescent ou en tant que matériau de matrice pour des émetteurs fluorescents.
